(19) **Europäisches Patentamt / European Patent Office / Office européen des brevets**

(11) **EP 3 355 815 B1**

(12) **EUROPEAN PATENT SPECIFICATION**

(45) Date of publication and mention of the grant of the patent:
**04.06.2025 Bulletin 2025/23**

(21) Application number: **16774794.8**

(22) Date of filing: **22.09.2016**

(51) International Patent Classification (IPC):
**A61B 18/12** *(2006.01)*  **A61B 18/14** *(2006.01)*
**A61B 17/29** *(2006.01)*  **A61B 17/32** *(2006.01)*
**A61B 18/00** *(2006.01)*

(52) Cooperative Patent Classification (CPC):
**A61B 18/1445; A61B 18/1206; A61B 18/1233;**
A61B 2017/320071; A61B 2017/320094;
A61B 2017/320095; A61B 2018/00178;
A61B 2018/00601; A61B 2018/00607;
A61B 2018/0063; A61B 2018/00642;
A61B 2018/00648; A61B 2018/00684;
A61B 2018/00732; A61B 2018/00779;   (Cont.)

(86) International application number:
**PCT/US2016/052979**

(87) International publication number:
**WO 2017/058617 (06.04.2017 Gazette 2017/14)**

(54) **CIRCUIT TOPOLOGIES FOR COMBINED GENERATOR**

SCHALTUNGSTOPOLOGIEN FÜR KOMBINIERTEN GENERATOR

TOPOLOGIES DE CIRCUIT POUR GÉNÉRATEUR COMBINÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2015 US 201562235368 P**
**07.09.2016 US 201615258570**

(43) Date of publication of application:
**08.08.2018 Bulletin 2018/32**

(73) Proprietor: **Ethicon LLC**
**00969 Guaynabo (PR)**

(72) Inventors:
• **WIENER, Eitan T.**
**Cincinnati, Ohio 45242 (US)**

• **YATES, David C.**
**Cincinnati, Ohio 45242 (US)**
• **HEIN, John E.**
**Neenah, Wisconsin 54957 (US)**

(74) Representative: **Carpmaels & Ransford LLP**
**One Southampton Row**
**London WC1B 5HA (GB)**

(56) References cited:
| | |
|---|---|
| GB-A- 2 521 228 | GB-A- 2 521 228 |
| US-A1- 2003 181 898 | US-A1- 2003 181 898 |
| US-A1- 2009 287 205 | US-A1- 2009 287 205 |
| US-A1- 2011 273 465 | US-A1- 2011 273 465 |

(52) Cooperative Patent Classification (CPC): (Cont.)
A61B 2018/00875; A61B 2018/00958;
A61B 2018/00994; A61B 2018/1273;
A61B 2018/128; A61B 2018/1286; A61B 2018/1293;
A61B 2018/1452; A61B 2090/0811; H03F 2200/18

## Description

## PRIORITY

This application claims the benefit of U.S. Provisional Application Serial No. 62/235,260, titled GENERATOR FOR PROVIDING COMBINED RADIO FREQUENCY AND ULTRASONIC ENERGIES, filed September 30, 2015, U.S. Provisional Application Serial No. 62/235,368, titled CIRCUIT TOPOLOGIES FOR GENERATOR, filed September 30, 2015, and U.S. Provisional Application Serial No. 62/235,466, titled SURGICAL INSTRUMENT WITH USER ADAPTABLE ALGORITHMS, filed September 30, 2015.

## TECHNICAL FIELD

The present disclosure generally relates to ultrasonic surgical systems, electrosurgical systems, and combination electrosurgical/ultrasonic systems for performing surgical procedures such as coagulating, sealing, and/or cutting tissue. In particular, the present disclosure relates to circuit topologies for a combined generator configured to deliver a combined signal for radio frequency (RF) and ultrasonic outputs to a medical instrument.

## BACKGROUND

The present disclosure is related generally to surgical instruments and associated surgical techniques. More particularly, the present disclosure is related to ultrasonic and electrosurgical systems that allow surgeons to perform cutting and coagulation and to adapt and customize such procedures based on the type of tissue being treated.

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and hemostasis by coagulation, desirably minimizing patient trauma. The cutting action is typically realized by an end effector, or blade tip, at the distal end of the instrument, which transmits ultrasonic energy to tissue brought into contact with the end effector. Ultrasonic instruments of this nature can be configured for open surgical use, laparoscopic, or endoscopic surgical procedures including robotic-assisted procedures.

Some surgical instruments utilize ultrasonic energy for both precise cutting and controlled coagulation. Ultrasonic energy cuts and coagulates by vibrating a blade in contact with tissue. Vibrating at high frequencies (e.g., 55,500 times per second), the ultrasonic blade denatures protein in the tissue to form a sticky coagulum. Pressure exerted on tissue with the blade surface collapses blood vessels and allows the coagulum to form a hemostatic seal. The precision of cutting and coagulation is controlled by the surgeon's technique and adjusting the power level, blade edge, tissue traction, and blade pressure.

Electrosurgical devices for applying electrical energy to tissue in order to treat and/or destroy the tissue are also finding increasingly widespread applications in surgical procedures. An electrosurgical device typically includes a hand piece, an instrument having a distallymounted end effector (e.g., one or more electrodes). The end effector can be positioned against the tissue such that electrical current is introduced into the tissue. Electrosurgical devices can be configured for bipolar or monopolar operation. During bipolar operation, current is introduced into and returned from the tissue by active and return electrodes, respectively, of the end effector. During monopolar operation, current is introduced into the tissue by an active electrode of the end effector and returned through a return electrode (e.g., a grounding pad) separately located on a patient's body. Heat generated by the current flowing through the tissue may form hemostatic seals within the tissue and/or between tissues and thus may be particularly useful for sealing blood vessels, for example. The end effector of an electrosurgical device may also include a cutting member that is movable relative to the tissue and the electrodes to transect the tissue.

Electrical energy applied by an electrosurgical device can be transmitted to the instrument by a generator in communication with the hand piece. The electrical energy may be in the form of radio frequency ("RF") energy. RF energy is a form of electrical energy that may be in the frequency range of 200 kilohertz (kHz) to 1 megahertz (MHz). In application, an electrosurgical device can transmit low frequency RF energy through tissue, which causes ionic agitation, or friction, in effect resistive heating, thereby increasing the temperature of the tissue. Because a sharp boundary is created between the affected tissue and the surrounding tissue, surgeons can operate with a high level of precision and control, without sacrificing un-targeted adjacent tissue. The low operating temperatures of RF energy is useful for removing, shrinking, or sculpting soft tissue while simultaneously sealing blood vessels. RF energy works particularly well on connective tissue, which is primarily comprised of collagen and shrinks when contacted by heat.

A challenge of using these medical devices is the inability to fully control and customize the functions of the surgical instruments. It would be desirable to provide a surgical instrument that overcomes some of the deficiencies of current instruments.

While several medical devices have been made and used, it is believed that no one prior to the inventors has made or used the subject matter described in the appended claims.

US 2011/273465 A1 relates to an ultrasound output apparatus that includes a first output connector for connecting a first handpiece, a second output connector arranged neighboring the first output connector for connecting a second handpiece, an HP connector connection detection circuit that detects that the first and second handpieces are connected to the first and second output connectors respectively, a display section provided with first and second display screens for displaying setting information of the first and second handpieces at predetermined positions according to an array of the first output connector and the second output connector and a GUI control section that controls a number of partitions of the display section according to connection statuses of the first and second handpieces so as to display, when the HP connector connection detection circuit detects that the first and second handpieces are connected to the first and second output connectors, setting information of the first and second handpieces on the first and second display screens, display, when the HP connector connection detection circuit detects that only the first handpiece is connected to the first output connector, the setting information of the first handpiece on the entire display section and display, when the HP connector connection detection circuit detects that only the second handpiece is connected to the second output connector, the setting information of the second handpiece on the entire display section, and performs a display reflecting the connection status of the HP connector connection detection circuit.

US 2003/181898 A1 relates to an electrosurgical generator that comprises a circuit and method to produce a filtering technique that utilizes the output transformer as the inductive element of the filter and places two capacitors on the output of the transformer to produce the desired filtering. The output filter configuration eliminates the use of power inductors and still creates the filtering required in electrosurgical applications. The filter utilizes the inductive properties of the output transformer along with several capacitors to form the bandpass filter.

GB 2521228 A relates to a medical device comprising: an end effector for gripping tissue; an ultrasonic transducer; a drive circuit generating a periodic signal and a controller to apply the signal to either the transducer or the effector.

## SUMMARY

The invention is limited by the scope of independent claims 1 and 8. Optional features are disclosed in the dependent claims.

In one aspect, the present disclosure is directed to a mixed energy surgical instrument that utilizes both Ultrasonic and RF energy modalities. Multiple circuit topologies are disclosed which when one (or more) of these circuit topologies are included in a mixed energy surgical instrument, the circuit topology enables a generator to drive both RF and Ultrasonic energy into tissue either simultaneously or by switching between RF and Ultrasonic.

In some aspects, the circuit topology may include high frequency filters configured to filter a combined ultrasonic and RF frequency signal into signals having only ultrasonic frequency content and separately, RF frequency content. In some cases, one or more band-stop filters are used. In some cases, one or more resonators are used to accentuate the desired frequencies. In other cases, one or more pass-based filters are used. In some aspects, the circuit topology allows for simultaneous application of both RF energy and ultrasonic energy, both derived from the single combined signal.

In some aspects, the circuit topology may include more or more switches configured to switch between the RF frequency and the ultrasonic frequency within the same combined signal. In some cases, one or more pairs of solid state switches provide the switching functionality. In one aspect, metal oxide semiconductor (MOSFET) switches may be employed to provide the switching functionality. In some cases, a control circuit, which may be implemented as n application specific integrated circuit (ASIC), is also used to control the switching. One or more pulse transformers may be coupled to the control circuit and the pairs of MOSFET switches, in some cases. In other cases, switching may occur through inclusion of one or more electromechanical relays coupled to the control circuit.

In addition to the foregoing, various other unclaimed method and/or system and/or program product aspects are set forth and described in the teachings such as text (e.g., detailed description) and/or drawings of the present disclosure.

The foregoing is a summary and thus may contain simplifications, generalizations, inclusions, and/or omissions of detail; consequently, those skilled in the art will appreciate that the summary is illustrative only and is NOT intended to be in any way limiting. Other aspects, features, and advantages of the devices and/or processes and/or other subject matter described herein will become apparent in the teachings set forth herein.

In one or more various aspects, related systems include but are not limited to circuitry and/or programming for effecting herein-referenced method aspects; the circuitry and/or programming can be virtually any combination of hardware, software, and/or firmware configured to affect the herein-referenced method aspects depending upon the design choices of the system. In addition to the foregoing, various other method and/or system aspects are set forth and described in the teachings such as text (e.g., claims and/or detailed description) and/or drawings of the present disclosure.

Various forms are directed to improved ultrasonic surgical instruments configured for effecting tissue dissecting, cutting, and/or coagulation during surgical procedures. In one form, an ultrasonic surgical instrument apparatus is configured for use in open surgical procedures, but has applications in other types of surgery, such as laparoscopic, endoscopic, and robotic-assisted procedures. Versatile use is facilitated by selective use of ultrasonic energy.

The various forms will be described in combination with an ultrasonic instrument as described herein. Such description is provided by way of example, and not limitation, and is not intended to limit the scope and applications thereof. For example, any one of the described forms is useful in combination with a multitude of ultrasonic instruments including those described in, for example, U.S. Patent Nos. 5,938,633; 5,935,144; 5,944,737; 5,322,055; 5,630,420; and 5,449,370.

As will become apparent from the following description, it is contemplated that forms of the surgical instrument described herein may be used in association with an oscillator unit of a surgical system, whereby ultrasonic energy from the oscillator unit provides the desired ultrasonic actuation for the present surgical instrument. It is also contemplated that forms of the surgical instrument described herein may be used in association with a signal generator unit of a surgical system, whereby electrical energy in the form of radio frequencies (RF), for example, is used to provide feedback to the user regarding the surgical instrument. The ultrasonic oscillator and/or the signal generator unit may be non-detachably integrated with the surgical instrument or may be provided as separate components, which can be electrically attachable to the surgical instrument.

One form of the present surgical apparatus is particularly configured for disposable use by virtue of its straightforward construction. However, it is also contemplated that other forms of the present surgical instrument can be configured for non-disposable or multiple uses. Detachable connection of the present surgical instrument with an associated oscillator and signal generator unit is presently disclosed for single-patient use for illustrative purposes only. However, non-detachable integrated connection of the present surgical instrument with an associated oscillator and/or signal generator unit is also contemplated. Accordingly, various forms of the presently described surgical instruments may be configured for single use and/or multiple uses with either detachable and/or non-detachable integral oscillator and/or signal generator unit.

The foregoing summary is illustrative only and is not intended to be in any way limiting. In addition to the illustrative aspects and features described above, further aspects and features will become apparent by reference to the drawings and the following detailed description.

## FIGURES

The novel features described herein are set forth with particularity in the appended claims. Various aspects, however, both as to organization and methods of operation may be better understood by reference to the following description, taken in conjunction with the accompanying drawings as follows:

FIG. 1 illustrates one form of a surgical system comprising a generator and various surgical instruments usable therewith;

FIG. 2 is a diagram of the combination electrosurgical and ultrasonic instrument shown in FIG. 1;

FIG. 3 is a diagram of the surgical system shown in FIG. 1;

FIG. 4 is a model illustrating motional branch current in one form;

FIG. 5 is a structural view of a generator architecture in one form;

FIG. 6 illustrates one form of a drive system of a generator, which creates the ultrasonic electrical signal for driving an ultrasonic transducer;

FIG. 7 illustrates one form of a drive system of a generator comprising a tissue impedance module;

FIG. 8 illustrates an example of a combined radio frequency and ultrasonic energy generator for delivering energy to a surgical instrument;

FIG. 9 is a diagram of a system for delivering combined radio frequency and ultrasonic energy to a plurality of surgical instruments;

FIG. 10 illustrates a communications architecture of a system for delivering combined radio frequency and ultrasonic energy to a plurality of surgical instruments;

FIG. 11 illustrates a communications architecture of a system for delivering combined radio frequency and ultrasonic energy to a plurality of surgical instruments;

FIG. 12 illustrates a communications architecture of a system for delivering combined radio frequency and ultrasonic energy to a plurality of surgical instruments;

FIG. 13 is a circuit schematic for a system that is configured to manage RF and ultrasonic currents output by a generator according to one aspect of the present disclosure;

FIG. 14 is a circuit schematic for a system that is configured to manage RF and ultrasonic currents output by a generator according to one aspect of the present disclosure;

FIG. 15 displays a graph of simulation results of the circuit schematic shown in FIG. 14;

FIG. 16 displays graphs of simulation results of the circuit schematic shown in FIG. 14;

FIG. 17 displays graphs of simulation results of the circuit schematic shown in FIG. 14;

FIG. 18 displays graphs of simulation results of the circuit schematic shown in FIG. 14;

FIG. 19 displays graphs of simulation results of the circuit schematic shown in FIG. 14;

FIG. 20 displays graphs of simulation results of the circuit schematic shown in FIG. 14;

FIG. 21 displays graphs of simulation results of the circuit schematic shown in FIG. 14;

FIG. 22 is a circuit schematic, including a high frequency band stop filter, for a system that is configured to manage RF and ultrasonic currents output by a generator according to one aspect of the present disclosure;

FIG. 23 is a circuit schematic, including tuned LC circuits, for a system that is configured to manage RF and ultrasonic currents output by a generator according to one aspect of the present disclosure;

FIG. 24 is a circuit schematic, including series connected MOSFET transistors, for a system that is configured to manage RF and ultrasonic currents output by a generator according to one aspect of the present disclosure;

FIG. 25 is a circuit schematic, including a pair of MOSFET switches arranged sourcesource, for a system that is configured to manage RF and ultrasonic currents output by a generator according to one aspect of the present disclosure;

FIG. 26 is a circuit schematic, including series connected MOSFET transistors, for a system that is configured to manage RF and ultrasonic currents output by a generator according to one aspect of the present disclosure;

FIG. 27 is a circuit schematic, including a pair of electromechanical relays, for a system that is configured to manage RF and ultrasonic currents output by a generator according to one aspect of the present disclosure;

FIG. 28 is a circuit schematic, including a switch actuation, for a system that is configured to manage RF and ultrasonic currents output by a generator according to one aspect of the present disclosure;

FIG. 29 is a circuit schematic, including components from multiple configurations discussed in previous figures, for a system that is configured to manage RF and ultrasonic currents output by a generator according to one aspect of the present disclosure;

FIG. 30 is an illustration of a system configuration for a circuit topology, including MOSFET switches and a control circuit in the proximal plug, configured to manage RF and ultrasonic currents output by a generator according to one aspect of the present disclosure;

FIG. 31 is an illustration of a system configuration for a circuit topology, including MOSFET switches and a control circuit in the distal plug, configured to manage RF and ultrasonic currents output by a generator according to one aspect of the present disclosure;

FIG. 32 is an illustration of a system configuration for a circuit topology, including MOSFET switches and a control circuit in the distal plug and a control circuit in the handle, configured to manage RF and ultrasonic currents output by a generator according to one aspect of the present disclosure;

FIG. 33 is an illustration of a system configuration for a circuit topology, including MOSFET switches in the distal plug and a control circuit in the handle, configured to manage RF and ultrasonic currents output by a generator according to one aspect of the present disclosure;

FIG. 34 is an illustration of a system configuration for a circuit topology, including MOSFET switches and a control circuit in the handle, configured to manage RF and ultrasonic currents output by a generator according to one aspect of the present disclosure;

FIG. 35 is an illustration of a system configuration for a circuit topology, including bandstop filters in the proximal plug and a control circuit in the handle, configured to manage RF and ultrasonic currents output by a generator according to one aspect of the present disclosure;

FIG. 36 is an illustration of a system configuration for a circuit topology, including bandstop filters in the distal plug and a control circuit in the handle, configured to manage RF and ultrasonic currents output by a generator according to one aspect of the present disclosure;

FIG. 37 is an illustration of a system configuration for a circuit topology, including bandstop filters and a control circuit in the handle, configured to manage RF and ultrasonic currents output by a generator according to one aspect of the present disclosure;

FIG. 38 is an illustration of a system configuration for a circuit topology, including bandstop filters in the distal plug, a control circuit in the handle, and a DC motor in the application portion, configured to manage RF and ultrasonic currents output by a generator according to one aspect of the present disclosure;

FIG. 39 is an illustration of a system configuration for a circuit topology, including a fixed high voltage RF output in the application portion, bandstop filters in the distal plug, and a control circuit and transformer in handle, configured to manage RF and ultrasonic currents output by a generator according to one aspect of the present disclosure;

FIG. 40 is an illustration of a system configuration for a circuit topology, including a mechanically switched high voltage/low voltage RF output in the application portion, bandstop filters in distal plug, and a control circuit and transformer in the handle, configured to manage RF and ultrasonic currents output by a generator according to one aspect of the present disclosure;

FIG. 41 is an illustration of a system configuration for a circuit topology, including an electrically switched high voltage/low voltage RF output in the application portion, bandstop filters in distal plug, and a control circuit and transformer in the handle, configured to manage RF and ultrasonic currents output by a generator according to one aspect of the present disclosure;

FIG. 42 is an illustration of a system configuration for a circuit topology, including a fixed high voltage RF output in the application portion, bandstop filters in the proximal plug, and a control circuit and transformer in handle, configured to manage RF and ultrasonic currents output by a generator according to one aspect of the present disclosure;

FIG. 43 is an example graph of two waveforms of energy from a generator;

FIG. 44 is an example graph of the sum of the waveforms of FIG. 43;

FIG. 45 is an example graph of sum of the waveforms of FIG. 43 with the RF waveform dependent on the ultrasonic waveform;

FIG. 46 is an example graph of the sum of the waveforms of FIG. 43 with the RF waveform being a function of the ultrasonic waveform; and

FIG. 47 is an example graph of a complex RF waveform with a high crest factor.

## DESCRIPTION

In the following detailed description, reference is made to the accompanying drawings, which form a part hereof. In the drawings, similar symbols and reference characters typically identify similar components throughout the several views, unless context dictates otherwise. The illustrative aspects described in the detailed description, drawings, and claims are not meant to be limiting. Other aspects may be utilized, and other changes may be made, without departing from the scope of the subject matter presented in the claims.

Also, in the following description, it is to be understood that terms such as front, back, inside, outside, top, bottom and the like are words of convenience and are not to be construed as limiting terms. Terminology used herein is not meant to be limiting insofar as devices described herein, or portions thereof, may be attached or utilized in other orientations. The various aspects will be described in more detail with reference to the drawings.

This application also is related to the following commonly owned patent applications filed on June 9, 2016:

> U.S. Patent Application No. 15/177,430, titled SURGICAL INSTRUMENT WITH USER ADAPTABLE TECHNIQUES;
> U.S. Patent Application No. 15/177,439, titled SURGICAL INSTRUMENT WITH USER ADAPTABLE TECHNIQUES BASED ON TISSUE TYPE;
> U.S. Patent Application No. 15/177,449, titled SURGICAL SYSTEM WITH USER ADAPTABLE TECHNIQUES EMPLOYING MULTIPLE ENERGY MODALITIES BASED ON TISSUE;
> U.S. Patent Application No. 15/177,456, titled SURGICAL SYSTEM WITH USER ADAPTABLE TECHNIQUES BASED ON TISSUE IMPEDANCE;
> U.S. Patent Application No. 15/177,466, titled SURGICAL SYSTEM WITH USER ADAPTABLE TECHNIQUES EMPLOYING SIMULTANEOUS ENERGY MODALITIES BASED ON TISSUE PARAMETERS;

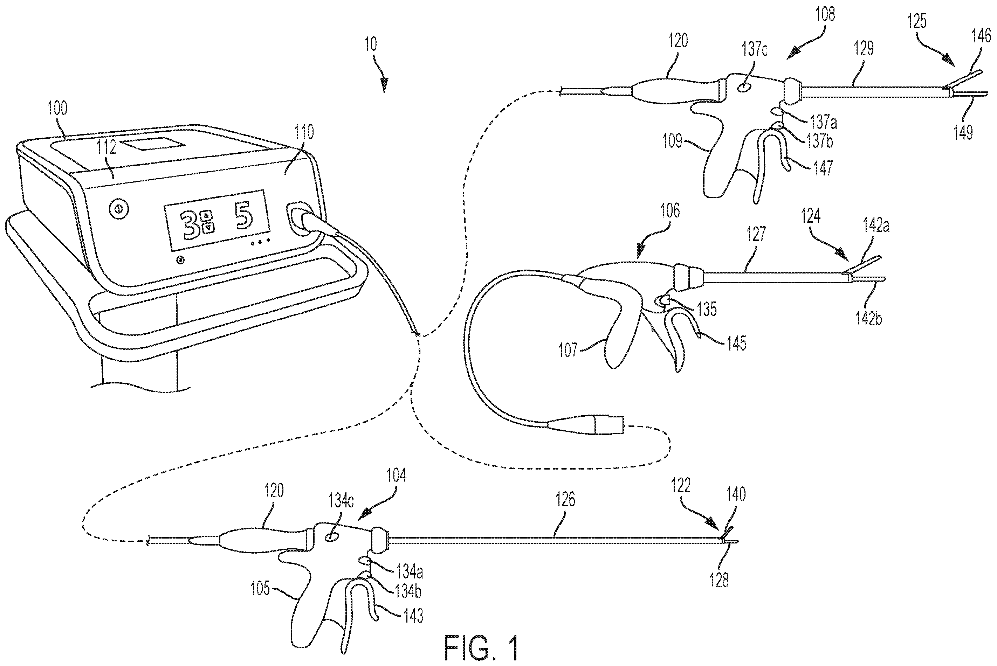

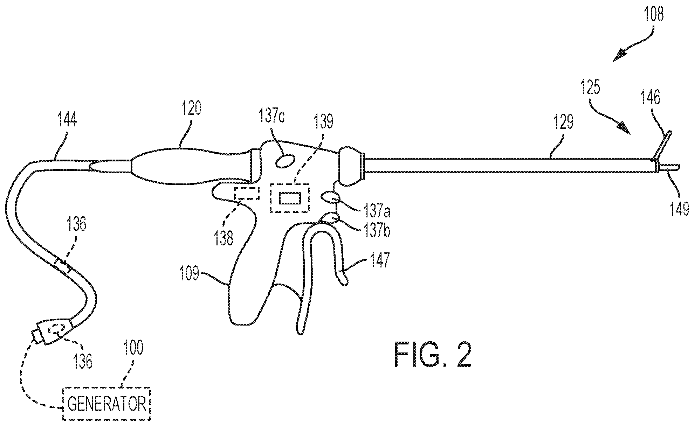

With reference to FIGS. 1-5, one form of a surgical system 10 including a surgical instrument is illustrated. FIG. 1 illustrates one form of a surgical system 10 comprising a generator 100 and various surgical instruments 104, 106, 108 usable therewith, where the surgical instrument 104 is an ultrasonic surgical instrument, the surgical instrument 106 is an RF electrosurgical instrument 106, and the multifunction surgical instrument 108 is a combination ultrasonic/RF electrosurgical instrument. FIG. 2 is a diagram of the multifunction surgical instrument 108 shown in FIG. 1. With reference to both FIGS. 1 and 2, the generator 100 is configurable for use with a variety of surgical instruments.

According to various forms, the generator 100 may be configurable for use with different surgical instruments of different types including, for example, ultrasonic surgical instruments 104, RF electrosurgical instruments 106, and multifunction surgical instruments 108 that integrate RF and ultrasonic energies delivered simultaneously from the generator 100. Although in the form of FIG. 1, the generator 100 is shown separate from the surgical instruments 104, 106, 108 in one form, the generator 100 may be formed integrally with any of the surgical instruments 104, 106, 108 to form a unitary surgical system. The generator 100 comprises an input device 110 located on a front panel of the generator 100 console. The input device 110 may comprise any suitable device that generates signals suitable for programming the operation of the generator 100.

FIG. 1 illustrates a generator 100 configured to drive multiple surgical instruments 104, 106, 108. The first surgical instrument 104 is an ultrasonic surgical instrument 104 and comprises a handpiece 105 (HP), an ultrasonic transducer 120, a shaft 126, and an end effector 122. The end effector 122 comprises an ultrasonic blade 128 acoustically coupled to the ultrasonic transducer 120 and a clamp arm 140. The handpiece 105 comprises a trigger 143 to operate the clamp arm 140 and a combination of the toggle buttons 134a, 134b, 134c to energize and drive the ultrasonic blade 128 or other function. The toggle buttons 134a, 134b, 134c can be configured to energize the ultrasonic transducer 120 with the generator 100.

Still with reference to FIG.1, the generator 100 also is configured to drive a second surgical instrument 106. The second surgical instrument 106 is an RF electrosurgical instrument and comprises a handpiece 107 (HP), a shaft 127, and an end effector 124. The end effector 124 comprises electrodes in the clamp arms 142a, 142b and return through an electrical conductor portion of the shaft 127. The electrodes are coupled to and energized by a bipolar energy source within the generator 100. The handpiece 107 comprises a trigger 145 to operate the clamp arms 142a, 142b and an energy button 135 to actuate an energy switch to energize the electrodes in the end effector 124.

Still with reference to FIG. 1, the generator 100 also is configured to drive a multifunction surgical instrument 108. The multifunction surgical instrument 108 comprises a handpiece 109 (HP), a shaft 129, and an end effector 125. The end effector comprises an ultrasonic blade 149 and a clamp arm 146. The ultrasonic blade 149 is acoustically coupled to the ultrasonic transducer 120. The handpiece 109 comprises a trigger 147 to operate the clamp arm 146 and a combination of the toggle buttons 137a, 137b, 137c to energize and drive the ultrasonic blade 149 or other function. The toggle buttons 137a, 137b, 137c can be configured to energize the ultrasonic transducer 120 with the generator 100 and energize the ultrasonic blade 149 with a bipolar energy source also contained within the generator 100.

With reference to both FIGS. 1 and 2, the generator 100 is configurable for use with a variety of surgical instruments. According to various forms, the generator 100 may be configurable for use with different surgical instruments of different types including, for example, the ultrasonic surgical instrument 104, the RF electrosurgical instrument 106, and the multifunction surgical instrument 108 that integrate RF and ultrasonic energies delivered simultaneously from the generator 100. Although in the form of FIG. 1, the generator 100 is shown separate from the surgical instruments 104, 106, 108, in one form, the generator 100 may be formed integrally with any one of the surgical instruments 104, 106, 108 to form a unitary surgical system. The generator 100 comprises an input device 110 located on a front panel of the generator 100 console. The input device 110 may comprise any suitable device that generates signals suitable for programming the operation of the generator 100. The generator 100 also may comprise one or more output devices 112.

With reference now to FIG. 2, the generator 100 is coupled to the multifunction surgical instrument 108. The generator 100 is coupled to the ultrasonic transducer 120 and electrodes located in the clamp arm 146 via a cable 144. The ultrasonic transducer 120 and a waveguide extending through a shaft 129 (waveguide not shown in FIG. 2) may collectively form an ultrasonic drive system driving an ultrasonic blade 149 of an end effector 125. The end effector 125 further may comprise a clamp arm 146 to clamp tissue located between the clamp arm 146 and the ultrasonic blade 149. The clamp arm 146 comprises one or more than one an electrode coupled to the a pole of the generator 100 (e.g., a positive pole). The ultrasonic blade 149 forms the second pole (e.g., the negative pole) and is also coupled to the generator 100. RF energy is applied to the electrode(s) in the clamp arm 146, through the tissue located between the clamp arm 146 and the ultrasonic blade 149, and through the ultrasonic blade 149 back to the generator 100 via the cable 144. In one form, the generator 100 may be configured to produce a drive signal of a particular voltage, current, and/or frequency output signal that can be varied or otherwise modified with high resolution, accuracy, and repeatability suitable for driving an ultrasonic transducer 120 and applying RF energy to tissue..

Still with reference to FIG. 2, It will be appreciated that the multifunction surgical instrument 108 may comprise any combination of the toggle buttons 137a, 137b, 134c. For example, the multifunction surgical instrument 108 could be configured to have only two toggle buttons: a toggle button 137a for producing maximum ultrasonic energy output and a toggle button 137b for producing a pulsed output at either the maximum or less than maximum power level. In this way, the drive signal output configuration of the generator 100 could be 5 continuous signals and 5 or 4 or 3 or 2 or 1 pulsed signals. In certain forms, the specific drive signal configuration may be controlled based upon, for example, nonvolatile memory such as an electrically erasable programmable read only memory (EEPROM) settings in the generator 100 and/or user power level selection(s).

In certain forms, a two-position switch may be provided as an alternative to a toggle button 137c. For example, the multifunction surgical instrument 108 may include a toggle button 137a for producing a continuous output at a maximum power level and a two-position toggle button 137b. In a first detented position, toggle button 137b may produce a continuous output at a less than maximum power level, and in a second detented position the toggle button 137b may produce a pulsed output (e.g., at either a maximum or less than maximum power level, depending upon the EEPROM settings). Any one of the buttons 137a, 137b, 137c may be configured to activate RF energy and apply the RF energy to the end effector 125.

Still with reference to FIG. 2, forms of the generator 100 may enable communication with instrument-based data circuits. For example, the generator 100 may be configured to communicate with a first data circuit 136 and/or a second data circuit 138. For example, the first data circuit 136 may indicate a burn-in frequency slope, as described herein. Additionally or alternatively, any type of information may be communicated to second data circuit for storage therein via a data circuit interface (e.g., using a logic device). Such information may comprise, for example, an updated number of operations in which the instrument has been used and/or dates and/or times of its usage. In certain forms, the second data circuit may transmit data acquired by one or more sensors (e.g., an instrument-based temperature sensor). In certain forms, the second data circuit may receive data from the generator 100 and provide an indication to a user (e.g., a light emitting diode (LED) indication or other visible indication) based on the received data. The second data circuit 138 contained in the multifunction surgical instrument 108. In some forms, the second data circuit 138 may be implemented in a many similar to that of the first data circuit 136 described herein. An instrument interface circuit may comprise a second data circuit interface to enable this communication. In one form, the second data circuit interface may comprise a tri-state digital interface, although other interfaces also may be used. In certain forms, the second data circuit may generally be any circuit for transmitting and/or receiving data. In one form, for example, the second data circuit may store information pertaining to the particular surgical instrument 104, 106, 108 with which it is associated. Such information may include, for example, a model number, a serial number, a number of operations in which the surgical instrument 104, 106, 108 has been used, and/or any other type of information. In the example of FIG. 2, the second data circuit 138 may store information about the electrical and/or ultrasonic properties of an associated ultrasonic transducer 120, end effector 125, ultrasonic energy drive system, or RF electrosurgical energy drive system. Various processes and techniques described herein may be executed by a generator. It will be appreciated, however, that in certain example forms, all or a part of these processes and techniques may be performed by internal logic 139 located in the multifunction surgical instrument 108.

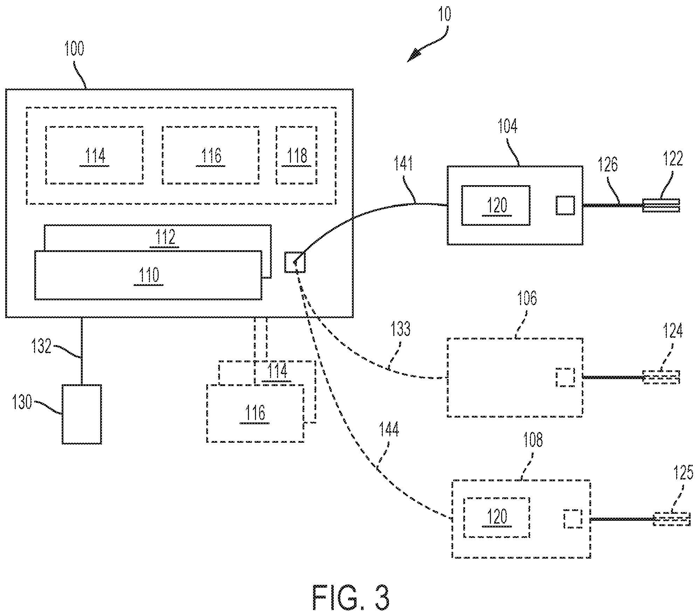

FIG. 3 is a diagram of the surgical system 10 of FIG. 1. In various forms, the generator 100 may comprise several separate functional elements, such as modules and/or blocks. Different functional elements or modules may be configured for driving the different kinds of surgical instruments 104, 106, 108. For example, an ultrasonic drive circuit 114 may drive ultrasonic devices such as the surgical instrument 104 via a cable 141. An electrosurgery/RF drive circuit 116 may drive the RF electrosurgical instrument 106 via a cable 133. The respective drive circuits 114, 116, 118 may be combined as a combined RF/ultrasonic drive circuit 118 to generate both respective drive signals for driving multifunction surgical instruments 108 via a cable 144. In various forms, the ultrasonic drive circuit 114 and/or the electrosurgery/RF drive circuit 116 each may be formed integrally or externally with the generator 100. Alternatively, one or more of the drive circuits 114, 116, 118 may be provided as a separate circuit module electrically coupled to the generator 100. (The drive circuits 114, 116, 118 are shown in phantom to illustrate this option.) Also, in some forms, the electrosurgery/RF drive circuit 116 may be formed integrally with the ultrasonic drive circuit 114, or vice versa. Also, in some forms, the generator 100 may be omitted entirely and the drive circuits 114, 116, 118 may be executed by processors or other hardware within the respective surgical instruments 104, 106, 108.

In other forms, the electrical outputs of the ultrasonic drive circuit 114 and the electrosurgery/RF drive circuit 116 may be combined into a single electrical signal capable of driving the multifunction surgical instrument 108 simultaneously with electrosurgical RF and ultrasonic energies. This single electrical drive signal may be produced by the combination drive circuit 118. The multifunction surgical instrument 108 comprises an ultrasonic transducer 120 coupled to an ultrasonic blade and one or more electrodes in the end effector 125 to receive ultrasonic and electrosurgical RF energy. The multifunction surgical instrument 108 comprises signal processing components to split the combined RF/ultrasonic energy signal such that the RF signal can be delivered to the electrodes in the end effector 125 and the ultrasonic signal can be delivered to the ultrasonic transducer 120.

In accordance with the described forms, the ultrasonic drive circuit 114 may produce a drive signal or signals of particular voltages, currents, and frequencies, e.g., 55,500 cycles per second (Hz). The drive signal or signals may be provided to the ultrasonic surgical instrument 104, and specifically to the ultrasonic transducer 120, which may operate, for example, as described above. The ultrasonic transducer 120 and a waveguide extending through the shaft 126 (waveguide not shown) may collectively form an ultrasonic drive system driving an ultrasonic blade 128 of an end effector 122. In one form, the generator 100 may be configured to produce a drive signal of a particular voltage, current, and/or frequency output signal that can be stepped or otherwise modified with high resolution, accuracy, and repeatability.

The generator 100 may be activated to provide the drive signal to the ultrasonic transducer 120 in any suitable manner. For example, the generator 100 may comprise a foot switch 130 coupled to the generator 100 via a foot switch cable 132. A clinician may activate the ultrasonic transducer 120 by depressing the foot switch 130. In addition, or instead of the foot switch 130 some forms of the ultrasonic surgical instrument 104 may utilize one or more switches positioned on the handpiece that, when activated, may cause the generator 100 to activate the ultrasonic transducer 120. In one form, for example, the one or more switches may comprise a pair of toggle buttons 137a, 137b (FIG. 2), for example, to determine an operating mode of the ultrasonic surgical instrument 104. When the toggle button 137a is depressed, for example, the generator 100 may provide a maximum drive signal to the ultrasonic transducer 120, causing it to produce maximum ultrasonic energy output. Depressing toggle button 137b may cause the generator 100 to provide a user-selectable drive signal to the ultrasonic transducer 120, causing it to produce less than the maximum ultrasonic energy output.

Additionally or alternatively, the one or more switches may comprise a toggle button 137c that, when depressed, causes the generator 100 to provide a pulsed output. The pulses may be provided at any suitable frequency and grouping, for example. In certain forms, the power level of the pulses may be the power levels associated with toggle buttons 137a, 137b (maximum, less than maximum), for example.

It will be appreciated that the ultrasonic surgical instrument 104 and/or the multifunction surgical instrument 108 may comprise any combination of the toggle buttons 137a, 137b, 137c. For example, the multifunction surgical instrument 108 could be configured to have only two toggle buttons: a toggle button 137a for producing maximum ultrasonic energy output and a toggle button 137c for producing a pulsed output at either the maximum or less than maximum power level. In this way, the drive signal output configuration of the generator 100 could be 5 continuous signals and 5 or 4 or 3 or 2 or 1 pulsed signals. In certain forms, the specific drive signal configuration may be controlled based upon, for example, EEPROM settings in the generator 100 and/or user power level selection(s).

In certain forms, a two-position switch may be provided as an alternative to a toggle button 137c. For example, the ultrasonic surgical instrument 104 may include a toggle button 137a for producing a continuous output at a maximum power level and a two-position toggle button 137b. In a first detented position, toggle button 137b may produce a continuous output at a less than maximum power level, and in a second detented position the toggle button 137b may produce a pulsed output (e.g., at either a maximum or less than maximum power level, depending upon the EEPROM settings).

In accordance with the described forms, the electrosurgery/RF drive circuit 116 may generate a drive signal or signals with output power sufficient to perform bipolar electrosurgery using RF energy. In bipolar electrosurgery applications, the drive signal may be provided, for example, to electrodes located in the end effector 124 of the RF electrosurgical instrument 106, for example. Accordingly, the generator 100 may be configured for therapeutic purposes by applying electrical energy to the tissue sufficient for treating the tissue (e.g., coagulation, cauterization, tissue welding). The generator 100 may be configured for sub-therapeutic purposes by applying electrical energy to the tissue for monitoring parameters of the tissue during a procedure.

As previously discussed, the combination drive circuit 118 may be configured to drive both ultrasonic and RF electrosurgical energies. The ultrasonic and RF electrosurgical energies may be delivered though separate output ports of the generator 100 as separate signals or though a single port of the generator 100 as a single signal that is a combination of the ultrasonic and RF electrosurgical energies. In the latter case, the single signal can be separated by circuits located in the surgical instruments 104, 106, 108.

The surgical instruments 104, 106, 108 additionally or alternatively may comprise a switch to indicate a position of a jaw closure trigger for operating jaws of the end effector 122, 124, 125. Also, in some forms, the generator 100 may be activated based on the position of the jaw closure trigger, (e.g., as the clinician depresses the jaw closure trigger to close the jaws, ultrasonic energy may be applied).

The generator 100 may comprise an input device 110 (FIG. 1) located, for example, on a front panel of the generator 100 console. The input device 110 may comprise any suitable device that generates signals suitable for programming the operation of the generator 100. In operation, the user can program or otherwise control operation of the generator 100 using the input device 110. The input device 110 may comprise any suitable device that generates signals that can be used by the generator (e.g., by one or more processors contained in the generator) to control the operation of the generator 100 (e.g., operation of the ultrasonic drive circuit 114, electrosurgery/RF drive circuit 116, combined RF/ultrasonic drive circuit 118). In various forms, the input device 110 includes one or more of buttons, switches, thumbwheels, keyboard, keypad, touch screen monitor, pointing device, remote connection to a general purpose or dedicated computer. In other forms, the input device 110 may comprise a suitable user interface, such as one or more user interface screens displayed on a touch screen monitor, for example. Accordingly, by way of the input device 110, the user can set or program various operating parameters of the generator, such as, for example, current (I), voltage (V), frequency (f), and/or period (T) of a drive signal or signals generated by the ultrasonic drive circuit 114 and/or electrosurgery/RF drive circuit 116.

The generator 100 also may comprise an output device 112 (FIG. 1), such as an output indicator, located, for example, on a front panel of the generator 100 console. The output device 112 includes one or more devices for providing a sensory feedback to a user. Such devices may comprise, for example, visual feedback devices (e.g., a visual feedback device may comprise incandescent lamps, LEDs, graphical user interface, display, analog indicator, digital indicator, bar graph display, digital alphanumeric display, liquid crystal display (LCD) screen, light emitting diode (LED) indicators), audio feedback devices (e.g., an audio feedback device may comprise speaker, buzzer, audible, computer generated tone, computerized speech, voice user interface (VUI) to interact with computers through a voice/speech platform), or tactile feedback devices (e.g., a tactile feedback device comprises any type of vibratory feedback, haptic actuator).

Although certain modules and/or blocks of the generator 100 may be described by way of example, it can be appreciated that a greater or lesser number of modules and/or blocks may be used and still fall within the scope of the forms. Further, although various forms may be described in terms of modules and/or blocks to facilitate description, such modules and/or blocks may be implemented by one or more hardware components, e.g., processors, Digital Signal Processors (DSPs), Programmable Logic Devices (PLDs), Application Specific Integrated Circuits (ASICs), circuits, registers and/or software components, e.g., programs, subroutines, logic and/or combinations of hardware and software components. Also, in some forms, the various modules described herein may be implemented utilizing similar hardware positioned within the surgical instruments 104, 106, 108 (*i.e.,* the external generator 100 may be omitted).

In one form, the ultrasonic drive circuit 114, electrosurgery/RF drive circuit 116, and/or the combination drive circuit 118 may comprise one or more embedded applications implemented as firmware, software, hardware, or any combination thereof. The drive circuits 114, 116, 118 may comprise various executable modules such as software, programs, data, drivers, application program interfaces (APIs), and so forth. The firmware may be stored in nonvolatile memory (NVM), such as in bit masked read-only memory (ROM) or flash memory. In various implementations, storing the firmware in ROM may preserve flash memory. The NVM may comprise other types of memory including, for example, programmable ROM (PROM), erasable programmable ROM (EPROM), electrically erasable programmable read only memory (EEPROM), or battery backed random-access memory (RAM) such as dynamic RAM (DRAM), Double-Data-Rate DRAM (DDRAM), and/or synchronous DRAM (SDRAM).

In one form, the drive circuits 114, 116, 118 comprise a hardware component implemented as a processor for executing program instructions for monitoring various measurable characteristics of the surgical instruments 104, 106, 108 and generating a corresponding output control signals for operating the surgical instruments 104, 106, 108. In forms in which the generator 100 is used in conjunction with the multifunction surgical instrument 108, the output control signal may drive the ultrasonic transducer 120 in cutting and/or coagulation operating modes. Electrical characteristics of the multifunction surgical instrument 108 and/or tissue may be measured and used to control operational aspects of the generator 100 and/or provided as feedback to the user. In forms in which the generator 100 is used in conjunction with the multifunction surgical instrument 108, the output control signal may supply electrical energy (e.g., RF energy) to the end effector 125 in cutting, coagulation and/or desiccation modes. Electrical characteristics of the multifunction surgical instrument 108 and/or tissue may be measured and used to control operational aspects of the generator 100 and/or provide feedback to the user. In various forms, as previously discussed, the hardware component may be implemented as a DSP, PLD, ASIC, circuits, and/or registers. In one form, the processor may be configured to store and execute computer software program instructions to generate the output signals for driving various components of the surgical instruments 104, 106, 108, such as the ultrasonic transducer 120 and the end effectors 122, 124, 125.

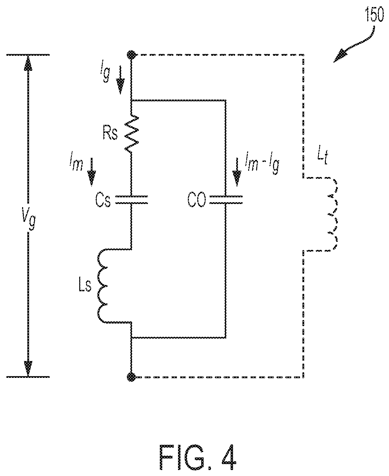

FIG. 4 illustrates an equivalent circuit 150 of an ultrasonic transducer, such as the ultrasonic transducer 120, according to one form. The equivalent circuit 150 comprises a first "motional" branch having a serially connected inductance $L_s$, resistance $R_s$ and capacitance $C_s$ that define the electromechanical properties of the resonator, and a second capacitive branch having a static capacitance $C_o$. Drive current $I_g$ may be received from a generator at a drive voltage $V_g$, with motional current $I_m$ flowing through the first branch and current $I_g - I_m$ flowing through the capacitive branch. Control of the electromechanical properties of the ultrasonic transducer may be achieved by suitably controlling $I_g$ and $V_g$. As explained above, conventional generator architectures may include a tuning inductor $L_t$ (shown in phantom in FIG. 4) for tuning out in a parallel resonance circuit the static capacitance Co at a resonant frequency so that substantially all of generator's current output $I_g$ flows through the motional branch. In this way, control of the motional branch current $I_m$ is achieved by controlling the generator current output $I_g$. The tuning inductor $L_t$ is specific to the static capacitance $C_o$ of an ultrasonic transducer, however, and a different ultrasonic transducer having a different static capacitance requires a different tuning inductor $L_t$. Moreover, because the tuning inductor $L_t$ is matched to the nominal value of the static capacitance Co at a single resonant frequency, accurate control of the motional branch current $I_m$ is assured only at that frequency, and as frequency shifts down with transducer temperature, accurate control of the motional branch current is compromised.

Forms of the generator 100 do not rely on a tuning inductor $L_t$ to monitor the motional branch current $I_m$. Instead, the generator 100 may use the measured value of the static capacitance $C_o$ in between applications of power for a specific ultrasonic surgical instrument 104 (along with drive signal voltage and current feedback data) to determine values of the motional branch current $I_m$ on a dynamic and ongoing basis (e.g., in real-time). Such forms of the generator 100 are therefore able to provide virtual tuning to simulate a system that is tuned or resonant with any value of static capacitance $C_o$ at any frequency, and not just at single resonant frequency dictated by a nominal value of the static capacitance $C_o$.

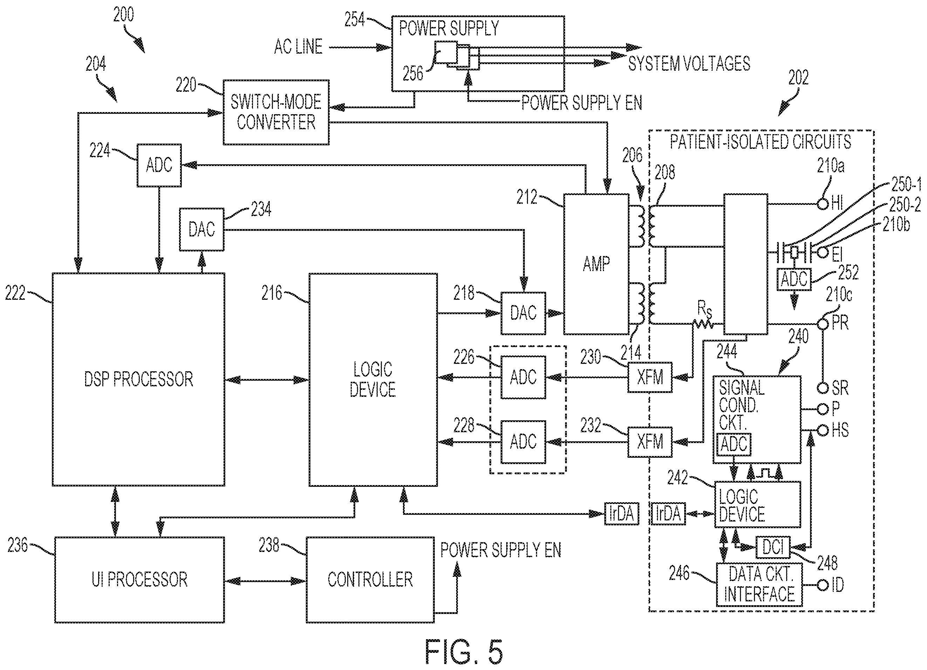

FIG. 5 is a simplified block diagram of a generator 200, which is one form of the generator 100 (FIGS. 1-3). The generator 200 is configured to provide inductorless tuning as described above, among other benefits. Additional details of the generator 200 are described in commonly assigned and contemporaneously filed U.S. Patent No. 9,060,775, titled SURGICAL GENERATOR FOR ULTRASONIC AND ELECTROSURGICAL DEVICES. With reference to FIG. 5, the generator 200 may comprise a patient isolated stage 202 in communication with a non-isolated stage 204 via a power transformer 206. A secondary winding 208 of the power transformer 206 is contained in the isolated stage 202 and may comprise a tapped configuration (e.g., a center-tapped or a non-center-tapped configuration) to define drive signal outputs 210a, 210b, 210c for delivering drive signals to different surgical instruments, such as, for example, an ultrasonic surgical instrument 104, an RF electrosurgical instrument 106, and a multifunction surgical instrument 108. In particular, drive signal outputs 210a, 210c may output an ultrasonic drive signal (e.g., a 420V root-mean-square [RMS] drive signal) to an ultrasonic surgical instrument 104, and drive signal outputs 210b, 210c may output an electrosurgical drive signal (e.g., a 100V RMS drive signal) to an RF electrosurgical instrument 106, with the drive signal output 2160b corresponding to the center tap of the power transformer 206.

In certain forms, the ultrasonic and electrosurgical drive signals may be provided simultaneously to distinct surgical instruments and/or to a single surgical instrument having the capability to deliver both ultrasonic and electrosurgical energy to tissue, such as the multifunction surgical instrument 108 (FIGS. 1-3). It will be appreciated that the electrosurgical signal, provided either to a dedicated electrosurgical instrument and/or to a combined multifunction ultrasonic/electrosurgical instrument may be either a therapeutic or sub-therapeutic level signal where the sub-therapeutic signal can be used, for example, to monitor tissue or instrument conditions and provide feedback to the generator. For example, the ultrasonic and RF signals can be delivered separately or simultaneously from a generator with a single output port in order to provide the desired output signal to the surgical instrument, as will be discussed in more detail below. Accordingly, the generator can combine the ultrasonic and electrosurgical RF energies and deliver the combined energies to the multifunction ultrasonic/electrosurgical instrument. Bipolar electrodes can be placed on one or both jaws of the end effector. One jaw may be driven by ultrasonic energy in addition to electrosurgical RF energy, working simultaneously. The ultrasonic energy may be employed to dissect tissue while the electrosurgical RF energy may be employed for vessel sealing.

The non-isolated stage 204 may comprise a power amplifier 212 having an output connected to a primary winding 214 of the power transformer 206. In certain forms the power amplifier 212 may be comprise a push-pull amplifier. For example, the non-isolated stage 204 may further comprise a logic device 216 for supplying a digital output to a digital-to-analog converter (DAC) circuit 218, which in turn supplies a corresponding analog signal to an input of the power amplifier 212. In certain forms the logic device 216 may comprise a programmable gate array (PGA), a field programmable gate array (FPGA), programmable logic device (PLD), among other logic circuits, for example. The logic device 216, by virtue of controlling the input of the power amplifier 212 via the DAC circuit 218, may therefore control any of a number of parameters (*e.g.*, frequency, waveform shape, waveform amplitude) of drive signals appearing at the drive signal outputs 210a, 210b, 210c. In certain forms and as discussed below, the logic device 216, in conjunction with a processor (e.g., a digital signal processor discussed below), may implement a number of digital signal processing (DSP)-based and/or other control algorithms to control parameters of the drive signals output by the generator 200.

Power may be supplied to a power rail of the power amplifier 212 by a switch-mode regulator 220, e.g., power converter. In certain forms the switch-mode regulator 220 may comprise an adjustable buck regulator, for example. The non-isolated stage 204 may further comprise a first processor 222, which in one form may comprise a DSP processor such as an Analog Devices ADSP-21469 SHARC DSP, available from Analog Devices, Norwood, MA, for example, although in various forms any suitable processor may be employed. In certain forms the DSP processor 222 may control operation of the switch-mode regulator 220 responsive to voltage feedback data received from the power amplifier 212 by the DSP processor 222 via an analog-to-digital converter (ADC) circuit 224. In one form, for example, the DSP processor 222 may receive as input, via the ADC circuit 224, the waveform envelope of a signal (*e.g.*, an RF signal) being amplified by the power amplifier 212. The DSP processor 222 may then control the switch-mode regulator 220 (*e.g.*, via a pulse-width modulated (PWM) output) such that the rail voltage supplied to the power amplifier 212 tracks the waveform envelope of the amplified signal. By dynamically modulating the rail voltage of the power amplifier 212 based on the waveform envelope, the efficiency of the power amplifier 212 may be significantly improved relative to a fixed rail voltage amplifier schemes.

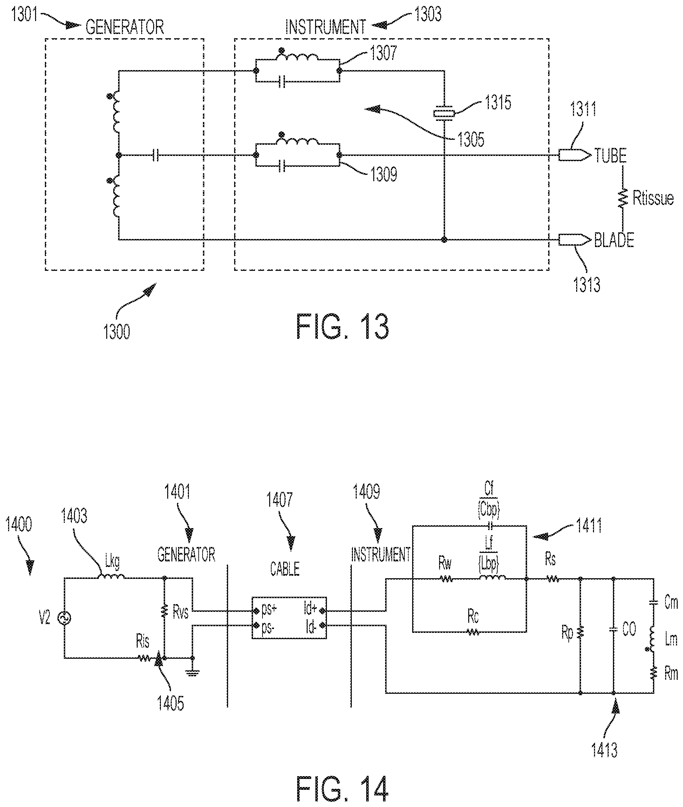

In certain forms, the logic device 216, in conjunction with the DSP processor 222, may implement a digital synthesis circuit such as a DDS (see e.g., FIGS. 13, 14) control scheme to control the waveform shape, frequency and/or amplitude of drive signals output by the generator 200. In one form, for example, the logic device 216 may implement a DDS control algorithm by recalling waveform samples stored in a dynamically-updated lookup table (LUT), such as a RAM LUT, which may be embedded in an FPGA. This control algorithm is particularly useful for ultrasonic applications in which an ultrasonic transducer, such as the ultrasonic transducer 120, may be driven by a clean sinusoidal current at its resonant frequency. Because other frequencies may excite parasitic resonances, minimizing or reducing the total distortion of the motional branch current may correspondingly minimize or reduce undesirable resonance effects. Because the waveform shape of a drive signal output by the generator 200 is impacted by various sources of distortion present in the output drive circuit (e.g., the power transformer 206, the power amplifier 212), voltage and current feedback data based on the drive signal may be input into an algorithm, such as an error control algorithm implemented by the DSP processor 222, which compensates for distortion by suitably pre-distorting or modifying the waveform samples stored in the LUT on a dynamic, ongoing basis (e.g., in real-time). In one form, the amount or degree of pre-distortion applied to the LUT samples may be based on the error between a computed motional branch current and a desired current waveform shape, with the error being determined on a sample-by-sample basis. In this way, the pre-distorted LUT samples, when processed through the drive circuit, may result in a motional branch drive signal having the desired waveform shape (e.g., sinusoidal) for optimally driving the ultrasonic transducer. In such forms, the LUT waveform samples will therefore not represent the desired waveform shape of the drive signal, but rather the waveform shape that is required to ultimately produce the desired waveform shape of the motional branch drive signal when distortion effects are taken into account.

The non-isolated stage 204 may further comprise a first ADC circuit 226 and a second ADC circuit 228 coupled to the output of the power transformer 206 via respective isolation transformers 230, 232 for respectively sampling the voltage and current of drive signals output by the generator 200. In certain forms, the ADC circuits 226, 228 may be configured to sample at high speeds (e.g., 80 mega samples per second [MSPS]) to enable oversampling of the drive signals. In one form, for example, the sampling speed of the ADC circuits 226, 228 may enable approximately 200x (depending on frequency) oversampling of the drive signals. In certain forms, the sampling operations of the ADC circuit 226, 228 may be performed by a single ADC circuit receiving input voltage and current signals via a two-way multiplexer. The use of highspeed sampling in forms of the generator 200 may enable, among other things, calculation of the complex current flowing through the motional branch (which may be used in certain forms to implement direct digital synthesis (DDS) based waveform shape control described above), accurate digital filtering of the sampled signals, and calculation of real power consumption with a high degree of precision. Voltage and current feedback data output by the ADC circuits 226, 228 may be received and processed (e.g., first-in-first-out [FIFO] buffer, multiplexer, etc.) by the logic device 216 and stored in data memory for subsequent retrieval by, for example, the DSP processor 222. As noted above, voltage and current feedback data may be used as input to an algorithm for pre-distorting or modifying LUT waveform samples on a dynamic and ongoing basis. In certain forms, this may require each stored voltage and current feedback data pair to be indexed based on, or otherwise associated with, a corresponding LUT sample that was output by the logic device 216 when the voltage and current feedback data pair was acquired. Synchronization of the LUT samples and the voltage and current feedback data in this manner contributes to the correct timing and stability of the pre-distortion algorithm.

In certain forms, the voltage and current feedback data may be used to control the frequency and/or amplitude (*e.g.*, current amplitude) of the drive signals. In one form, for example, voltage and current feedback data may be used to determine impedance phase. The frequency of the drive signal may then be controlled to minimize or reduce the difference between the determined impedance phase and an impedance phase setpoint (*e.g.*, 0°), thereby minimizing or reducing the effects of harmonic distortion and correspondingly enhancing impedance phase measurement accuracy. The determination of phase impedance and a frequency control signal may be implemented in the DSP processor 222, for example, with the frequency control signal being supplied as input to a DDS control algorithm implemented by the logic device 216.

In another form, for example, the current feedback data may be monitored in order to maintain the current amplitude of the drive signal at a current amplitude setpoint. The current amplitude setpoint may be specified directly or determined indirectly based on specified voltage amplitude and power setpoints. In certain forms, control of the current amplitude may be implemented by control algorithm, such as, for example, a proportional-integral-derivative (PID) control algorithm, in the DSP processor 222. Variables controlled by the control algorithm to suitably control the current amplitude of the drive signal may include, for example, the scaling of the LUT waveform samples stored in the logic device 216 and/or the full-scale output voltage of the DAC circuit 218 (which supplies the input to the power amplifier 212) via a DAC circuit 234.

The non-isolated stage 204 may further comprise a second processor 236 for providing, among other things user interface (UI) functionality. In one form, the UI processor 236 may comprise an Atmel AT91SAM9263 processor having an ARM 926EJ-S core, available from Atmel Corporation, San Jose, CA, for example. Examples of UI functionality supported by the UI processor 236 may include audible and visual user feedback, communication with peripheral devices (e.g., via a Universal Serial Bus [USB] interface), communication with the foot switch 130, communication with an input device 110 (e.g., a touch screen display) and communication with an output device 112 (e.g., a speaker), as shown in FIGS. 1 and 3. The UI processor 236 may communicate with the DSP processor 222 and the logic device 216 (e.g., via serial peripheral interface [SPI] buses). Although the UI processor 236 may primarily support UI functionality, it may also coordinate with the DSP processor 222 to implement hazard mitigation in certain forms. For example, the UI processor 236 may be programmed to monitor various aspects of user input and/or other inputs (e.g., touch screen inputs, foot switch 130 inputs as shown in FIG. 3, temperature sensor inputs) and may disable the drive output of the generator 200 when an erroneous condition is detected.

In certain forms, both the DSP processor 222 and the UI processor 236, for example, may determine and monitor the operating state of the generator 200. For the DSP processor 222, the operating state of the generator 200 may dictate, for example, which control and/or diagnostic processes are implemented by the DSP processor 222. For the UI processor 236, the operating state of the generator 200 may dictate, for example, which elements of a user interface (e.g., display screens, sounds) are presented to a user. The respective DSP and UI processors 222, 236 may independently maintain the current operating state of the generator 200 and recognize and evaluate possible transitions out of the current operating state. The DSP processor 222 may function as the master in this relationship and determine when transitions between operating states are to occur. The UI processor 236 may be aware of valid transitions between operating states and may confirm if a particular transition is appropriate. For example, when the DSP processor 222 instructs the UI processor 236 to transition to a specific state, the UI processor 236 may verify that requested transition is valid. In the event that a requested transition between states is determined to be invalid by the UI processor 236, the UI processor 236 may cause the generator 200 to enter a failure mode.

The non-isolated stage 204 may further comprise a controller 238 for monitoring input devices 110 (e.g., a capacitive touch sensor used for turning the generator 200 on and off, a capacitive touch screen). In certain forms, the controller 238 may comprise at least one processor and/or other controller device in communication with the UI processor 236. In one form, for example, the controller 238 may comprise a processor (e.g., a Mega168 8-bit controller available from Atmel) configured to monitor user input provided via one or more capacitive touch sensors. In one form, the controller 238 may comprise a touch screen controller (e.g., a QT5480 touch screen controller available from Atmel) to control and manage the acquisition of touch data from a capacitive touch screen.

In certain forms, when the generator 200 is in a "power off" state, the controller 238 may continue to receive operating power (e.g., via a line from a power supply of the generator 200, such as the power supply 254 discussed below). In this way, the controller 196 may continue to monitor an input device 110 (e.g., a capacitive touch sensor located on a front panel of the generator 200) for turning the generator 200 on and off. When the generator 200 is in the power off state, the controller 238 may wake the power supply (e.g., enable operation of one or more DC/DC voltage converters 256 of the power supply 254) if activation of the "on/off" input device 110 by a user is detected. The controller 238 may therefore initiate a sequence for transitioning the generator 200 to a "power on" state. Conversely, the controller 238 may initiate a sequence for transitioning the generator 200 to the power off state if activation of the "on/off" input device 110 is detected when the generator 200 is in the power on state. In certain forms, for example, the controller 238 may report activation of the "on/off" input device 110 to the UI processor 236, which in turn implements the necessary process sequence for transitioning the generator 200 to the power off state. In such forms, the controller 196 may have no independent ability for causing the removal of power from the generator 200 after its power on state has been established.

In certain forms, the controller 238 may cause the generator 200 to provide audible or other sensory feedback for alerting the user that a power on or power off sequence has been initiated. Such an alert may be provided at the beginning of a power on or power off sequence and prior to the commencement of other processes associated with the sequence.

In certain forms, the isolated stage 202 may comprise an instrument interface circuit 240 to, for example, provide a communication interface between a control circuit of a surgical instrument (e.g., a control circuit comprising handpiece switches) and components of the non-isolated stage 204, such as, for example, the logic device 216, the DSP processor 222 and/or the UI processor 236. The instrument interface circuit 240 may exchange information with components of the non-isolated stage 204 via a communication link that maintains a suitable degree of electrical isolation between the isolated and non-isolated stages 202, 204, such as, for example, an infrared (IR)-based communication link. Power may be supplied to the instrument interface circuit 240 using, for example, a low-dropout voltage regulator powered by an isolation transformer driven from the non-isolated stage 204.

In one form, the instrument interface circuit 240 may comprise a logic circuit 242 (*e.g.,* logic circuit, programmable logic circuit, PGA, FPGA, PLD) in communication with a signal conditioning circuit 244. The signal conditioning circuit 244 may be configured to receive a periodic signal from the logic circuit 242 (*e.g.,* a 2 kHz square wave) to generate a bipolar interrogation signal having an identical frequency. The interrogation signal may be generated, for example, using a bipolar current source fed by a differential amplifier. The interrogation signal may be communicated to a surgical instrument control circuit (*e.g.*, by using a conductive pair in a cable that connects the generator 200 to the surgical instrument) and monitored to determine a state or configuration of the control circuit. The control circuit may comprise a number of switches, resistors and/or diodes to modify one or more characteristics (*e.g.,* amplitude, rectification) of the interrogation signal such that a state or configuration of the control circuit is uniquely discernable based on the one or more characteristics. In one form, for example, the signal conditioning circuit 244 may comprise an ADC circuit for generating samples of a voltage signal appearing across inputs of the control circuit resulting from passage of interrogation signal therethrough. The logic circuit 242 (or a component of the non-isolated stage 204) may then determine the state or configuration of the control circuit based on the ADC circuit samples.

In one form, the instrument interface circuit 240 may comprise a first data circuit interface 246 to enable information exchange between the logic circuit 242 (or other element of the instrument interface circuit 240) and a first data circuit disposed in or otherwise associated with a surgical instrument. In certain forms, for example, a first data circuit 136 (FIG. 2) may be disposed in a cable integrally attached to a surgical instrument handpiece, or in an adaptor for interfacing a specific surgical instrument type or model with the generator 200. The first data circuit 136 may be implemented in any suitable manner and may communicate with the generator according to any suitable protocol including, for example, as described herein with respect to the first data circuit 136. In certain forms, the first data circuit may comprise a nonvolatile storage device, such as an EEPROM device. In certain forms and referring again to FIG. 5, the first data circuit interface 246 may be implemented separately from the logic circuit 242 and comprise suitable circuitry (*e.g.,* discrete logic devices, a processor) to enable communication between the logic circuit 242 and the first data circuit. In other forms, the first data circuit interface 246 may be integral with the logic circuit 242.

In certain forms, the first data circuit 136 *FIG. 2) may store information pertaining to the particular surgical instrument with which it is associated. Such information may include, for example, a model number, a serial number, a number of operations in which the surgical instrument has been used, and/or any other type of information. This information may be read by the instrument interface circuit 240 (*e.g.,* by the logic circuit 242), transferred to a component of the non-isolated stage 204 (*e.g.,* to logic device 216, DSP processor 222 and/or UI processor 236) for presentation to a user via an output device 112 (FIGS. 1 and 3) and/or for controlling a function or operation of the generator 200. Additionally, any type of information may be communicated to first data circuit 136 for storage therein via the first data circuit interface 246 (*e.g.,* using the logic circuit 242). Such information may comprise, for example, an updated number of operations in which the surgical instrument has been used and/or dates and/or times of its usage.

As discussed previously, a surgical instrument may be detachable from a handpiece (*e.g.,* the multifunction surgical instrument 108 may be detachable from the handpiece 109) to promote instrument interchangeability and/or disposability. In such cases, conventional generators may be limited in their ability to recognize particular instrument configurations being used and to optimize control and diagnostic processes accordingly. The addition of readable data circuits to surgical instruments to address this issue is problematic from a compatibility standpoint, however. For example, designing a surgical instrument to remain backwardly compatible with generators that lack the requisite data reading functionality may be impractical due to, for example, differing signal schemes, configuration complexity, and cost. Forms of instruments discussed herein address these concerns by using data circuits that may be implemented in existing surgical instruments economically and with minimal configuration changes to preserve compatibility of the surgical instruments with current generator platforms.

Additionally, forms of the generator 200 may enable communication with instrument-based data circuits. For example, the generator 200 may be configured to communicate with a second data circuit 138 (FIG. 2) contained in an instrument (*e.g.,* the multifunction surgical instrument 108 shown in FIG. 2). In some forms, the second data circuit 138 may be implemented in a many similar to that of the first data circuit 136 (FIG. 2) described herein. The instrument interface circuit 240 may comprise a second data circuit interface 248 to enable this communication. In one form, the second data circuit interface 248 may comprise a tri-state digital interface, although other interfaces may also be used. In certain forms, the second data circuit may generally be any circuit for transmitting and/or receiving data. In one form, for example, the second data circuit may store information pertaining to the particular surgical instrument with which it is associated. Such information may include, for example, a model number, a serial number, a number of operations in which the surgical instrument has been used, and/or any other type of information.

In some forms, the second data circuit 138 (FIG. 2) may store information about the electrical and/or ultrasonic properties of an associated ultrasonic transducer 120, end effector 125, or ultrasonic drive system. For example, the first data circuit 136 (FIG. 2) may indicate a burn-in frequency slope, as described herein. Additionally or alternatively, any type of information may be communicated to second data circuit for storage therein via the second data circuit interface 248 (*e.g.,* using the logic circuit 242). Such information may comprise, for example, an updated number of operations in which the instrument has been used and/or dates and/or times of its usage. In certain forms, the second data circuit may transmit data acquired by one or more sensors (*e.g.,* an instrument-based temperature sensor). In certain forms, the second data circuit may receive data from the generator 200 and provide an indication to a user (*e.g.,* an LED indication or other visible indication) based on the received data.

In certain forms, the second data circuit and the second data circuit interface 248 may be configured such that communication between the logic circuit 242 and the second data circuit can be effected without the need to provide additional conductors for this purpose (*e.g.,* dedicated conductors of a cable connecting a handpiece to the generator 200). In one form, for example, information may be communicated to and from the second data circuit using a 1-wire bus communication scheme implemented on existing cabling, such as one of the conductors used transmit interrogation signals from the signal conditioning circuit 244 to a control circuit in a handpiece. In this way, configuration changes or modifications to the surgical instrument that might otherwise be necessary are minimized or reduced. Moreover, because different types of communications implemented over a common physical channel can be frequency-band separated, the presence of a second data circuit may be "invisible" to generators that do not have the requisite data reading functionality, thus enabling backward compatibility of the surgical instrument.

In certain forms, the isolated stage 202 may comprise at least one blocking capacitor 250-1 connected to the drive signal output 210b to prevent passage of DC current to a patient. A single blocking capacitor may be required to comply with medical regulations or standards, for example. While failure in single-capacitor configurations is relatively uncommon, such failure may nonetheless have negative consequences. In one form, a second blocking capacitor 250-2 may be provided in series with the blocking capacitor 250-1, with current leakage from a point between the blocking capacitors 250-1, 250-2 being monitored by, for example, an ADC circuit 252 for sampling a voltage induced by leakage current. The samples may be received by the logic circuit 242, for example. Based changes in the leakage current (as indicated by the voltage samples in the form of FIG. 5), the generator 200 may determine when at least one of the blocking capacitors 250-1, 250-2 has failed. Accordingly, the form of FIG. 5 provides a benefit over single-capacitor configurations having a single point of failure.

In certain forms, the non-isolated stage 204 may comprise a power supply 254 for delivering DC power at a suitable voltage and current. The power supply may comprise, for example, a 400 W power supply for delivering a 48 VDC system voltage. The power supply 254 may further comprise one or more DC/DC voltage converters 256 for receiving the output of the power supply to generate DC outputs at the voltages and currents required by the various components of the generator 200. As discussed above in connection with the controller 238, one or more of the DC/DC voltage converters 256 may receive an input from the controller 238 when activation of the "on/off" input device 110 by a user is detected by the controller 238 to enable operation of, or wake, the DC/DC voltage converters 256.

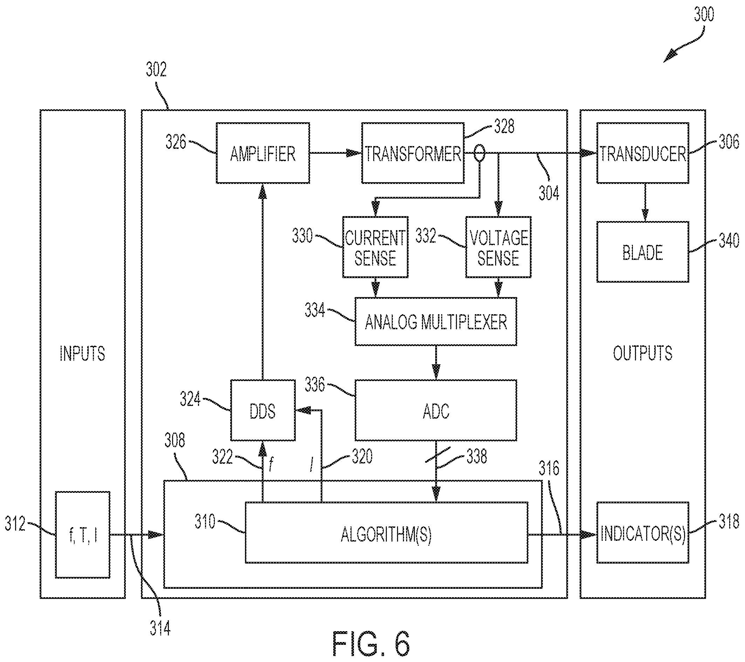

FIG. 6 illustrates one form of a drive system 302 of a generator 300, which is one form of the generator 100 (FIGS. 1-3). The generator 300 is configured to provide an ultrasonic electrical signal for driving an ultrasonic transducer (e.g., ultrasonic transducer 120 FIGS. 1-3), also referred to as a drive signal. The generator 300 is similar to and may be interchangeable with the generators 100, 200 (FIGS, 1-3 and 5). The drive system 302 is flexible and can create an ultrasonic electrical drive signal 304 at a desired frequency and power level setting for driving the ultrasonic transducer 306. In various forms, the generator 300 may comprise several separate functional elements, such as modules and/or blocks. Although certain modules and/or blocks may be described by way of example, it can be appreciated that a greater or lesser number of modules and/or blocks may be used and still fall within the scope of the forms. Further, although various forms may be described in terms of modules and/or blocks to facilitate description, such modules and/or blocks may be implemented by one or more hardware components, *e.g.*, processors, Digital Signal Processors (DSPs), Programmable Logic Devices (PLDs), Application Specific Integrated Circuits (ASICs), circuits, registers and/or software components, *e.g.*, programs, subroutines, logic and/or combinations of hardware and software components.

In one form, the generator 300 drive system 302 may comprise one or more embedded applications implemented as firmware, software, hardware, or any combination thereof. The generator 300 drive system 302 may comprise various executable modules such as software, programs, data, drivers, application program interfaces (APIs), and so forth. The firmware may be stored in nonvolatile memory (NVM), such as in bit-masked read-only memory (ROM) or flash memory. In various implementations, storing the firmware in ROM may preserve flash memory. The NVM may comprise other types of memory including, for example, programmable ROM (PROM), erasable programmable ROM (EPROM), EEPROM, or battery backed random-access memory (RAM) such as dynamic RAM (DRAM), Double-Data-Rate DRAM (DDRAM), and/or synchronous DRAM (SDRAM).

In one form, the generator 300 drive system 302 comprises a hardware component implemented as a processor 308 for executing program instructions for monitoring various measurable characteristics of the ultrasonic surgical instrument 104 (FIG. 1) and generating an output signal for driving the ultrasonic transducer in cutting and/or coagulation operating modes. It will be appreciated by those skilled in the art that the generator 300 and the drive system 302 may comprise additional or fewer components and only a simplified version of the generator 300 and the drive system 302 are described herein for conciseness and clarity. In various forms, as previously discussed, the hardware component may be implemented as a DSP, PLD, ASIC, circuits, and/or registers. In one form, the processor 308 may be configured to store and execute computer software program instructions to generate the output signals for driving various components of the ultrasonic surgical instrument 104, such as a transducer, an end effector, and/or a blade.

In one form, under control of one or more software program routines, the processor 308 executes the methods in accordance with the described forms to generate an electrical signal output waveform comprising current (I), voltage (V), and/or frequency (f) for various time intervals or periods (T). The stepwise waveforms of the drive signals may be generated by forming a piecewise linear combination of constant functions over a plurality of time intervals created by stepping the generator 300 drive signals, *e.g.,* output drive current (I), voltage (V), and/or frequency (f). The time intervals or periods (T) may be predetermined (*e.g.,* fixed and/or programmed by the user) or may be variable. Variable time intervals may be defined by setting the drive signal to a first value and maintaining the drive signal at that value until a change is detected in a monitored characteristic. Examples of monitored characteristics may comprise, for example, transducer impedance, tissue impedance, tissue heating, tissue transection, tissue coagulation, and the like. The ultrasonic drive signals generated by the generator 300 include, without limitation, ultrasonic drive signals capable of exciting the ultrasonic transducer 306 in various vibratory modes such as, for example, the primary longitudinal mode and harmonics thereof as well flexural and torsional vibratory modes.

In one form, the executable modules comprise one or more algorithm(s) 310 stored in memory that when executed causes the processor 308 to generate an electrical signal output waveform comprising current (I), voltage (V), and/or frequency (f) for various time intervals or periods (T). The stepwise waveforms of the drive signals may be generated by forming a piecewise linear combination of constant functions over two or more time intervals created by stepping the output drive current (I), voltage (V), and/or frequency (f) of the generator 300. The drive signals may be generated either for predetermined fixed time intervals or periods (T) of time or variable time intervals or periods of time in accordance with the one or more algorithm(s) 310. Under control of the processor 308, the generator 100 outputs (*e.g.,* increases or decreases) the current (I), voltage (V), and/or frequency (f) up or down at a particular resolution for a predetermined period (T) or until a predetermined condition is detected, such as a change in a monitored characteristic (*e.g.,* transducer impedance, tissue impedance). The steps can change in programmed increments or decrements. If other steps are desired, the generator 300 can increase or decrease the step adaptively based on measured system characteristics.

In operation, the user can program the operation of the generator 300 using the input device 312 located on the front panel of the generator 300 console. The input device 312 may comprise any suitable device that generates signals 314 that can be applied to the processor 308 to control the operation of the generator 300. In various forms, the input device 312 includes buttons, switches, thumbwheels, keyboard, keypad, touch screen monitor, pointing device, remote connection to a general purpose or dedicated computer. In other forms, the input device 312 may comprise a suitable user interface. Accordingly, by way of the input device 312, the user can set or program the current (I), voltage (V), frequency (f), and/or period (T) for programming the output of the generator 300. The processor 308 then displays the selected power level by sending a signal on line 316 to an output indicator 318.

In various forms, the output indicator 318 may provide visual, audible, and/or tactile feedback to the surgeon to indicate the status of a surgical procedure, such as, for example, when tissue cutting and coagulating is complete based on a measured characteristic of the ultrasonic surgical instrument 104, *e.g.,* transducer impedance, tissue impedance, or other measurements as subsequently described. By way of example, and not limitation, visual feedback comprises any type of visual indication device including incandescent lamps or LEDs, graphical user interface, display, analog indicator, digital indicator, bar graph display, digital alphanumeric display. By way of example, and not limitation, audible feedback comprises any type of buzzer, computer generated tone, computerized speech, voice user interface (VUI) to interact with computers through a voice/speech platform. By way of example, and not limitation, tactile feedback comprises any type of vibratory feedback provided through an instrument housing handle assembly.

In one form, the processor 308 may be configured or programmed to generate a digital current signal 320 and a digital frequency signal 322. These digital signals 320, 322 are applied to a digital synthesis circuit such as the DDS circuit 324 (see e.g., FIGS. 13, 14) to adjust the amplitude and the frequency (f) of the ultrasonic electrical drive signal 304 to the transducer. The output of the DDS circuit 324 is applied to a power amplifier 326 whose output is applied to a transformer 328. The output of the transformer 328 is the ultrasonic electrical drive signal 304 applied to the ultrasonic transducer 306, which is coupled to a blade by way of a waveguide. The output of the DDS circuit 324 may be stored in one more memory circuits including volatile (RAM) and non-volatile (ROM) memory circuits.

In one form, the generator 300 comprises one or more measurement modules or components that may be configured to monitor measurable characteristics of the ultrasonic instrument 104 (FIGS. 1, 2) or the multifunction electrosurgical/ultrasonic instrument 108 (FIGS.1-3). In the illustrated form, the processor 308 may be employed to monitor and calculate system characteristics. As shown, the processor 308 measures the impedance Z of the transducer by monitoring the current supplied to the ultrasonic transducer 306 and the voltage applied to the transducer. In one form, a current sense circuit 330 is employed to sense the current flowing through the transducer and a voltage sense circuit 332 is employed to sense the output voltage applied to the ultrasonic transducer 306. These signals may be applied to the ADC circuit 336 via an analog multiplexer 334 circuit or switching circuit arrangement. The analog multiplexer 334 routes the appropriate analog signal to the ADC circuit 336 for conversion. In other forms, multiple ADC circuits 336 may be employed for each measured characteristic instead of the analog multiplexer 334 circuit. The processor 308 receives the digital output 338 of the ADC circuit 336 and calculates the transducer impedance Z based on the measured values of current and voltage. The processor 308 adjusts the ultrasonic electrical drive signal 304 such that it can generate a desired power versus load curve. In accordance with programmed algorithm(s) 310, the processor 308 can step the ultrasonic electrical drive signal 304, *e.g.,* the current or frequency, in any suitable increment or decrement in response to the transducer impedance Z.

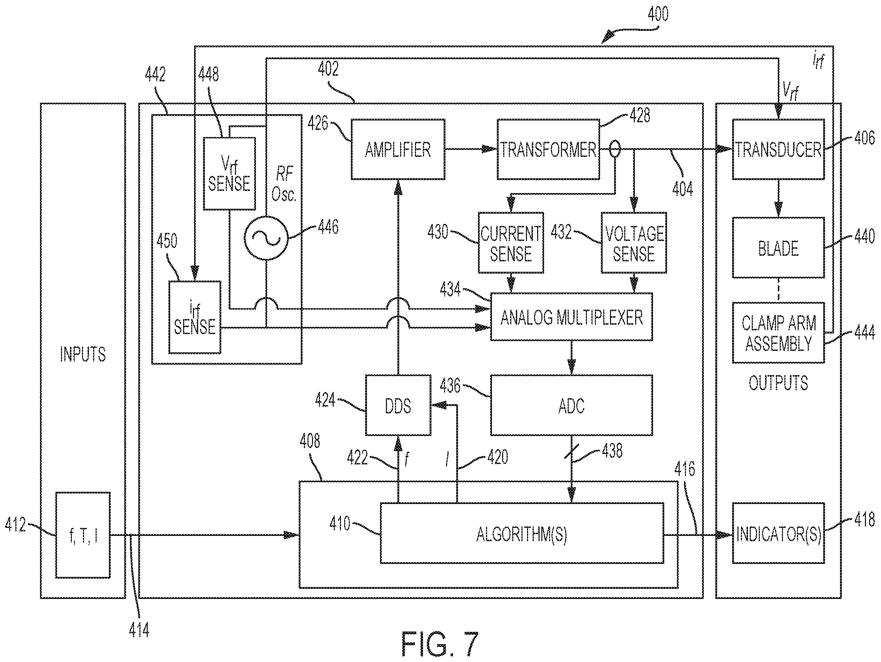

FIG. 7 illustrates one aspect of a drive system 402 of the generator 400, which is one form of the generator 100 (FIGS. 1-3). In operation, the user can program the operation of the generator 400 using the input device 412 located on the front panel of the generator 400 console. The input device 412 may comprise any suitable device that generates signals 414 that can be applied to the processor 408 to control the operation of the generator 400. In various forms, the input device 412 includes buttons, switches, thumbwheels, keyboard, keypad, touch screen monitor, pointing device, remote connection to a general purpose or dedicated computer. In other forms, the input device 412 may comprise a suitable user interface. Accordingly, by way of the input device 412, the user can set or program the current (I), voltage (V), frequency (f), and/or period (T) for programming the output of the generator 400. The processor 408 then displays the selected power level by sending a signal on line 416 to an output indicator 418.

The generator 400 comprises a tissue impedance module 442. The drive system 402 is configured to generate electrical drive signal 404 to drive the ultrasonic transducer 406. In one aspect, the tissue impedance module 442 may be configured to measure the impedance Zt of tissue grasped between the blade 440 and the clamp arm assembly 444. The tissue impedance module 442 comprises an RF oscillator 446, an RF voltage sensing circuit 448, and an RF current sensing circuit 450. The RF voltage and RF current sensing circuits 448, 450 respond to the RF voltage Vrf applied to the blade 440 electrode and the RF current irf flowing through the blade 440 electrode, the tissue, and the conductive portion of the clamp arm assembly 444. The sensed voltage Vrf and current Irf are converted to digital form by the ADC circuit 436 via the analog multiplexer 434. The processor 408 receives the digital output 438 of the ADC circuit 436 and determines the tissue impedance Zt by calculating the ratio of the RF voltage Vrf to current Irf measured by the RF voltage sense circuit 448 and the RF current sense circuit 450. In one aspect, the transection of the inner muscle layer and the tissue may be detected by sensing the tissue impedance Zt. Accordingly, detection of the tissue impedance Zt may be integrated with an automated process for separating the inner muscle layer from the outer adventitia layer prior to transecting the tissue without causing a significant amount of heating, which normally occurs at resonance.

In one form, the RF voltage Vrf applied to the blade 440 electrode and the RF current Irf flowing through the blade 440 electrode, the tissue, and the conductive portion of the clamp arm assembly 451 are suitable for vessel sealing and//or dissecting. Thus, the RF power output of the generator 400 can be selected for non-therapeutic functions such as tissue impedance measurements as well as therapeutic functions such as vessel sealing and/or dissection. It will be appreciated, that in the context of the present disclosure, the ultrasonic and the RF electrosurgical energies can be supplied by the generator either individually or simultaneously.

In various forms, feedback is provided by the output indicator 418 shown in FIGS. 6 and 7. The output indicator 418 is particularly useful in applications where the tissue being manipulated by the end effector is out of the user's field of view and the user cannot see when a change of state occurs in the tissue. The output indicator 418 communicates to the user that a change in tissue state has occurred. As previously discussed, the output indicator 418 may be configured to provide various types of feedback to the user including, without limitation, visual, audible, and/or tactile feedback to indicate to the user (e.g., surgeon, clinician) that the tissue has undergone a change of state or condition of the tissue. By way of example, and not limitation, as previously discussed, visual feedback comprises any type of visual indication device including incandescent lamps or LEDs, graphical user interface, display, analog indicator, digital indicator, bar graph display, digital alphanumeric display. By way of example, and not limitation, audible feedback comprises any type of buzzer, computer generated tone, computerized speech, VUI to interact with computers through a voice/speech platform. By way of example, and not limitation, tactile feedback comprises any type of vibratory feedback provided through the instrument housing handle assembly. The change of state of the tissue may be determined based on transducer and tissue impedance measurements as previously described, or based on voltage, current, and frequency measurements.

In one form, the processor 408 may be configured or programmed to generate a digital current signal 420 and a digital frequency signal 422. These digital signals 420, 422 are applied to a digital synthesis circuit such as the DDS circuit 424 (see e.g., FIGS. 13, 14) to adjust the amplitude and the frequency (f) of the electrical drive signal 404 to the transducer 406. The output of the DDS circuit 424 is applied to a power amplifier 426 whose output is applied to a transformer 428. The output of the transformer 428 is the electrical drive signal 404 applied to the ultrasonic transducer 406, which is coupled to a blade by way of a waveguide. The output of the DDS circuit 424 may be stored in one more memory circuits including volatile (RAM) and non-volatile (ROM) memory circuits.

In one form, the generator 400 comprises one or more measurement modules or components that may be configured to monitor measurable characteristics of the ultrasonic instrument 104 (FIGS. 1, 3) or the multifunction electrosurgical/ultrasonic instrument 108 (FIGS.1-3). In the illustrated form, the processor 408 may be employed to monitor and calculate system characteristics. As shown, the processor 408 measures the impedance Z of the transducer by monitoring the current supplied to the ultrasonic transducer 406 and the voltage applied to the transducer. In one form, a current sense circuit 430 is employed to sense the current flowing through the transducer and a voltage sense circuit 432 is employed to sense the output voltage applied to the ultrasonic transducer 406. These signals may be applied to the ADC circuit 436 via an analog multiplexer 434 circuit or switching circuit arrangement. The analog multiplexer 434 routes the appropriate analog signal to the ADC circuit 436 for conversion. In other forms, multiple ADC circuits 436 may be employed for each measured characteristic instead of the analog multiplexer 434 circuit. The processor 408 receives the digital output 438 of the ADC circuit 436 and calculates the transducer impedance Z based on the measured values of current and voltage. The processor 308 adjusts the electrical drive signal 404 such that it can generate a desired power versus load curve. In accordance with programmed algorithm(s) 410, the processor 408 can step the ultrasonic electrical drive signal 404, *e.g.,* the current or frequency, in any suitable increment or decrement in response to the transducer impedance Z.

With reference to FIGS. 6 and 7, in various forms, the various executable instructions or modules (*e.g.,* algorithms 310, 410) comprising computer readable instructions can be executed by the processor 308, 408 portion of the generator 300, 400. In various forms, the operations described with respect to the algorithms may be implemented as one or more software components, *e.g.,* programs, subroutines, logic; one or more hardware components, *e.g.,* processors, DSPs, PLDs, ASICs, circuits, registers; and/or combinations of software and hardware. In one form, the executable instructions to perform the algorithms may be stored in memory. When executed, the instructions cause the processor 308, 408 to determine a change in tissue state provide feedback to the user by way of the output indicator 318, 418. In accordance with such executable instructions, the processor 308, 408 monitors and evaluates the voltage, current, and/or frequency signal samples available from the generator 300, 400 and according to the evaluation of such signal samples determines whether a change in tissue state has occurred. As further described below, a change in tissue state may be determined based on the type of ultrasonic instrument and the power level that the instrument is energized at. In response to the feedback, the operational mode of the surgical instruments 104, 106, 108 (FIGS. 1-3) may be controlled by the user or may be automatically or semi-automatically controlled.

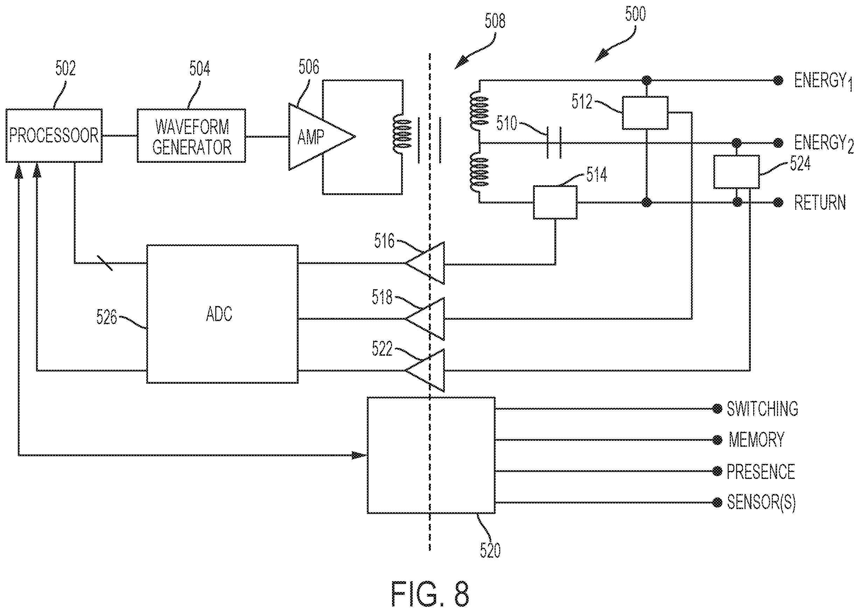

FIG. 8 illustrates an example of a generator 500, which is one form of the generator 100 (FIGS. 1-3). The generator 500 is configured to deliver multiple energy modalities to a surgical instrument. The generator 500 includes functionalities of the generators 200, 300, 400 shown in FIGS. 5-7. The generator 500 provides RF and ultrasonic signals for delivering energy to a surgical instrument. The RF and ultrasonic signals may be provided alone or in combination and may be provided simultaneously. As noted above, at least one generator output can deliver multiple energy modalities (e.g., ultrasonic, bipolar or monopolar RF, irreversible and/or reversible electroporation, and/or microwave energy, among others) through a single port and these signals can be delivered separately or simultaneously to the end effector to treat tissue. The generator 500 comprises a processor 502 coupled to a waveform generator 504. The processor 502 and waveform generator 504 are configured to generate a variety of signal waveforms based on information stored in a memory coupled to the processor 502, not shown for clarity of disclosure. The digital information associated with a waveform is provided to the waveform generator 504 which includes one or more DAC circuits to convert the digital input into an analog output. The analog output is fed to an amplifier 1106 for signal conditioning and amplification. The conditioned and amplified output of the amplifier 506 is coupled to a power transformer 508. The signals are coupled across the power transformer 508 to the secondary side, which is in the patient isolation side. A first signal of a first energy modality is provided to the surgical instrument between the terminals labeled ENERGY1 and RETURN. A second signal of a second energy modality is coupled across a capacitor 510 and is provided to the surgical instrument between the terminals labeled ENERGY2 and RETURN. It will be appreciated that more than two energy modalities may be output and thus the subscript "n" may be used to designate that up to n ENERGYn terminals may be provided, where n is a positive integer greater than 1. It also will be appreciated that up to "n" return paths RETURNn may be provided without departing from the scope of the present disclosure.

A first voltage sensing circuit 512 is coupled across the terminals labeled ENERGY1 and the RETURN path to measure the output voltage therebetween. A second voltage sensing circuit 524 is coupled across the terminals labeled ENERGY2 and the RETURN path to measure the output voltage therebetween. A current sensing circuit 514 is disposed in series with the RETURN leg of the secondary side of the power transformer 508 as shown to measure the output current for either energy modality. If different return paths are provided for each energy modality, then a separate current sensing circuit should be provided in each return leg. The outputs of the first and second voltage sensing circuits 512, 524 are provided to respective isolation transformers 516, 522 and the output of the current sensing circuit 514 is provided to another isolation transformer 518. The outputs of the isolation transformers 516, 518, 522 in the on the primary side of the power transformer 508 (non-patient-isolated side) are provided to a one or more ADC circuit 526. The digitized output of the ADC circuit 526 is provided to the processor 502 for further processing and computation. The output voltages and output current feedback information can be employed to adjust the output voltage and current provided to the surgical instrument and to compute output impedance, among other parameters. Input/output communications between the processor 502 and patient isolated circuits is provided through an interface circuit 520. Sensors also may be in electrical communication with the processor 502 by way of the interface circuit 520.

In one aspect, the impedance may be determined by the processor 502 by dividing the output of either the first voltage sensing circuit 512 coupled across the terminals labeled ENERGY1/RETURN or the second voltage sensing circuit 524 coupled across the terminals labeled ENERGY2/RETURN by the output of the current sensing circuit 514 disposed in series with the RETURN leg of the secondary side of the power transformer 508. The outputs of the first and second voltage sensing circuits 512, 524 are provided to separate isolations transformers 516, 522 and the output of the current sensing circuit 514 is provided to another isolation transformer 516. The digitized voltage and current sensing measurements from the ADC circuit 526 are provided the processor 502 for computing impedance. As an example, the first energy modality ENERGY1 may be ultrasonic energy and the second energy modality ENERGY2 may be RF energy. Nevertheless, in addition to ultrasonic and bipolar or monopolar RF energy modalities, other energy modalities include irreversible and/or reversible electroporation and/or microwave energy, among others. Also, although the example illustrated in FIG. 8 shows a single return path RETURN may be provided for two or more energy modalities, in other aspects multiple return paths RETURNn may be provided for each energy modality ENERGYn. Thus, as described herein, the ultrasonic transducer impedance may be measured by dividing the output of the first voltage sensing circuit 512 by the current sensing circuit 514 and the tissue impedance may be measured by dividing the output of the second voltage sensing circuit 524 by the current sensing circuit 514.

As shown in FIG. 8, the generator 500 comprising at least one output port can include a power transformer 508 with a single output and with multiple taps to provide power in the form of one or more energy modalities, such as ultrasonic, bipolar or monopolar RF, irreversible and/or reversible electroporation, and/or microwave energy, among others, for example, to the end effector depending on the type of treatment of tissue being performed. For example, the generator 500 can deliver energy with higher voltage and lower current to drive an ultrasonic transducer, with lower voltage and higher current to drive RF electrodes for sealing tissue, or with a coagulation waveform for spot coagulation using either monopolar or bipolar RF electrosurgical electrodes. The output waveform from the generator 500 can be steered, switched, or filtered to provide the frequency to the end effector of the surgical instrument. The connection of an ultrasonic transducer to the generator 500 output would be preferably located between the output labeled ENERGY1 and RETURN as shown in FIG. 8. An In one example, a connection of RF bipolar electrodes to the generator 500 output would be preferably located between the output labeled ENERGY2 and RETURN. In the case of monopolar output, the preferred connections would be active electrode (e.g., pencil or other probe) to the ENERGY2 output and a suitable return pad connected to the RETURN output.

In other aspects, the generators 100, 200, 300, 400, 500 described in connection with FIGS. 1-3 and 5-8, the ultrasonic drive circuit 114, and/or electrosurgery/RF drive circuit 116 as described in connection with FIG. 3 may be formed integrally with any one of the surgical instruments 104, 106, 108 described in connection with FIGS. 1 and 2. Accordingly, any of the processors, digital signal processors, circuits, controllers, logic devices, ADCs, DACs, amplifiers, converters, transformers, signal conditioners, data interface circuits, current and voltage sensing circuits, direct digital synthesis circuits, multiplexer (analog or digital), waveform generators, RF generators, memory, and the like, described in connection with any one of the generators 100, 200, 300, 400, 500 can be located within the surgical instruments 104, 106, 108 or may be located remotely from the surgical instruments 104, 106, 108 and coupled to the surgical instruments via wired and/or wireless electrical connections.

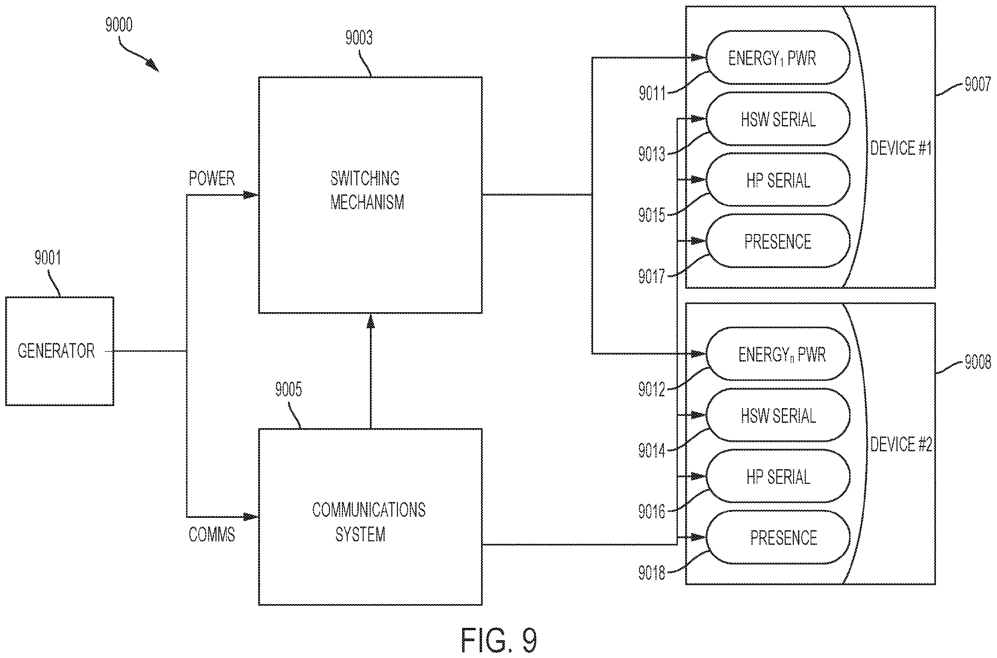

FIG. 9 shows a diagram of an electrosurgical system 9000 that allows for two ports on a generator 9001 and accounts for electrical isolation between two surgical instruments 9007, 9008. A scheme is provided for electrical isolation between the two instruments 9007, 9008 as they are located on the same patient isolation circuit.

According to the configuration shown in FIG. 9, unintended electrical power feedback is prevented through the electrosurgical system 9000. In various aspects, power field effect transisotrs (FETs) or relays are used to electrically isolate all power lines for each of the two surgical instruments 9007, 9008. According to one aspect, the power FETs or relays are controlled by a 1-wire communication protocol.

As shown in FIG. 9, a generator 9001, which is one form of the generator 100 (FIGS. 1-3), is coupled to a power switching mechanism 9003 and a communications system 9005. In one aspect, the power switching mechanism 9003 comprises power solid state switches such as, for example, FET or MOSFET transistors, and/or relays, such as electromechanical relays. In one aspect, the communications system 9005 comprises components for D1 emulation, FPGA expansion, and time slicing functionalities. The power switching mechanism 9003 is coupled to the communications system 9005. Each of the power switching mechanism 9003 and the communications system 9005 are coupled to surgical instruments 9007, 9009 (labeled device 1 and device 2). Each of surgical instruments 9007, 9009 comprise components for a combined RF and ultrasonic energy input 9011, handswitch (HSW) 1-wire serial protocol interface 9013, HP 1-wire serial protocol interface 9015, and a presence resistor interface 9017. The power switching mechanism 9003 is coupled to the RF and Ultrasonic energy input 9011 for each of surgical instruments 9007, 9008. The communications system 9005 is coupled to the HSW 1-wire serial interface 9013, 9014, the HP 1-wire serial protocol interface 9015, 9016, and presence interface 9017, 9018 for each of surgical instruments 9007, 9008. While two surgical instruments are shown in FIG. 9, there may be more than two devices according to various aspects.

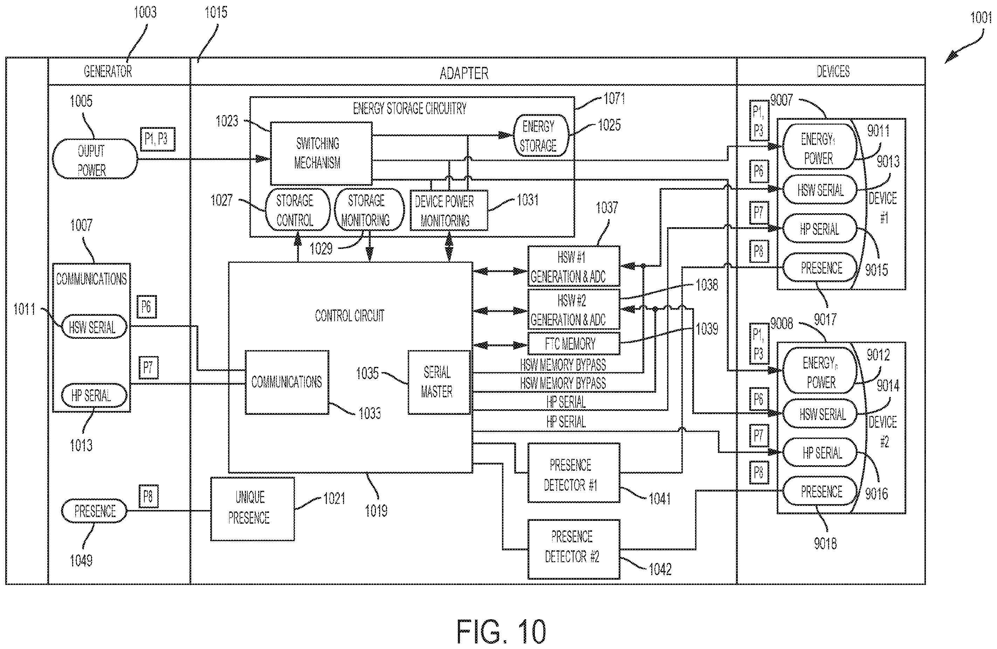

FIGS. 10-12 illustrate aspects of an interface with a generator to support two instruments simultaneously that allows the instruments to quickly switch between active/inactive by a user in a sterile field. FIGS. 10-12 describe multiple communication schemes which would allow for a super cap / battery charger and dual surgical instruments. The aspects of FIGS. 10-12 allow for communications to two surgical instruments in the surgical field from a generator with at least one communications port and allow for an operator in sterile field to switch between devices, for example, without modifying the surgical instruments.

FIG. 10 is a diagram of a communications architecture of system 1001 comprising a generator 1003, which is one form of the generator 100 (FIGS. 1-3), and surgical instruments 9007, 9008, which are shown in FIG. 9. According to FIG. 10, the generator 9001 is configured for delivering multiple energy modalities to a plurality of surgical instruments. As discussed herein the various energy modalities include, without limitation, ultrasonic, bipolar or monopolar RF, reversible and/or irreversible electroporation, and/or microwave energy modalities.

The generator 9001 comprises a combined energy modality power output 1005, a communications interface 1007, and a presence interface 1049. According to the aspect of FIG. 10, the communications interface 1007 comprises an handswitch (HSW) serial interface 1011 and an handpiece (HP) serial interface 1013. The serial interfaces 1011, 1013 may comprise interintegrated circuit (I$^2$C), half duplex SPI, and/or Universal Asynchronous Receiver Transmitter (UART) components and/or functionalities. The generator 1003 provides the combined energy modalities power output 1005 to an adapter 1015, for example, a pass-through charger (PTC). The adapter 1015 comprises energy storage circuit 1071, control circuit 1019, a unique presence element 1021, and associated circuit discussed below. In one aspect, the presence element 1021 is a resistor. In another aspect, the presence element 1021 may be a bar code, Quick Response (QR) code, or similar code, or a value stored in memory such as, for example, a value stored in NVM. The presence element 1021 may be unique to the adapter 1015 so that, in the event that another adapter that did not use the same wire interfaces could not be used with the unique presence element 1021. In one aspect, the unique presence element 1021 is a resistor. The energy storage circuit 1071 comprises a switching mechanism 1023, energy storage device 1025, storage control 1027, storage monitoring component 1029, and a device power monitoring component 1031. The control circuit 1019 may comprise a processor, FPGA, PLD, complex programmable logic device (CPLD), microcontroller, DSP, and/or ASIC, for example. According to the aspect shown in FIG. 10, an FPGA or microcontroller would act as an extension of an existing, similar computing hardware and allows for information to be relayed from on entity to another entity.

The switching mechanism 1023 is configured to receive the combined energy power output 1005 from the generator 1003 and it may be provided to the energy storage device 1025, surgical instrument 9007, and/or surgical instrument 9008. The device power monitoring component 1031 is coupled to the channels for the energy storage device 1025, surgical instrument 9007, surgical instrument 9008, and may monitor where power is flowing. The control circuit 1019 comprises communication interface 1033 coupled to the handswitch serial interface 1011 and an handpiece serial interface 1013 of the generator 1003. The control circuit 1019 is also coupled to the storage control 1027, storage monitoring component 1029, and device power monitoring component 1031 of the energy storage circuit 1071.

The control circuit 1019 further comprises a serial master interface 1035 that is coupled to handswitch (HSW) #1 circuit 1037 and handswitch (HSW) #2 circuit 1038, includes generation and ADC, a form of memory (non volatile or flash) 1039, along with a method for detecting the presence of an attached instrument (Presence) #1 circuit 1041 and Presence #2 circuit 1042, which includes a voltage or current source and ADC. The serial master interface 1035 also includes handswitch NVM bypass channels, which couple the serial master interface 1035 to the outputs of the handswitch #1 circuit 1037 and the handswitch #2 circuit 1038, respectively. The handswitch #1 circuit 1037 and handswitch #2 circuit 1038 are coupled to the HSW 1-wire serial protocol interfaces 9013, 9014 of the surgical instruments 9007, 9008, respectively. The serial master interface 1035 further includes handpiece serial channels that are coupled to the HP 1-wire serial protocol interfaces 9015, 9016 of the surgical instruments 9007, 9008, respectively. Further, Presence #1 and Presence #2 circuits 1041, 1042 are coupled to the presence interfaces 9017, 9018 of the surgical instruments 9007, 9008, respectively.

The system 1001 allows the control circuit 1019, such as an FPGA, to communicate with more surgical instruments using adapter 1015, which acts as an expansion adapter device. According to various aspects, the adapter 1015 expands the Input/Output (I/O) capability of the generator 1003 control. The adapter 1015 may function as an extension of the central processing unit that allows commands to be transmitted over a bus between the adapter 1015 and the generator 1003 and unpacks the commands and use them to bit-bang over interfaces or to control connected analog circuit. The adapter 1015 also allows for reading in ADC values from connected surgical instruments 9007, 9008 and relay this information to the generator control and the generator control would then control the two surgical instruments 9007, 9008. According to various aspects, the generator 1003 may control the surgical instruments 9007, 9008 as two separate state machines and may store the data.

Existing interfaces (the handswitch serial interface 1011 and the handpiece serial interface 1013 lines from generator 1003) may be used in a two-wire communication protocol that enables the generator 1003 control to communicate with multiple surgical instruments connected to a dual port interface, similar to the topology of a universal serial bus (USB) hub. This allows interfacing with two separate surgical instruments simultaneously. The system 1001 may be able to generate and read hand switch waveforms and be able to handle incoming handpiece serial buses. It would also monitor two separate presence elements in the surgical instruments 9007, 9008. In one aspect, the system 1001 may include a unique presence element and may have its own NVM.

Further, according to various aspects, the control circuit 1019 may be controlled by the generator 1003. The communication between the adapter 1015 and connected surgical instruments 9007, 9008 may be relayed to generator control. The generator 1003 would control the waveform generation circuit connected to the adapter 1015 to simultaneously generate handswitch signals for surgical instruments 9007, 9008.

The system 1001 may allow surgical instrument activity that can be simultaneously detected/monitored for two surgical instruments, even during activation. If upgradeable, the adapter 1015 would be capable of handling new surgical instrument communications protocols. Further, fast switching between surgical instruments may be accomplished.

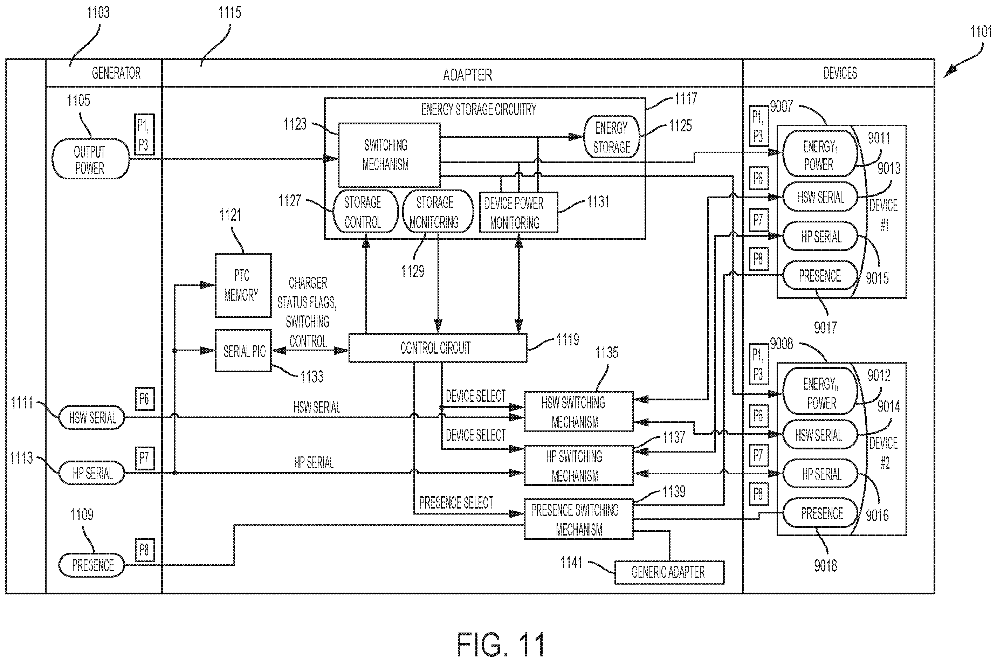

FIG. 11 illustrates a communication architecture of system 1101 of a generator 1103, which is one form of the generator 100 (FIGS. 1-3), and surgical instruments 9007, 9008 shown in FIG. 9. According to FIG. 11, the generator 1103 is configured for delivering multiple energy modalities to a plurality of surgical instruments. As discussed herein the various energy modalities include, without limitation, ultrasonic, bipolar or monopolar RF, reversible and/or irreversible electroporation, and/or microwave energy modalities. As shown in FIG. 11, the generator 1103 comprises a combined energy modality power output 1105, an handswitch (HSW) serial interface 1111, a handpiece (HP) serial interface 1113, and a presence interface 1109. The generator 1103 provides the power output 1105 to an adapter 1115. According to the aspect shown in FIG. 11, communications between the adapter 1115 and the generator 1103 may be done solely through serial interfaces, such as the handswitch serial and handpiece serial interfaces 1111, 1113. The generator 1103 may use these handswitch and handpiece serial interfaces 1111, 1113 to control which instrument the generator 1103 is communicating with. Further, switching between instruments could occur between handswitch frames or at a much slower rate.

The adapter 1115 comprises energy storage circuit 1117, control circuit 1119, an adapter memory 1121 (e.g., a NVM such as an EEPROM), a serial programmable input/output (PIO) integrated circuit 1133, an handswitch Switching Mechanism 1135, an handpiece Switching Mechanism 1137, a Presence Switching Mechanism 1139, and a Generic Adapter 1141. In one aspect, the serial PIO integrated circuit 1133 may be an addressable switch. The energy storage circuity 1117 comprises a switching mechanism 1123, energy storage device 1125, storage control component 1127, storage monitoring component 1129, and a device power monitoring component 1131. The control circuit 1119 may comprise a processor, FPGA, CPLD, PLD, microcontroller, DSP, and/or an ASIC, for example. According to the aspect of FIG. 11, an FPGA or microcontroller may have limited functionality and may solely comprise functionality for monitoring and communicating energy storage.

The switching mechanism 1123 is configured to receive the combined energy power output 1105 from the generator 1103 and it may be provided to the energy storage device 1125, surgical instrument 9007, and/or surgical instrument 9008. The device power monitoring component 1131 is coupled to the channels for the energy storage device 1125, surgical instrument 9007, surgical instrument 9008, and may monitor where power is flowing.

The control circuit 1119 is coupled to the serial PIO integrated circuit 1133 and the serial PIO integrated circuit 1133 is coupled to the handpiece serial interface 1113 of the generator 1103. The control circuit 1119 may receive information regarding charger status flags and switching controls from the serial PIO integrated circuit 1133. Further, the control circuit 1119 is coupled to the handswitch switching mechanism 1135, the handpiece switching mechanism 1137, and the presence switching mechanism 1139. According to the aspect of FIG. 11, the control circuit 1119 may be coupled to the handswitch (HSW) switching mechanism 1135 and the handpiece switching mechanism 1137 for device selection and the control circuit 1119 may be coupled to the presence switching Mechanism 1139 for presence selection.

The handswitch switching mechanism 1135, the handpiece switching mechanism 1137, and the presence switching mechanism 1139 are coupled to the handswitch serial interface 1111, the handpiece serial interface 1113, and the presence interface 1109 of generator 1103, respectively. Further, the handswitch switching mechanism 1135, the handpiece switching mechanism 1137, and the presence switching mechanism 1139 are coupled to the HSW 1-wire serial protocol interfaces 9013, 9014, the HP 1-wire serial protocol interfaces 9015, 9016, and the presence interfaces 9017, 9018 of the surgical instruments 9007, 9008, respectively. Further, the presence switching mechanism 1139 is coupled to the generic adapter 1141.

The generator 1103 switches between monitoring the surgical instruments 9007, 9008. According to various aspects, this switching may require the generator 1103 control to keep track of surgical instruments 9007, 9008 and run two separate state machines. The control circuit 1119 will need to remember which surgical instruments are connected, so that it can output an appropriate waveform to the ports where appropriate. The generator 1103 may generate/monitor hand switch signals, as well as communicating with serial NVM devices, such adapter memory 1121. The generator 1103 may maintain constant communication with the activating surgical instrument for the duration of the activation.

System 1101 also allows for a generic adapter presence element. When first plugged in or powered on, the adapter 1115 would present this adapter resistance to the generator 1103. The generator 1103 may then relay commands to the adapter 1115 to switch between the different presence elements corresponding to the different surgical instruments 9007, 9008 connected to it. Accordingly, the generator 1103 is able to use its existing presence resistance circuit. The NVM memory 1121 exists on the adapter 1115 for additional identification of the adapter and to provide a level of security. In addition, the adapter 1115 has a serial I/O device, i.e. serial PIO integrated circuit 1133. The serial PIO integrated circuit 1133 provides a communication link between the generator 1103 and the adapter 1115.

It may be possible to communicate over the handpiece serial bus using serial communications to handpiece NVMs and UART style communication to the control circuit 1119. According to one aspect, if SLOW serial communication is used (i.e. not overdrive) and a high speed serial protocol is used, system 1101 may need to ensure that the communications protocol does not generate a signal that looked like a serial reset pulse. This would allow better generator 1103 to adapter 1115 communications and faster switching times between surgical instruments 9007, 9008.

The system 1101 uses generator communications protocol and analog circuit and allows the generator to accomplish decision making. It is a simple and efficient solution that uses a small number of circuit devices.

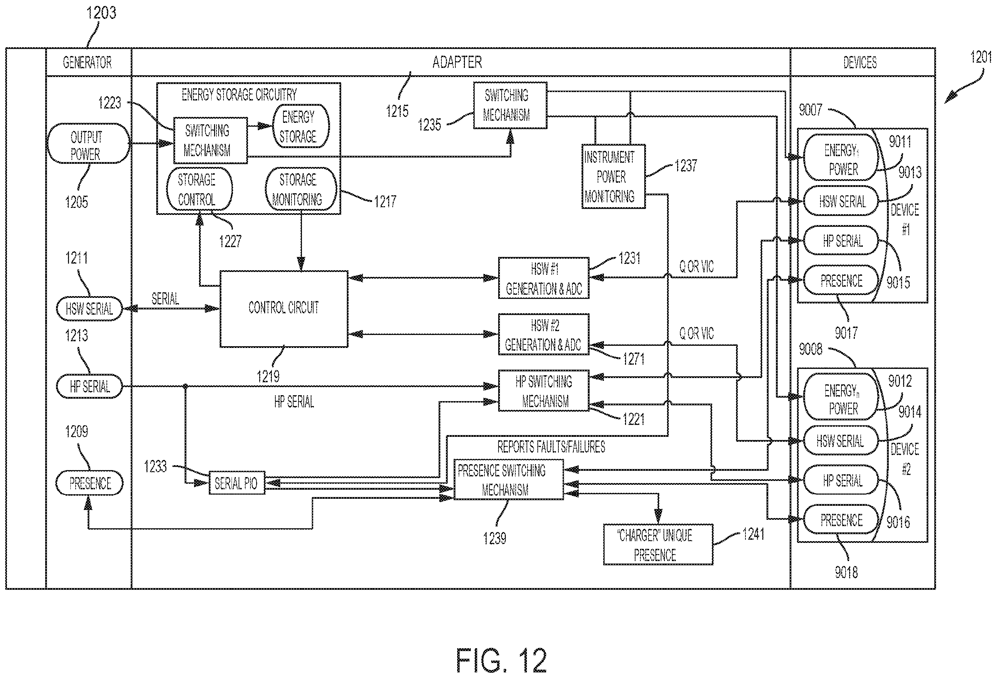

FIG. 12 illustrates a communications architecture of system 1201 of a generator 1203, which is one form of the generator 100 (FIGS. 1-3), and surgical instruments 9007, 9008 shown in FIG. 9. According to FIG. 12, the generator 1203 is configured for delivering multiple energy modalities to a plurality of surgical instruments. As discussed herein the various energy modalities include, without limitation, ultrasonic, bipolar or monopolar RF, reversible and/or irreversible electroporation, and/or microwave energy modalities. As shown in FIG. 12, the generator 1203 comprises a combined energy modality power output 1205, an handswitch serial interface 1211, an handpiece serial interface 1213, and a presence interface 1209. In one aspect, the handpiece serial interface 1213 allows for communication with the handpiece lines of the surgical instruments 9007, 9008 and also allows for control of the adapter 1215. The generator 1203 provides the combined energy modality power output 1205 to an adapter 1215. The adapter 1215 comprises energy storage circuit 1217, control circuit 1219, a serial PIO integrated circuit 1233, handswitch (HSW) #1 circuit 1231, handswitch (HSW) #2 circuit 1271, handpiece switching mechanism 1221, presence switching mechanism 1239, switching mechanism 1235, instrument power monitoring 1237, and unique presence 1241. As shown in FIG. 12, the handswitch #1 circuit 1231 and the handswitch #2 circuit 1271 may comprise generation and ADC circuits. In one aspect, handswitch #1 circuit 1231 and/or handswitch #2 circuit 1271 comprise generation circuit with the ability to generate handswitch waveforms.

The control circuit 1219 is coupled to the handswitch serial interface 1211 of the generator 1203 while the serial PIO integrated circuit 1233 is coupled to the handpiece serial interface 1213 as is the handpiece switching mechanism 1221. Further, the control circuit 1119 is coupled to the handswitch #1 circuit 1231 and the handswitch #2 circuit 1271. The control circuit 1119 may comprise a processor, FPGA, CPLD, PLD, microcontroller, and/or ASIC, for example. In the example shown in FIG. 12, the control circuit 1219 modulates two devices into at least one digital waveform, which enable the generator 1203 to perform the button monitoring and decision making. The control circuit 1219 also may allow for communication to two independent surgical instruments could receive either waveform. The serial PIO integrated circuit 1233 is further coupled to the handpiece switching mechanism 1221, the instrument power monitoring 1237, and the presence switching mechanism 1239. The instrument power monitoring 1237 and the serial PIO integrated circuit1233 may communicate results and failures to the generator 1203.

The switching mechanism 1223 is configured to receive the combined RF/ultrasonic power output 1205 from the generator 1203 and it may be provided to the energy storage circuit 1225 or the switching mechanism 1235. The control circuit 1219 is also coupled to the storage control 1227 and storage monitoring 1229 of the energy storage circuit 1217. The switching mechanism 1235 may provide the power output received from the switching mechanism 1223 to surgical instrument 9007, and/or surgical instrument 9008. The instrument power monitoring 1237 is coupled to the channels for the power output to the surgical instrument 9007 and surgical instrument 9008. The instrument power monitoring 1237 also may ensure that the switching mechanism 1235 is outputting power to correct location.

The handswitch #1 circuit 1231 and the handswitch #2 circuit 1271 are coupled to the HSW 1-wire serial protocol interfaces 9013, 9014 of the surgical instruments 9007, 9008, respectively. The handpiece switching mechanism 1221 is coupled to the handpiece serial interface 1213 of the generator 1203 and to the HP 1-wire serial protocol interfaces 9015, 9016 of the surgical instruments 9007, 9008, respectively. Further, the presence switching mechanism 1239 is coupled to the presence interface 1209 of the generator 1203 and to the presence Interfaces 9017, 9018 of the surgical instruments 9007, 9008, respectively. Further, Presence Switching mechanism is coupled to the unique presence 1241. In one aspect, different instrument presence elements may be switched on an on-demand basis using serial I/O or an adapter micro protocol.

A first communications protocol will be used to communicate to the control circuit 1219 on the adapter 1215. The generator 1203 also may have the ability to monitor surgical instruments 9007, 9008 at once. The adapter 1215 may comprise circuit to provide handswitch signal generation (e.g., in handswitch #1 circuit 1231 and handswitch #2 circuit 1271) along with ADCs to interpret this data. The adapter 1215 may modulate two surgical instrument signals into at least a first waveform and may have the ability to read in the fist and second waveforms. In various aspects, the second waveforms may be interpreted and translated into the format of the first waveforms. Further, the first protocol has the ability to send 12 bits at 615 bits/sec.

The control circuit 1219 may take the handswitch data from surgical instruments 9007, 9008 and modulate it into a first protocol. There are a few ways of doing this, but it may mean that surgical instruments 9007, 9008 may comprise a first protocol functionality. The system 1201 could communicate 4-6 buttons from the surgical instrument 9007 and 4-6 buttons from the surgical instrument 9008 in the first protocol frame. Alternatively, the system 1201 could use some form of addressing to access the surgical instruments 9007, 9008. The control circuit 1219 may have the ability to address separate devices by having the generator 1203 send the control circuit 1219 different addresses split into two different address spaces, one for surgical instrument 9007 and one for surgical instrument 9008.

The handpiece communications may involve some form of switch that could either be controlled via a serial I/O device or through the control circuit 1219 via a first protocol style communication interface from the generator 1203. In one aspect, energy storage monitoring 1229 and switching between surgical instruments 9007, 9008 and charging states could be handled in this manner as well. Certain first protocol addresses could be assigned to the data from the energy storage circuit 1225 and to the surgical instruments 9007, 9008 themselves. Presence elements could also be switched in with this format. Further, in one aspect, the control circuit 1219 may translate frames into a separate format, which may mean that the control circuit 1219 might need to make some decisions on whether button presses on surgical instruments 9007, 9008 are valid or not. The system 1201 would, however, allow the generator 1203 to fully monitor the surgical instruments 9007, 9008 at the same time time-slicing or handling a new communications protocol on the handswitch serial interface 1211 of the generator 1203. The system 1201 uses generator communications to simultaneously detect the activity of two surgical instruments, even during activation.

As noted above, a single output generator can deliver both RF and ultrasonic energy through a single port and these signals can be delivered separately or simultaneously to the end effector to treat tissue. One aspect of a combined RF and ultrasonic generator is shown in FIG. 1. As shown in FIG. 1, a single output port generator can include a single output transformer with multiple taps to provide power, either RF or ultrasonic energy, to the end effector depending on the type of treatment of tissue being performed. For example, the generator can deliver energy with higher voltage and lower current to drive an ultrasonic transducer, with lower voltage and higher current as required to drive electrodes for sealing tissue, or with a coagulation waveform for spot coagulation using either monopolar or bipolar electrosurgical electrodes. The output waveform from the generator can be steered, switched, or filtered to provide the desired frequency to the end effector of the surgical instrument.

The surgical instruments described herein can also include features to allow the energy being delivered by the generator to be dynamically changed based on the type of tissue being treated by an end effector of a surgical instrument. An algorithm for controlling the power output from a generator, such as generator 100, that is delivered to the end effector of the surgical instrument can include an input that represents the tissue type to allow the energy profile from the generator to be dynamically changed during the procedure based on the type of tissue being effected by the end effector of the surgical instrument.

Various algorithms can be used to select a power profile to allow the energy being delivered from the generator to dynamically change based on the tissue type being treated by the surgical instrument.

In order to determine the type of tissue being treated by the end effector of the surgical instrument, a tissue coefficient of friction can be calculated. The calculated tissue coefficient of friction is compared to a database of tissue coefficients of friction that correlates each tissue coefficient with a tissue type, as will be discussed in more detail below. The calculated tissue coefficient of friction and its related tissue type are used by an algorithm to control the energy being delivered from the generator to the surgical instrument. In one form, the tissue coefficient of friction is described by:

$$\mu = \frac{Q}{\vartheta \cdot N}$$

Where Q is the rate of heat generation, $\vartheta$ is the velocity of the ultrasonic motion of the end effector, and N is the force applied to the tissue by the end effector. The velocity of the ultrasonic motion is a known value from the settings of the generator. Since the value $\vartheta$ is a known value, the tissue coefficient of friction can be calculated using the slope of a graph of heat generation versus force on the tissue.

The force applied to the tissue by the end effector can be measured in a variety of ways using different type of components to measure force. This force measurement can be used, for example in the equation above, to determine the tissue coefficient of friction of the tissue being treated to determine its tissue type.

FIGS. 13-42 describe various examples of circuit topologies of a system with a combined generator configured to provide a combined signal with RF and ultrasonic energy frequencies to one or more surgical instruments.

FIG. 13 displays a circuit schematic for a system 1300 including a band-stop filtering circuit 1305 for a combined ultrasonic and RF surgical instrument 1303 that is configured to manage RF and ultrasonic currents output by a generator 1301. The band-stop filter circuit 1305 leverages a variable output frequency of the generator 1301 and employs tuned LC filter circuits 1307, 1309 to block unwanted output current. The circuit band-stop filter 1305 does not disconnect either the ultrasonic transducer or the RF output galvanically. Instead, both outputs are connected to their respective output terminals 1311, 1313 through an LC parallel resonant network of the tuned LC filter circuits 1307, 1309, respectively. A resonator 1315 is coupled to LC filter circuit 1307 and ultrasonic output terminal 1313 (labeled blade). As shown in FIG. 13, the LC filter circuit 1307 forms a parallel resonant network in series with the ultrasonic output terminal 1313 is tuned to the RF output frequency and exhibits an extremely high impedance at ~300kHz. The parallel resonant network of the LC filter circuit 1309 in series with the RF output terminal 1311 is tuned to the ultrasonic output frequency and exhibits an extremely high impedance at ~55kHz.

Undesirable frequency content is blocked to each output terminal1311, 113, thereby preventing unwanted excitation/dissipation in the transducer and unwanted low frequency current in the tissue. Depending on the attenuation required for each output terminal 1311, 1313, in aspects, the LC filter circuits 1307, 1309 can be further enhanced with additional resonant components. Since this concept does not rely on direct interaction with a control circuit in the surgical instrument, no changes or direct interface to the ASIC would be needed.

The band-stop filter circuit 1305 has the potential of allowing simultaneous RF and Ultrasonic therapy to the same surgical instrument. According to vairous aspects, a complex, multi-frequency output waveform may be applied and the resonant filters remain effective.

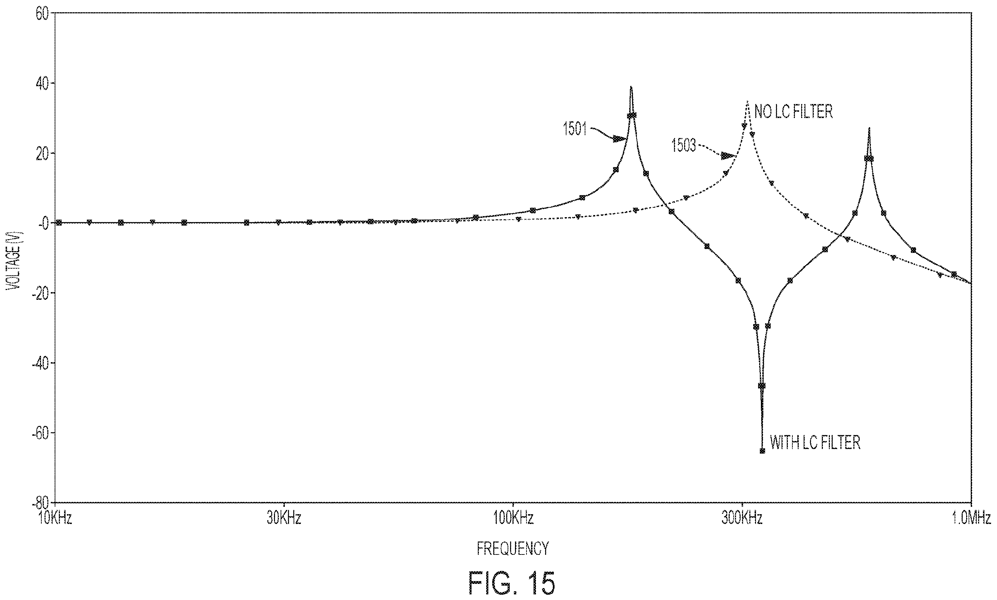

FIG. 14 displays a circuit schematic for a system 1400 for a combined ultrasonic and RF surgical instrument 1409 that is configured to manage RF and ultrasonic currents output by a generator 1401. System 1400 may be used for analyzing a high frequency band-stop filter. As shown in FIG. 14, system 1400 includes: an ultrasonic output of the generator 1401, with leakage inductance 1403 and sensing components 1405; 10' Cable model 1407 (which, according to the aspect of FIG. 14, has properties of 3.1uH, 194pF, 1 ohm); and a combined ultrasonic and RF surgical instrument 1409 that includes a parallel LC filter circuit 1411 for blocking RF content and an ultrasonic transducer 1413. FIGS. 15-21 are graphical depictions of simulation results for the system 1400 that illustrate the effect of the LC filter circuit 1411 that is intended to block high frequency RF content and prevent excessive RF current to the ultrasonic transducer 1413.

The system 1400 was simulated in both the stop-band, at 350kHz, and the pass band, at 55kHz, to shown that the parallel LC filter circuit 1411 is effective at blocking the unwanted current while still allowing normal operation in the pass-band. For the stop-band simulation, the generator 1401 is set to maximum RF amplitude of $100\ V_{rms}$ on the RF output which results in $365\ V_{pk}$ on the ultrasonic tap as shown below. For the pass-band simulation, the generator 1401 is set to maximum ultrasonic output voltage of $150\ V_{rms}$ or $212\ V_{pk}$ and the transducer is loaded to 400 ohms.

According to FIG. 15, the normalized transducer voltage versus frequency is shown in the plots 1501, 1503 with and without the LC filter circuit 1411, respectively. The voltage rise in the unfiltered plot 1503 is due to the un-damped series resonance of the transformer leakage inductance and C0. This resonance coincidently falls near the 350 kHz output frequency for the RF output of the generator 1401. The presence of the band-stop filter dominates the output impedance and results in significant attenuation at 350 kHz, however, it also results in some gain peaking at a lower frequency than is seen without the LC filter circuit 1411. Any gain peaks shown on these plots 1501, 1503 may amplify ultrasonic content at these frequencies, therefore, aspects of the present disclosure may maintain a low distortion output while in ultrasonic mode.

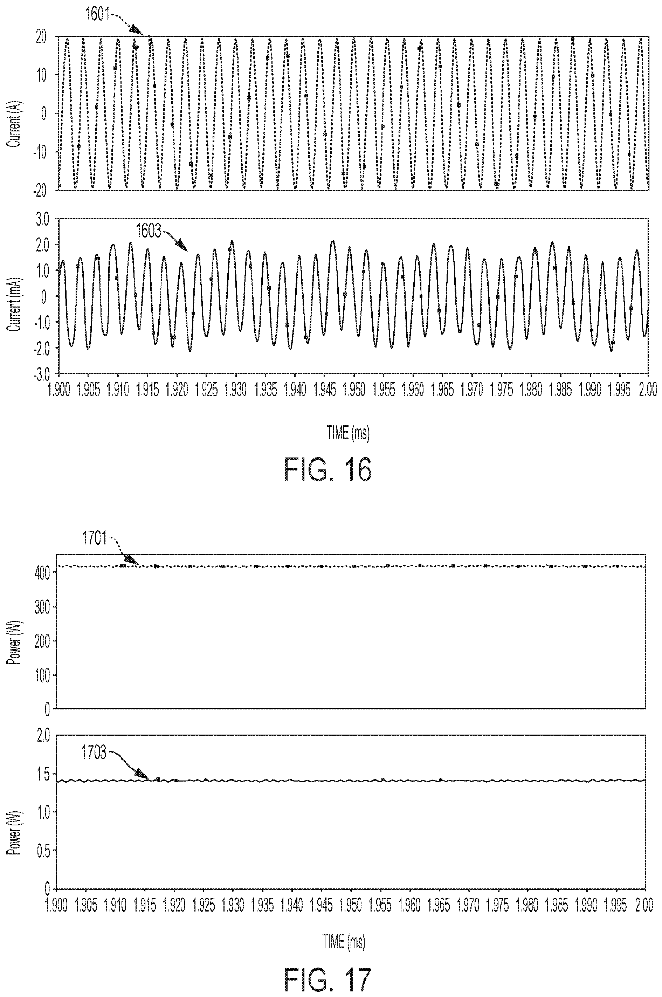

FIG. 16 shows the time domain waveforms of the system 1400 with the output frequency set to the stop-band, at 350kHz. The plots 1603, 1601 illustrate the transducer current versus time with and without the band-stop LC filter circuit 1411, respectively. Plot 1601 demonstrates that without a filter or switch to disconnect the ultrasonic transducer 1413, the large value of C0 acts as a short circuit and results in large output currents. The currents shown may not be realized in some generator configurations and might result instead in a shutdown or service-oriented architecture (SOA) fault. Plot 1603 demonstrates that the LC filter circuit 1411 can effectively block the RF content at the resonant frequency, resulting in only milliamps of current in the transducer 1413. The LC filter circuit 1411 may block/cancel the full output voltage amplitude of 365 $V_{pk}$.

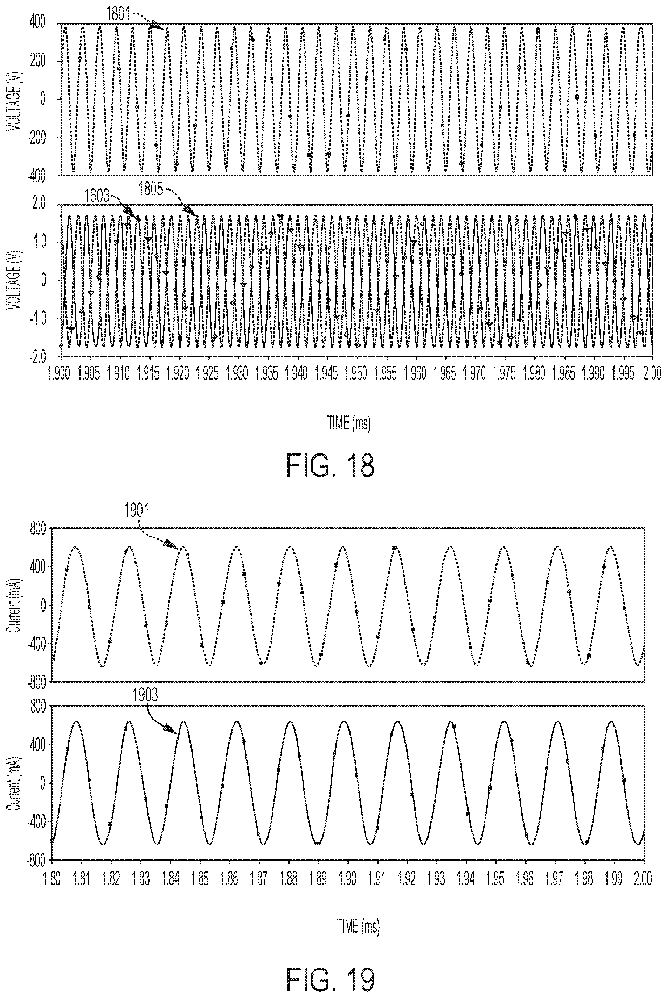

FIG. 17 shows the generator 1401 output power versus time at 350kHz, where the plots 1701, 1703 are without and with the band-stop LC filter circuit 1411, respectively. FIG. 17 illustrates that the total dissipation in the system 1400 may be kept low; however, this aspect does not include core losses in the inductor (labeled Lf) and only reflects conduction losses from the circulating current in the LC tank of the LC filter circuit 1411. The circulating currents 1803, 1805, of the inductor (labeled Lf) and capacitor (labeled Cf), respectively, of the LC filter circuit 1411 are shown according to current versus time in FIG. 18. The voltage 1801 across the LC filter circuit 1411 is shown according to voltage versus time. The core losses in the inductor (labeled Lf) may be very significant under test conditions, depending on the specific material, size and configuration of the resonant inductor shown in FIG. 14.

FIG. 19 shows the current of the transducer 1413 where the output frequency of the generator 1401 is set to a pass-band of 55kHz. Plots 1901, 1903 are in terms of current versus time, and show the current of the ultrasonic transducer 1413 without and with LC filter circuit 1411, respectively. The plots 1901, 1903 illustrate the time domain waveforms of the system 1400 and demonstrate that the normal operation of the ultrasonic transducer 1413 would be unaffected by the presence of the LC filter circuit 1411. According to the aspect of FIG. 14, the inductor (labeled Lf) in the LC filter circuit 1411 is dominant at the pass-band frequency (55kHz) and represents a low enough impedance to allow normal load current to flow to the ultrasonic transducer 1413 with minimal phase shift or loss.

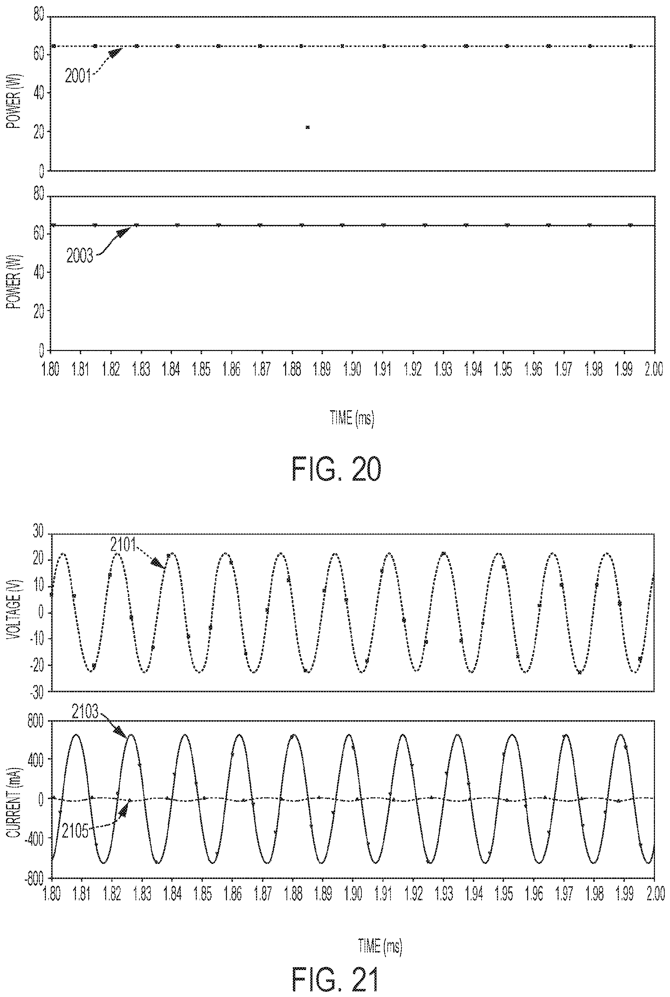

Additionally, FIG. 20 shows the generator 1401 output power in terms of real power versus time, where the output frequency of the generator 1401 is set to the pass-band of 55kHz. Plots 2001, 2003 are without and with the LC filter circuit 1411, respectively. Further, FIG. 21 shows plots of the voltage 2101 across the LC filter circuit 1411 at 55kHz and of the currents in the inductor (labeled 2103 and capacitor 2105 of the LC filter circuit 1411 at 55kHz, where the inductor, L, is equal to 100uH, and the capacitor, C, is equal to 2.07nF. According to aspects, generator configuration software may need to be updated to compensate for the presence of the LC filter circuit 1411 and account for its effects in the control loop, but the plots of FIGS. 19-21 indicate that ultrasonic functionality of the ultrasonic transducer 1413 should remain unaffected by the LC filter circuit 1411.

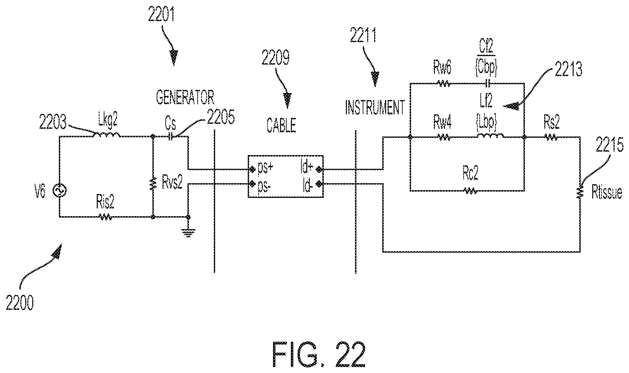

FIG. 22 illustrates a circuit schematic for a system 2200 that includes a high frequency band-stop filter according to one aspect of the present disclosure. The system 2200 includes: the RF output of the generator 2201, with leakage inductance 2203 and series output capacitor 2207; cable model 2209 (which, according to the aspect of FIG. 22, has properties of 3.1 uH, 194 pF, 1 ohm); a surgical instrument 2211 including parallel LC filter circuit 2213 for blocking ultrasonic content; and tissue impedance 2215.

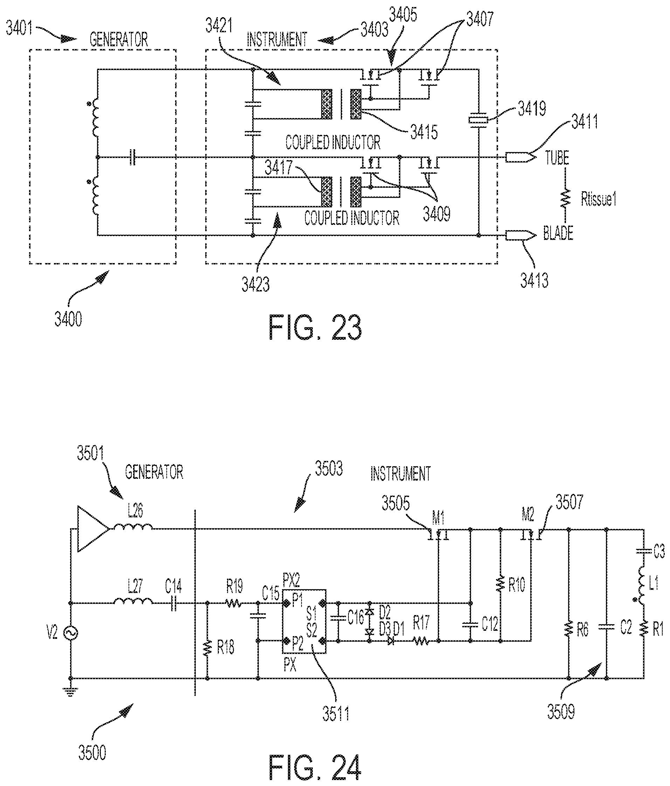

This system 2200 was simulated in both the stop-band, at 55kHz, and the pass band, at 350kHz. For the stop-band simulation, the generator 2301 is set to maximum amplitude of 150 $V_{rms}$ on the ultrasonic output which results in 82 $V_{pk}$ on the RF output. For the pass-band simulation, the generator 2301 is set to maximum RF output voltage of 100Vrms or 141Vpk and the tissue impedance is set to 50 ohms. FIGS. 23-33 provide simulation results that show that the system 2200 is effective at blocking unwanted current while still allowing normal operation in the pass-band.

According to various aspects, the inductor may be the primary source of loss in a LC band-stop filter circuit and may also drive the overall size of the circuit. There may be tradeoffs between these two factors, because smaller core geometries operate at high flux density and consequently, may have more loss. Although core loss is a consideration for both the high frequency and low frequency inductor components, core loss will be more critical for the high frequency component. According to one aspect, selection of a high efficiency core material that is optimized to operate at frequencies above 300 kHz is beneficial in order to keep losses at a particular level.

Configuration parameters for selection of an inductor include: the efficiency and performance of an optimized inductor configuration; the space available in the handle of a hand piece of the surgical instrument; the duty cycle of the application; the ability of the hand piece configuration to dissipate heat and remain acceptably cool.

As mentioned, losses may be driven predominantly by the inductor, assuming an appropriate capacitor is used that has a low dissipation factor. The mode of operation that will dissipate the most power may be when the LC circuit is operating at resonance (blocking) and not when it is passing the generator output to the respective load of the LC circuit. While at resonance, the full output voltage of the generator is seen by the LC circuit causing maximum core loss and the circulating current that is being exchanged between the capacitor and inductor may also cause copper losses.

The robustness and simplicity of the circuit components enhances safety parameters of the systems described above, for example, because the likelihood of component failure is diminished. According to various aspects, in the event of a damaged defective resonant component, systems of the present disclosure may rely on a generator to detect the presence of the defective component and correct the impedance vs frequency characteristic with a pre-run diagnostic. A pre-run diagnostic may offer direct confirmation that the LC circuit is tuned and undamaged.

According to various aspects, size and weight for the systems may be controlled by the inductor. The configuration considerations for the inductor configurations discussed above may push the inductor to be larger and heavier in order to keep the efficiency at an acceptable level. According to various aspects, given an identical set of output power requirements, the size and weight of LC band-stop filter circuit configuration may be greater than a solid state switch-based (e.g., MOSFET switch-based) configuration discussed below. According to one aspect, an LC circuit may potentially consist of a mere four passive components. According to another aspect, the LC circuit may not require the use of a printed circuit board. According to other aspects, the LC circuit may be implemented without any ASIC or hand switch electronics. Complexity of the LC circuit may increase depending on the needs of the specific application and on the possibility of implementing a hybrid concept that combines more than one of the circuits of the present disclosure. The prospect of very few components, no printed circuit board, or at least a minimalistic one, will serve to reduce both component and labor costs for the circuit configurations discussed above with regard to FIGS. 13-22. According to various aspects, some components may be custom engineered or high performance off-the-shelf items.

FIG. 23 is a circuit diagram of a system 3400 for a combined Ultrasonic and RF surgical instrument 3403 that is configured to manage RF and ultrasonic currents output by a generator 3401. The instrument circuit 3405 uses solid state switches such as MOSFET switches 3407, 3409 arranged in series to provide AC switching for each output 3411, 3413. Rather than the MOSFET switches 3407, 3409 being directly controlled by a control circuit, the instrument circuit 3405 employs tuned LC circuits 3421, 3423 to enhance the MOSFET switches 3407, 3409. Resonator 3419 is coupled to MOSFET switch 3407 is coupled to and ultrasonic output 3413 (labeled blade). This approach uses a pair of MOSFET switches 3407, 3409 that are arranged source-source, creating an AC switch. Rather than having the ASIC control the MOSFET enhancement, this approach leverages a coupled inductor 3415, 3417 that is capacitor tuned to enhance the gates of the MOSFET switches 3407, 3409 at the appropriate output frequency. When driving at the resonant frequency, the LC circuit 3421, 3423 generates a voltage on the primary of the coupled inductor 3415, 3417, which produces a gate enhancement potential. Since the MOSFET gate represents a minimal load, the inductor 3415, 3417 and gate drive circuitry can be relatively small and efficient.

FIG. 24 illustrates a circuit schematic for a system 3500 that includes: the Ultrasonic and RF output of the generator 3501; and an instrument 3503 that includes series connected solid state switches such as MOSFET switches 3505, 3507, a coupled inductor 3511, and an ultrasonic transducer model 3509 loaded to 400 ohms. The system 3500 was simulated at both 350 kHz and 55 kHz to verify correct functionality in both operating states: the blocking mode and the pass-through mode. For the blocking mode, the generator 3501 was set to maximum amplitude of $100V_{rms}$ on the RF output, which results in $365V_{pk}$ on the ultrasonic output. For the pass-through mode, the generator 3501 is set to maximum ultrasonic output voltage of $150V_{rms}$ or $212V_{pk}$ and the ultrasonic transducer model 3509 is loaded to 400 ohms.

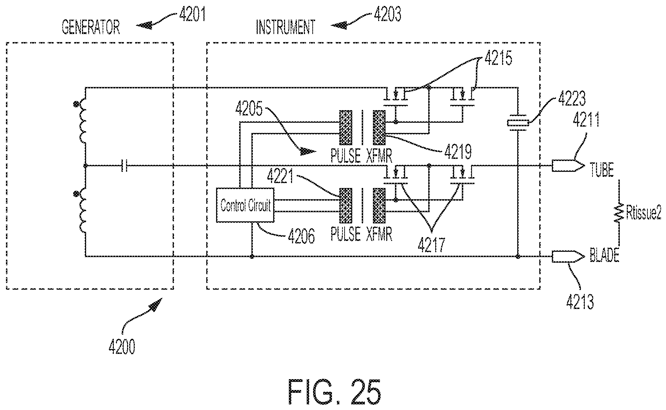

FIG. 25 is a circuit diagram of a system 4200 for a combined ultrasonic and RF surgical instrument 4203 that is configured to manage RF and ultrasonic currents output by a generator 4201. The instrument circuit 4205 uses a pair of solid stae switches such as MOSFET switches 4215, 4217 that are arranged source-source, creating an AC switch. The control circuit 4206 (e.g., ASIC) controls the MOSFET enhancement with control signals that are coupled via pulse transformers 4219, 4221. Since the MOSFET gate represents a minimal load and the switching rate is low, the power required to enhance the pair of MOSFET switches 4215, 4217 is very low. The control circuit 4206 outputs would be buffered by a small driver IC (not shown) that provides the pulse current needed to drive one or both of the pulse transformers 4219, 4221. One polarity of differential pulse applied to a pulse transformer 4219, 4221 will enhance the pair of MOSFET switches 4215, 4217, and the opposite polarity will turn the pair of MOSFET switches 4215, 4217 off. The pulse pattern will repeat at a maintenance interval to enforce the gate-source condition of the pair of MOSFET switches 4215, 4217. Discrete logic hardware can also be used to enforce mutually exclusive conditions in each AC switch and provide a partial mitigation to potential safety concerns.

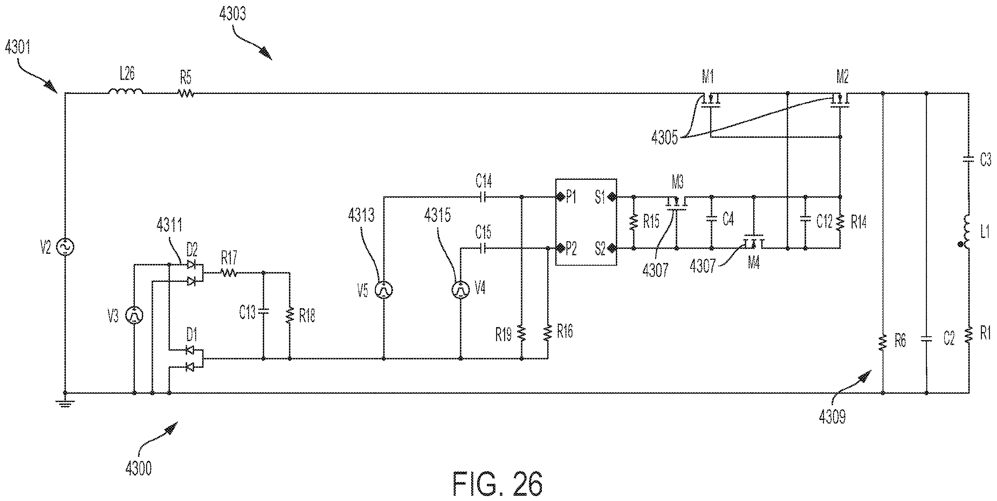

FIG. 26 illustrates a circuit schematic for a system 4300 that includes: the Ultrasonic and RF output of the generator 4301; and an instrument 4303 including series connected pair of solid state switches such as MOSFET switches 4305, 4307, a transducer model 4309 loaded to 400 ohms; power/GND reference 4311 via a rectified hand switch; and pulse generators 4313, 4315 representing logic buffers or MOSFET driver ICs. This system 4300 was simulated at 55 kHz in both the 'On' state and the 'Off' state to verify correct functionality in both operating states. For the both operating states the generator 4301 is set to maximum amplitude of 150Vrms on the ultrasonic output.

Losses for the system 4300 are driven primarily by the pair of MOSFET switches 4305, 4307. According to various aspects, these parts can be optimized for each output and since the switching time is in the millisecond scale and not nanoseconds, high efficiency parts may be available that contribute low total loss. The remaining components that enhance the MOSFET are not power components and should not be a significant contributor to losses. Overall power efficiency of the system 4300 is considered to be good. According to various aspects, a single fault tolerant architecture that employs redundant components may provide a solution to a component failure hazard. The size and weight of the ASIC controlled MOSFET switch circuit configuration of instrument 4303 is excellent. The MOSFETs used to interrupt the generator output may be smaller than equivalent RF filter implementations, but may also require some board level thermal management that consumes board space. According to various aspects, the pulse transformer and buffer that enhance the MOSFET switch may also be very small. Additionally, the components for the ASIC controlled MOSFET switch circuit configuration of instrument 4303 may off-the shelf and require no custom engineered components.

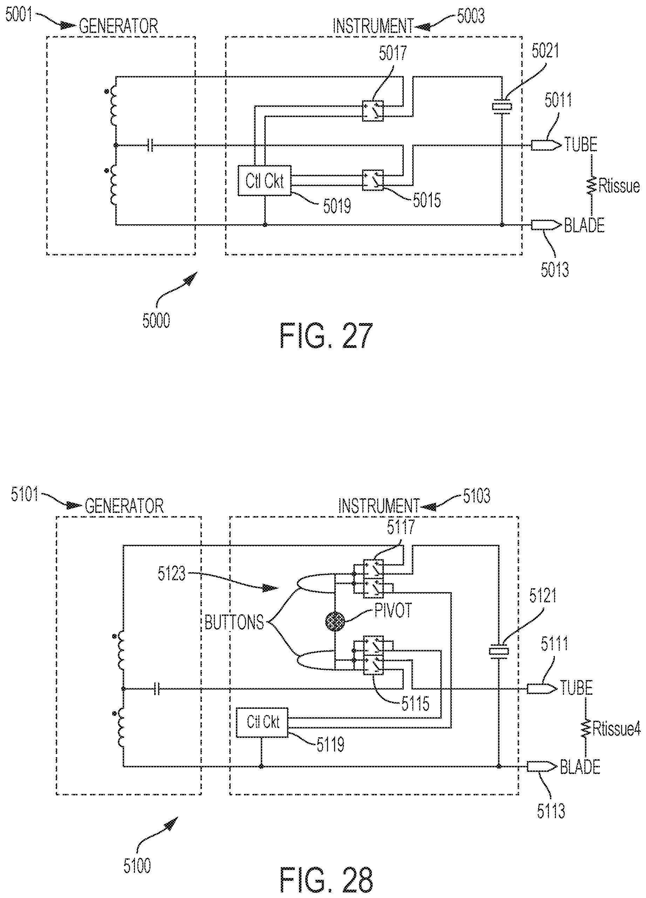

FIG. 27 is a circuit diagram of a system 5000 for a combined ultrasonic and RF surgical instrument 5003 that is configured to manage RF and ultrasonic currents output by a generator 5001. The system 5000 uses electromechanical relays 5015, 5017 for switching for each output 5011, 5013. The relays 5015, 5017 are driven by control signals from the control circuit 5019 (e.g., ASIC). Resonator 5021 is coupled to a relay 5017 and the ultrasonic output 5013 (labeled blade).

The system 5000 is similar to the ASIC controlled MOSFET switch configurations discussed with regard to FIGS. 25-26, but the MOSFET switching element(s) have been replaced with an electromechanical relay. Unlike the MOSFET which requires minimal power to enhance, a non-latching relay may require too much power to actuate continuously; therefore a latching type relay is a preferred option for the electromechanical relays 5015, 5017 of system 5000. According to various aspects, the isolation that was provided by a pulse transformer in the MOSFET configurations, discussed with regard to FIGS.25-26, may be integrated in the relay which gives an intrinsic isolation between coil and contacts. Furthermore, in other aspects where a relay is chosen that employs force guided contacts, an auxiliary set of sensing contacts can be used to provide feedback signals to the control circuit 5019 that confirm the state of the contacts for safety mitigation.

FIG. 28 is a circuit diagram of a system 5100 for a combined ultrasonic and RF surgical instrument 5103 that is configured to manage RF and ultrasonic currents output by a generator 5101. The system 5100 includes electromechanical relays 5115, 5117 and system 5100 uses a switch actuation initiated by an operator of the combined ultrasonic and RF surgical instrument 5103 to connect the appropriate output 5111 or 5113 within the combined ultrasonic and RF surgical instrument 5103 via the electromechanical relays 5115, 5117. As shown in the aspect of FIG. 51, the switch actuation may be accomplished via buttons and a pivot or toggle that accomplishes the appropriate opening or closure of the electromechanical relays 5115 or 5117. Resonator 5121 is coupled to electromechanical relay 5117 and ultrasonic output 5113 (labeled blade).

Other configurations discussed herein may use a signal switch closure to command a secondary power switch actuation via a control circuit. The system 5100 eliminates circuitry that may be necessary for such a secondary electronic actuation and uses an input to a switch 5123 to both command the control circuit 5119 (e.g., ASIC) for activation and engage the appropriate output 5111 or 5113 to the combined ultrasonic and RF surgical instrument 5103. In one aspect, the switch that performs this may be a snap-action variety which requires very little throw to engage the contacts. In another aspect, a mechanical rocker style switch mechanism may be employed in the combined ultrasonic and RF surgical instrument 5103 which ensures that the two states are mutually exclusive.

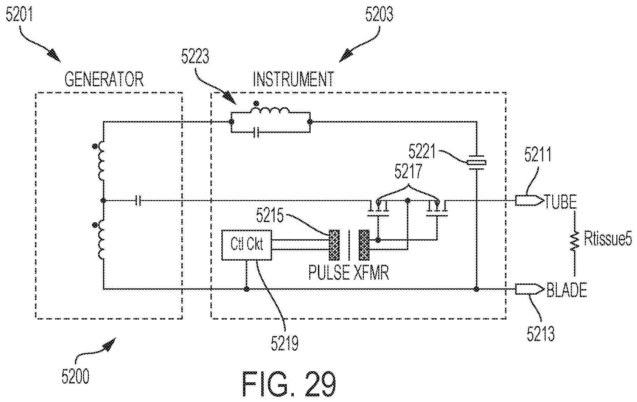

FIG. 29 is a circuit diagram of a system 5200 for a combined ultrasonic and RF surgical instrument 5203 that is configured to manage RF and ultrasonic currents output by a generator 5201. The combined ultrasonic and RF surgical instrument 5203 employs a configuration that shares components from other circuit configurations discussed herein to form an optimized device. The combined ultrasonic and RF surgical instrument 5203 includes a parallel LC filter circuit 5223 coupled to a crystal 5221, which feeds the output of the generator 5201 to the ultrasonic output 5213 of the combined ultrasonic and RF surgical instrument 5203. Additionally, a control circuit 5219 is coupled to a pulse transformer 5215. The pulse transformer 5215 is coupled to a pair of solid state switches such as MOSFET switches 5217 that are arranged source-source, creating an AC switch, which feeds the output of the generator 5201 to the RF output 5211 of the combined ultrasonic and RF surgical instrument 5203.

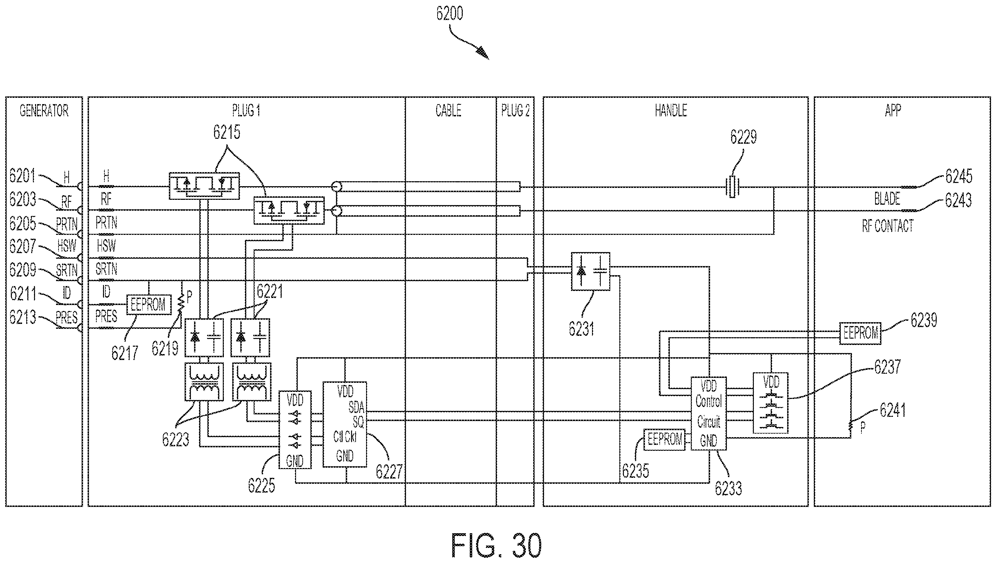

FIG. 30 illustrates system 6200 that includes electro-mechanical or solid state switches comprising transistors such as MOSFET switches and a control circuit in the proximal plug and a control circuit in the handle. The generator comprises interfaces for an ultrasonic signal 6201, an interface for an RF signal 6203, a primary return terminal interface 6205, an HSW interface 6207, a secondary return terminal interface 6209, an identification interface 6211, and a presence interface 6213. The proximal plug comprises matching interfaces to those of generator, a pair of MOSFET switches 6215, an EEPROM 6217, and presence resistor 6219. The MOSFET switches 6215 are each coupled to rectifier circuits 6221 that are each coupled to a pair of coupling inductors 6223. Each rectifier circuit 6221 may comprise at least one diode and at least one capacitor. The control circuit 6227 (e.g., ASIC) is coupled to a driver circuit 6225 that feeds the coupling inductors 6223 and the rectifier circuits 6221 to control the state of the MOSFET switches 6215. The proximal plug outputs are carried through the cable and the distal plug to the handle without any component circuitry in either the cable or the distal plug. The handle comprises resonator 6229, rectifier circuit 6231, control circuit 6233 (e.g., ASIC), EEPROM 6235, and switch array 6237. The switch array 6237 may comprise electro-mechanical devices, transistor devices, and the like. The transistor devices may include bipolar junction transistors (BJT), FETs, MOSFETs, or a combination thereof. Rectifier circuit 6231 may comprise at least one diode and at least one capacitor. Control circuit 6233 is coupled to EEPROM 6235 and receives outputs from control circuit 6227 in the proximal plug.

The application portion comprises EEPROM 6239, presence resistor 6241, and outputs for RF and ultrasonic energy 6243, 6245, respectively. EEPROM 6239 and presence resistor 6241 are coupled to control circuit 6233. The system 6200 allows switching between an RF mode and an ultrasonic, also called ultrasonic, mode and allows for a transfer of weight, volume, and heat away from the handle and application portion. The two control circuits 6227, 6233 (e.g., ASIC devices) may also add flexibility to features that are available in the handle and the proximal plug.

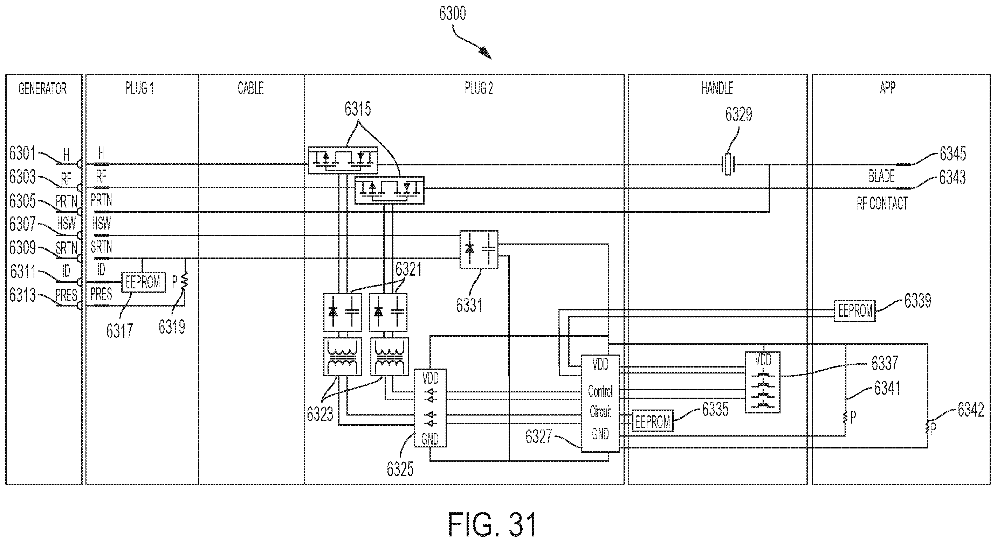

FIG. 31 illustrates a system 6300 that includes electro-mechanical or solid state switches such as MOSFET switches and a control circuit in the distal plug. The generator comprises interfaces for an ultrasonic signal 6301, an interface for an RF signal 6303, a primary return terminal interface 6305, an HSW interface 6307, a secondary return terminal interface 6309, an identification interface 6311, and a presence interface 6313. The proximal plug comprises matching interfaces to those of generator, an EEPROM 6317, and presence resistor 6319. The proximal plug outputs are carried through the cable without any component circuitry in the cable. The distal plug comprises MOSFET switches 6315 that are each coupled to rectifier circuits 6321 that are each coupled to a pair of coupling inductors 6323. Each of rectifier circuits 6321 comprises at least one diode and at least one capacitor. The control circuit 6327 (e.g., ASIC) is coupled to a driver circuit 6325 that feeds into the coupling inductors 6323 and the rectifier circuits 6321 to control the state of the MOSFET switches 6315. The distal plug also includes rectifier circuit 6331 coupled to the HSW interface 6307 and the secondary return terminal interface 6309 of the generator and feed into control circuit 6327. Rectifier circuit 6331 comprises at least one diode and at least one capacitor. The handle comprises resonator 6329, EEPROM 6335, switch array 6337, and presence resistor 6341. The switch array 6337 may comprise electro-mechanical devices, transistor devices, and the like. The transistor devices may include bipolar junction transistors (BJT), FETs, MOSFETs, or a combination thereof. Control circuit 6327 is coupled to EEPROM 6335, switch array 6337, and presence resistor 6341 in the handle.

The application portion comprises EEPROM 6339, presence resistor 6342, and outputs for RF and ultrasonic energy 6343, 6345, respectively. EEPROM 6339 and presence resistor 6342 are coupled to control circuit 6327. The system 6300 allows switching between an RF mode and an ultrasonic mode and allows for minimal cost and complexity in the handle of the surgical instrument.

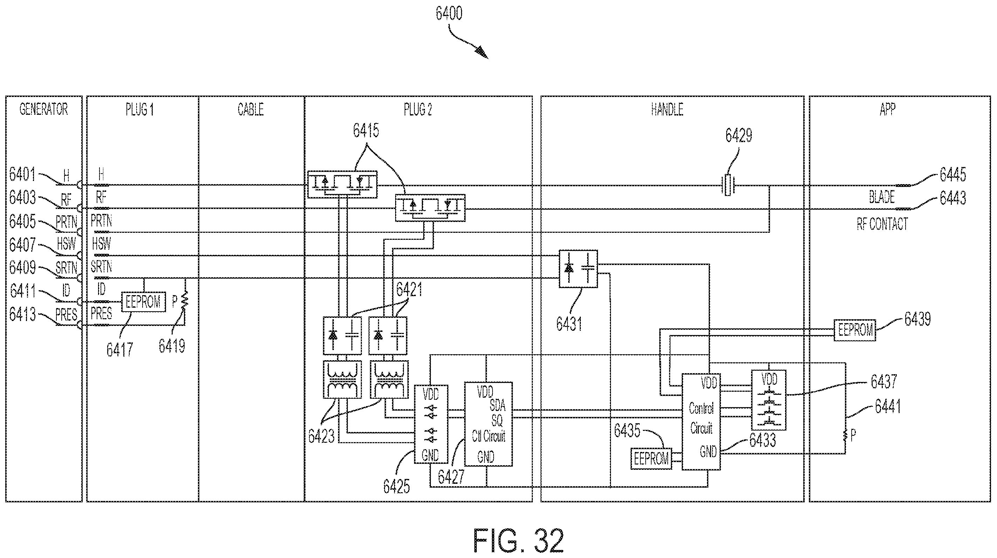

FIG. 32 illustrates a system 6400 that includes electro-mechanical or solid state switches such as MOSFET switches and a control circuit in the distal plug and a control circuit in the handle. The generator comprises interfaces for an ultrasonic signal 6401, an interface for an RF signal 6403, a primary return terminal interface 6405, an HSW interface 6407, a secondary return terminal interface 6409, an identification interface 6411, and a presence interface 6413. The proximal plug comprises matching interfaces to those of generator, an EEPROM 6417, and presence resistor 6419. The proximal plug outputs are carried through the cable without any component circuitry in the cable. Distal plug comprises a pair of MOSFET switches 6415, The MOSFET switches 6415 are each coupled to rectifier circuits 6421 that are each coupled to a pair of coupling inductors 6423, all located within the distal plug. The rectifier circuits 6421 each comprise at least one diode and at least one capacitor. The control circuit 6427 (e.g., ASIC) is coupled to a driver circuit 6425, which are also located in the distal plug, that feeds the coupling inductors 6423 and the rectifier circuits 6421 to control the state of the MOSFET switches 6415. The handle comprises resonator 6429, rectifier circuit 6431, control circuit 6433 (e.g., ASIC), EEPROM 6435, and switch array 6437. The switch array 6437 may comprise electro-mechanical devices, transistor devices, and the like. The transistor devices may include bipolar junction transistors (BJT), FETs, MOSFETs, or a combination thereof. Rectifier circuit 6431 comprises at least one diode and at least one capacitor. Control circuit 6433 is coupled to EEPROM 6435 and receives outputs from control circuit 6427 in the distal plug.

The application portion comprises EEPROM 6439, presence resistor 6441, and outputs for RF and ultrasonic energy 6443, 6445, respectively. EEPROM 6439 and presence resistor 6441 are coupled to control circuit 6433. The system 6400 allows switching between an RF mode and an ultrasonic mode and the two control circuits 6427, 6433 (e.g., ASIC devices) may also add flexibility to features that are available in the handle and the distal plug.

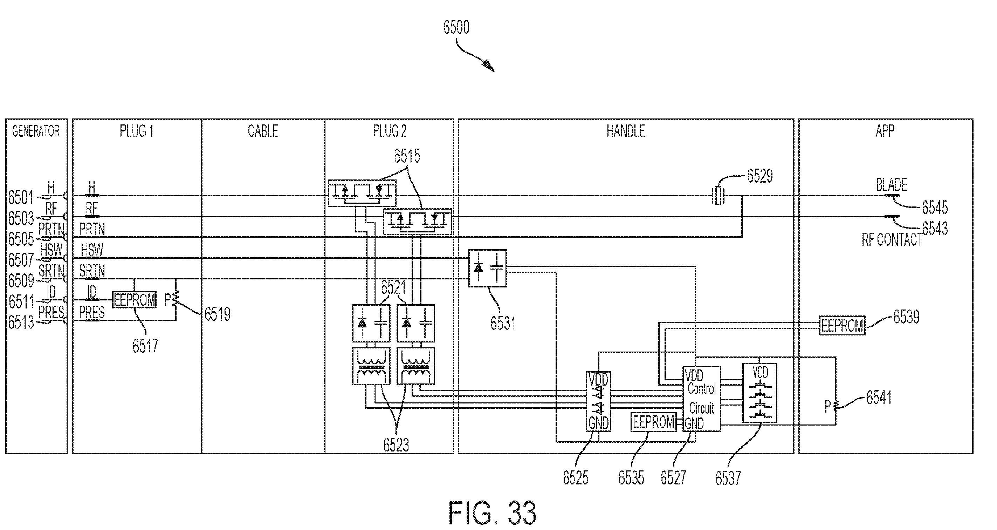

FIG. 33 illustrates a system 6500 that includes electro-mechanical or solid state switches such as MOSFET switches in the distal plug and a control circuit in the handle. The generator comprises interfaces for an ultrasonic signal 6501, an interface for an RF signal 6503, a primary return terminal interface 6505, an HSW interface 6507, a secondary return terminal interface 6509, an identification interface 6511, and a presence interface 6513. The proximal plug comprises matching interfaces to those of generator, an EEPROM 6517, and presence resistor 6519. The proximal plug outputs are carried through the cable without any component circuitry in the cable. The distal plug comprises MOSFET switches 6515 that are each coupled to rectifier circuits 6521, which are each coupled to a pair of coupling inductors 6523. Each of rectifier circuits 6521 comprise at least one diode and at least one capacitor. The handle comprises rectifier circuit 6531, driver circuit 6525, control circuit 6327, EEPROM 6535, switch array 6537, and resonator 6529. The switch array 6537 may comprise electro-mechanical devices, transistor devices, and the like. The transistor devices may include bipolar junction transistors (BJT), FETs, MOSFETs, or a combination thereof. The control circuit 6527 (e.g., ASIC) is coupled to a driver circuit 6525 that feeds into the coupling inductors 6523 and the rectifier circuits 6521 to control the state of the MOSFET switches 6515. Rectifier circuit 6531 is coupled to the HSW interface 6507 and the secondary return terminal interface 6509 of the generator and feed into control circuit 6527. As shown, rectifier circuit 6531 may comprise at least one diode and at least one capacitor. Control circuit 6527 is coupled to EEPROM 6535, switch array 6537.

The application portion comprises EEPROM 6539, presence resistor 6541, and outputs for RF and ultrasonic energy 6543, 6545, respectively. EEPROM 6539 and presence resistor 6541 are coupled to control circuit 6527. The system 6500 allows switching between an RF mode.

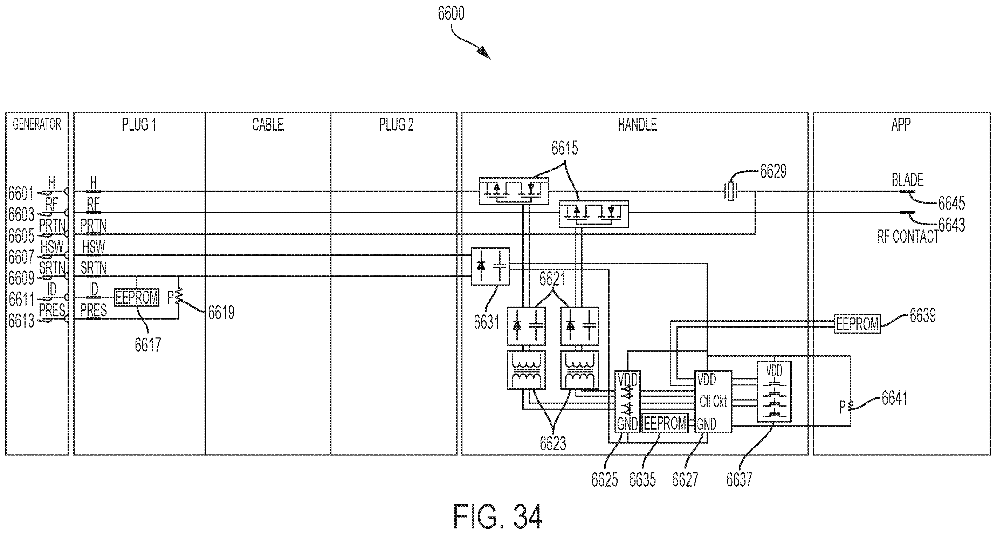

FIG. 34 illustrates a system 6600 that includes electro-mechanical or solid state switches such as MOSFET switches and a control circuit in the handle. The generator comprises interfaces for an ultrasonic signal 6601, an interface for an RF signal 6603, a primary return terminal interface 6605, an HSW interface 6607, a secondary return terminal interface 6609, an identification interface 6611, and a presence interface 6613. The proximal plug comprises matching interfaces to those of generator, an EEPROM 6617, and presence resistor 6619. The proximal plug outputs are carried through the cable and the distal plug to the handle without any component circuitry in either the cable or the distal plug. The handle comprises the MOSFET switches 6615 that are each coupled to rectifier circuits 6621, which are each coupled to a pair of coupling inductors 6623, also in the handle. The rectifier circuits 6621 each comprise at least one diode and at least one capacitor. The control circuit 6627 (e.g., ASIC) is coupled to a driver circuit that feeds into the coupling inductors 6623 and the rectifier circuits 6621 to control the state of the MOSFET switches 6615. The driver circuit 6625 and control circuit 6627 are located in the handle. The handle further comprises resonator 6629, rectifier circuits 6631 comprising a diode and a capacitor, EEPROM 6635, and switch array 6637. The switch array 6637 may comprise electro-mechanical devices, transistor devices, and the like. The transistor devices may include bipolar junction transistors (BJT), FETs, MOSFETs, or a combination thereof. The rectifier portion of the diode and capacitor circuit 6631 is coupled to the HSW interface 6607 and the secondary return terminal interface 6609 of the generator and feed into control circuit 6627.

The application portion comprises EEPROM 6639, presence resistor 6641, and outputs for RF and ultrasonic energy 6643, 6645, respectively. EEPROM 6639 and presence resistor 6241 are coupled to control circuit 6627. The system 6600 allows switching between an RF mode and an ultrasonic mode and allows for a low cost cable configuration.

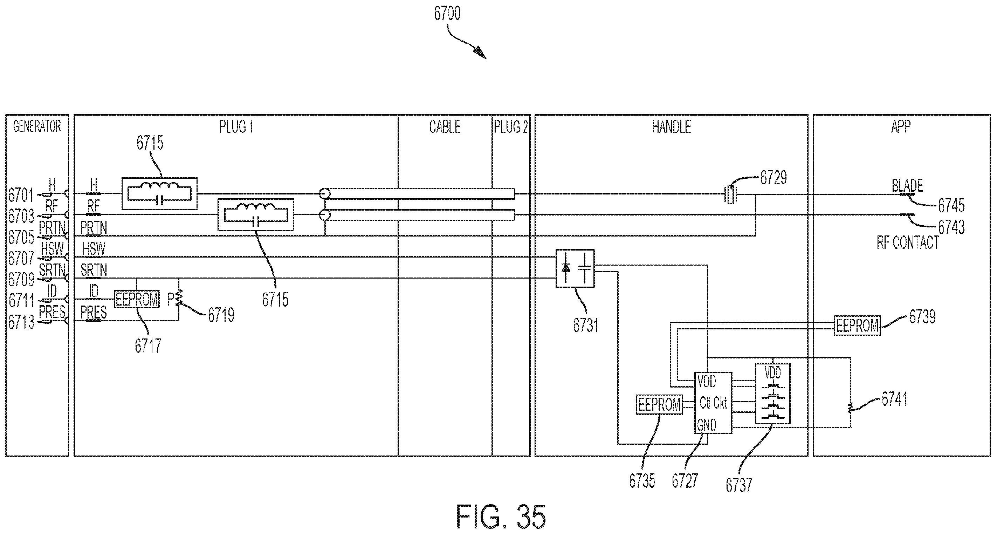

FIG. 35 illustrates a system 6700 that includes bandstop filters in the proximal plug and a control circuit in the handle. The generator comprises interfaces for an ultrasonic signal 6701, an interface for an RF signal 6703, a primary return terminal interface 6705, an HSW interface 6707, a secondary return terminal interface 6709, an identification interface 6711, and a presence interface 6713. The proximal plug comprises matching interfaces to those of generator, a pair of bandstop filters 6715, an EEPROM 6717, and presence resistor 6719. The proximal plug outputs are carried through the cable and the distal plug to the handle without any component circuitry in either the cable or the distal plug. The handle comprises resonator 6729, rectifier circuit 6731, control circuit 6727 (e.g., ASIC), EEPROM 6735, and switch array 6737. Rectifier circuit 6731 comprises at least one diode and at least one capacitor. The switch array 6737 may comprise electro-mechanical devices, transistor devices, and the like. The transistor devices may include bipolar junction transistors (BJT), FETs, MOSFETs, or a combination thereof. Control circuit 6727 is coupled to EEPROM 6735 and rectifier circuit 6731 are coupled to the HSW interface 6707 and the secondary return terminal interface 6709 of the generator and feed into control circuit 6727.

The application portion comprises EEPROM 6739, presence resistor 6741, and outputs for RF and ultrasonic energy 6743, 6745, respectively. The pair of bandstop filters 6715 are coupled to the outputs for RF and ultrasonic energy 6743, 6745. EEPROM 6739 and presence resistor 6741 are coupled to control circuit 6727. The system 6700 allows switching between an RF mode and an ultrasonic mode and supports mixed output frequencies, which allows tissues impedance sensing while the ultrasonic output is active. It also allows for a transfer of weight, volume, and heat away from the handle and application portion.

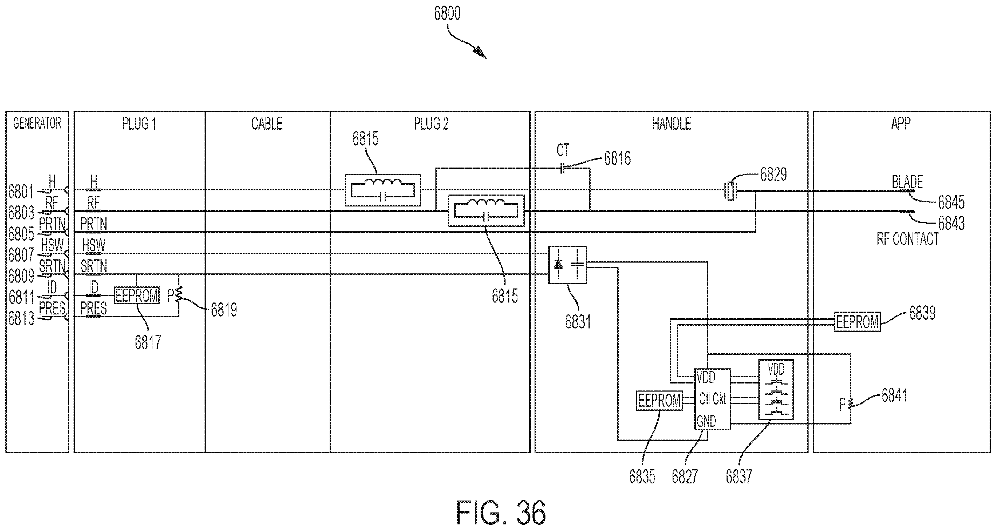

FIG. 36 illustrates a system 6800 that includes bandstop filters in the distal plug and a control circuit in the handle. The generator comprises interfaces for an ultrasonic signal 6801, an interface for an RF signal 6803, a primary return terminal interface 6805, an HSW interface 6807, a secondary return terminal interface 6809, an identification interface 6811, and a presence interface 6813. The proximal plug comprises matching interfaces to those of generator, an EEPROM 6817, and presence resistor 6819. The proximal plug outputs are carried through the cable without any component circuitry in the cable. The distal plug comprises a pair of bandstop filters 6715. The handle comprises rectifier circuit 6831, EEPROM 6835, control circuit 6827, switch array 6837, capacitor 6816, and resonator 6829. Rectifier circuit 6831 comprises at least one diode and at least one capacitor. The control circuit 6827 is coupled to EEPROM 6835, switch array 6837, and rectifier circuit 6831. The switch array 6837 may comprise electro-mechanical devices, transistor devices, and the like. The transistor devices may include bipolar junction transistors (BJT), FETs, MOSFETs, or a combination thereof.

The application portion comprises EEPROM 6839, presence resistor 6841, and outputs for RF and ultrasonic energy 6843, 6845, respectively. The pair of bandstop filters 6815 are coupled to the outputs for RF and ultrasonic energy 6843, 6845. EEPROM 6839 and presence resistor 6841 are coupled to control circuit 6827. The system 6800 allows switching between an RF mode and an ultrasonic mode and supports mixed output frequencies, which allows tissues impedance sensing while the ultrasonic output is active.

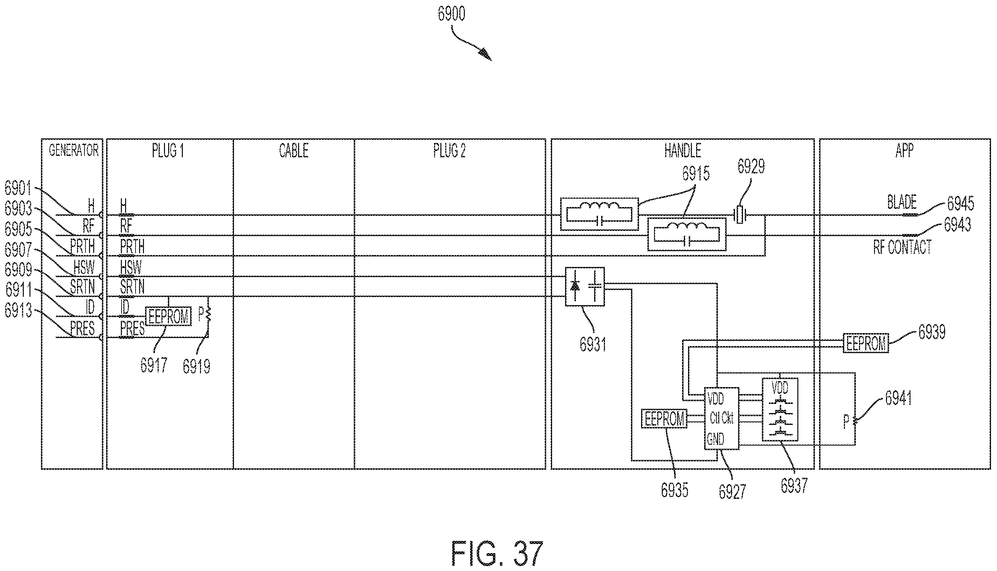

FIG. 37 illustrates a system 6900 that includes bandstop filters and a control circuit in the handle. The generator comprises interfaces for an ultrasonic signal 6901, an interface for an RF signal 6903, a primary return terminal interface 6905, an HSW interface 6907, a secondary return terminal interface 6909, an identification interface 6911, and a presence interface 6913. The proximal plug comprises matching interfaces to those of generator, an EEPROM 6917, and presence resistor 6919. The proximal plug outputs are carried through the cable and distal plug without any component circuitry in either the cable or the distal plug. The handle comprises a pair of bandstop filters 6915, rectifier circuit 6931, EEPROM 6935, control circuit 6927, switch array 6937, and resonator 6929. Rectifier circuit 6931 comprises at least one diode and at least one capacitor. Control circuit 6927 is coupled to EEPROM 6935, switch array 6937, and rectifier circuit 6931. The switch array 6937 may comprise electro-mechanical devices, transistor devices, and the like. The transistor devices may include bipolar junction transistors (BJT), FETs, MOSFETs, or a combination thereof.

The application portion comprises EEPROM 6939, presence resistor 6941, and outputs for RF and ultrasonic energy 6943, 6945, respectively. The pair of bandstop filters 6915 are coupled to the outputs for RF and ultrasonic energy 6943, 6945. EEPROM 6939 and presence resistor 6941 are coupled to control circuit 6927. The system 6900 allows switching between an RF mode and an ultrasonic mode and supports mixed output frequencies, which allows tissues impedance sensing while the ultrasonic output is active. It also provides for a low cost cable configuration.

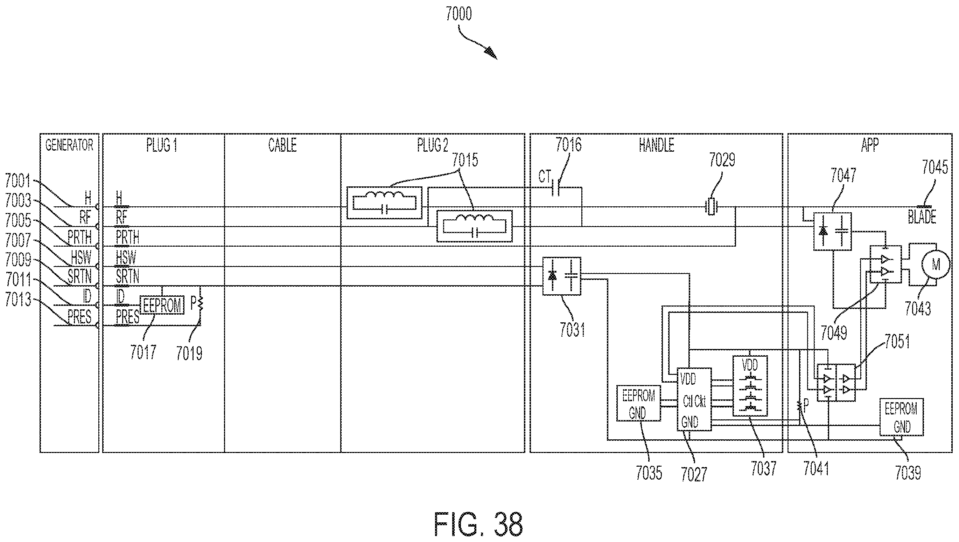

FIG. 38 illustrates a system 7000 that includes bandstop filters in the distal plug, a control circuit in the handle, and a DC motor in the application portion. The generator comprises interfaces for an ultrasonic signal 7001, an interface for an RF signal 7003, a primary return terminal interface 7005, an HSW interface 7007, a secondary return terminal interface 7009, an identification interface 7011, and a presence interface 7013. The proximal plug comprises matching interfaces to those of generator, an EEPROM 7017, and presence resistor 7019. The proximal plug outputs are carried through the cable without any component circuitry in the cable. The distal plug comprises a pair of bandstop filters 7015. The handle comprises rectifier circuit 7031, EEPROM 7035, control circuit 7027, switch array 7037, capacitor 7016, and resonator 7029. Rectifier circuit 7031 comprises at least one diode and at least one capacitor. Control circuit 7027 is coupled to EEPROM 7035, switch array 7037, and rectifier circuit 7031. The switch array 7037 may comprise electro-mechanical devices, transistor devices, and the like. The transistor devices may include bipolar junction transistors (BJT), FETs, MOSFETs, or a combination thereof.

The application portion comprises EEPROM 7039, presence resistor 7041, and an output for ultrasonic energy 7045. The application portion further comprises rectifier circuit 7047, driver circuit 7049, driver circuit 7051, and DC motor 7043. Rectifier circuit 7047 comprises at least one diode and at least one capacitor. The rectifier circuit 7047 is coupled to the driver circuit 7049, which is coupled to the DC motor 7043. Driver circuit 7051 is coupled to control circuit 7027 and driver circuit 7049. EEPROM 7039 and presence resistor 7041 are also coupled to control circuit 7027. The system 7000 allows switching between an RF mode and an ultrasonic mode and supports mixed output frequencies, which allows tissues impedance sensing while the ultrasonic output is active. It also provides for a DC motor at the ultrasonic output that uses energy directed to the RF output terminal for generating a DC voltage.

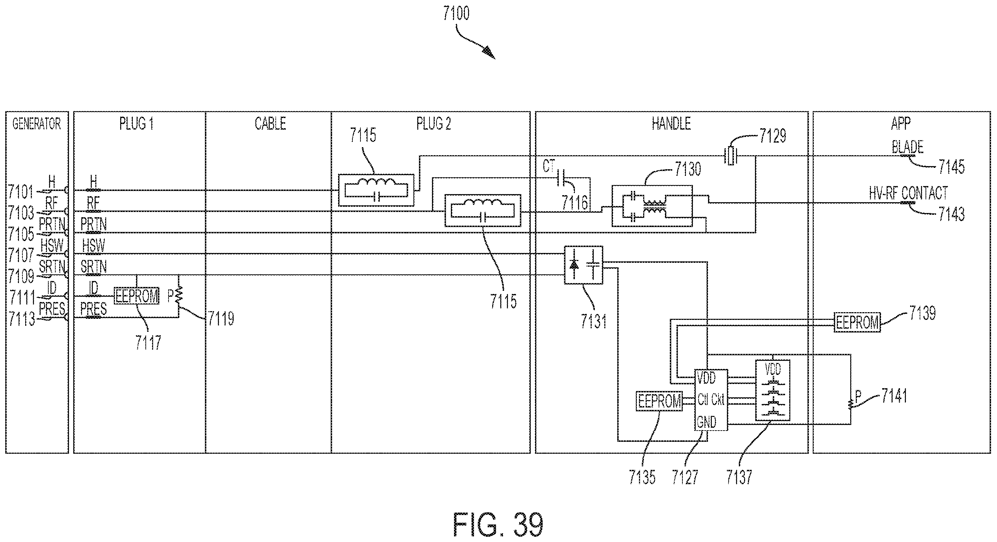

FIG. 39 illustrates a system 7100 that includes a fixed high voltage RF output in the application portion, bandstop filters in the distal plug, and a control circuit and transformer in handle. The generator comprises interfaces for an ultrasonic signal 7101, an interface for an RF signal 7103, a primary return terminal interface 7105, an HSW interface 7107, a secondary return terminal interface 7109, an identification interface 7111, and a presence interface 7113. The proximal plug comprises matching interfaces to those of generator, an EEPROM 7117, and presence resistor 7119. The proximal plug outputs are carried through the cable without any component circuitry in the cable. The distal plug comprises a pair of bandstop filters 7115. The handle comprises rectifier circuits 7131 comprising a diode and a capacitor, EEPROM 7135, control circuit 7127, transformer 7130, capacitor 7116, switch array 7137, and resonator 7129. The control circuit 7127 is coupled to EEPROM 7135, switch array 7137, and rectifier circuits 7131 comprising a diode and a capacitor. The switch array 7137 may comprise electro-mechanical devices, transistor devices, and the like. The transistor devices may include bipolar junction transistors (BJT), FETs, MOSFETs, or a combination thereof.

The application portion comprises EEPROM 7139, presence resistor 7141, and high voltage RF and ultrasonic energy outputs 7143, 7145, respectively. Transformer 7130 is coupled to one of the bandstop filters 7115 and the secondary side of transformer 7130 is coupled to the high voltage RF and ultrasonic energy outputs 7143, 7145. EEPROM 7139 and presence resistor 7141 are coupled to control circuit 7127. The system 7100 allows switching between an RF mode and an ultrasonic mode and supports mixed output frequencies, which allows tissues impedance sensing while the ultrasonic output is active. It also supports high RF output voltage for surface coagulation.

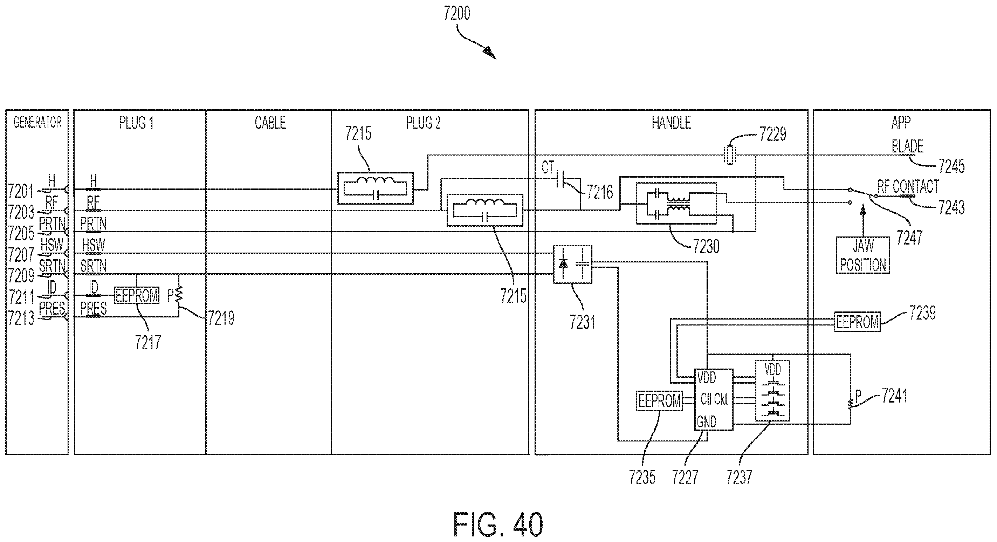

FIG. 40 illustrates a system 7200 that includes a mechanically switched high voltage/low voltage RF output in the application portion, bandstop filters in distal plug, and a control circuit and transformer in the handle. The generator comprises interfaces for an ultrasonic signal 7201, an interface for an RF signal 7203, a primary return terminal interface 7205, an HSW interface 7207, a secondary return terminal interface 7209, an identification interface 7211, and a presence interface 7213. The proximal plug comprises matching interfaces to those of generator, an EEPROM 7217, and presence resistor 7219. The proximal plug outputs are carried through the cable without any component circuitry in the cable. The distal plug comprises a pair of bandstop filters 7215. The handle comprises rectifier circuit 7231, EEPROM 7235, control circuit 7227, transformer 7230, capacitor 7216, switch array 7237, and resonator 7229. The control circuit 7227 is coupled to EEPROM 7235, switch array 7237, and rectifier circuit 7231. Rectifier circuit 7231 comprises at least one diode and at least one capacitor. The switch array 7237 may comprise electro-mechanical devices, transistor devices, and the like. The transistor devices may include bipolar junction transistors (BJT), FETs, MOSFETs, or a combination thereof.

The application portion comprises EEPROM 7239, presence resistor 7241, end effector jaw position switch 7247, and RF and ultrasonic energy outputs 7243, 7245, respectively. Transformer 7230 is coupled to one of the bandstop filters 7215. The secondary side of transformer 7230 is coupled to the ultrasonic energy output 7245 and one position of the end effector jaw position switch 7247, while the other position of the end effector jaw position switch 7247 is coupled to the primary side of transformer 7230. EEPROM 7239 and presence resistor 7241 are coupled to control circuit 7227. The system 7200 allows switching between an RF mode and an ultrasonic mode and supports mixed output frequencies, which allows tissues impedance sensing while the ultrasonic output is active. It also supports high RF output voltage for surface coagulation when end effector jaws are open and supports standard RF voltages for sealing and cutting when the jaws are closed.

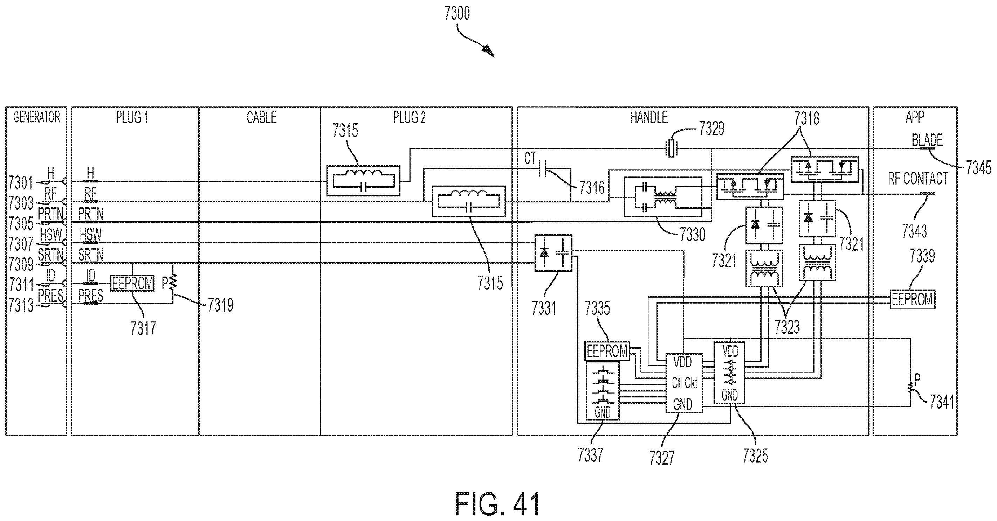

FIG. 41 illustrates a system 7300 that includes an electrically switched high voltage/low voltage RF output in the application portion, bandstop filters in distal plug, and a control circuit and transformer in the handle. The generator comprises interfaces for an ultrasonic signal 7301, an interface for an RF signal 7303, a primary return terminal interface 7305, an HSW interface 7307, a secondary return terminal interface 7309, an identification interface 7311, and a presence interface 7313. The proximal plug comprises matching interfaces to those of generator, an EEPROM 7317, and presence resistor 7319. The proximal plug outputs are carried through the cable without any component circuitry in the cable. The distal plug comprises a pair of bandstop filters 7315. The handle comprises rectifier circuit 7331, EEPROM 7335, control circuit 7327, transformer 7330, capacitor 7316, switch array 7337, and resonator 7329, driver circuit 7325, a pair of MOSFET switches 7318, rectifier circuits 7321, and a pair of coupling inductors 7323. The switch array 7337 may comprise electro-mechanical devices, transistor devices, and the like. The transistor devices may include bipolar junction transistors (BJT), FETs, MOSFETs, or a combination thereof. The pair of MOSFET switches 7318 are coupled to the rectifier circuits 7321 are coupled to coupling inductors 6223. Rectifier circuits 7321 each comprise at least one diode and at least one capacitor. The coupling inductors are coupled to driver circuit 6225, which is coupled to control circuit 7327. The coupling inductors are also coupled to driver circuit 7325. The control circuit 7227 is coupled to EEPROM 7335, switch array 7337, and rectifier circuit 7331.

The application portion comprises EEPROM 7339, presence resistor 7341, and outputs for RF and ultrasonic energy 7343, 7345, respectively. Transformer 7230 is coupled to one of the bandstop filters 7315 and one of the MOSFET switches 7318 on the primary side. The secondary side of transformer 7230 is coupled to the other MOSFET switches 7318. EEPROM 7339 and presence resistor 7341 are coupled to control circuit 7327. The system 7300 allows switching between an RF mode and an ultrasonic mode and supports mixed output frequencies, which allows tissues impedance sensing while the ultrasonic output is active. It also supports high RF output voltage for surface coagulation when end effector jaws are open and supports standard RF voltages for sealing and cutting when the jaws are closed.

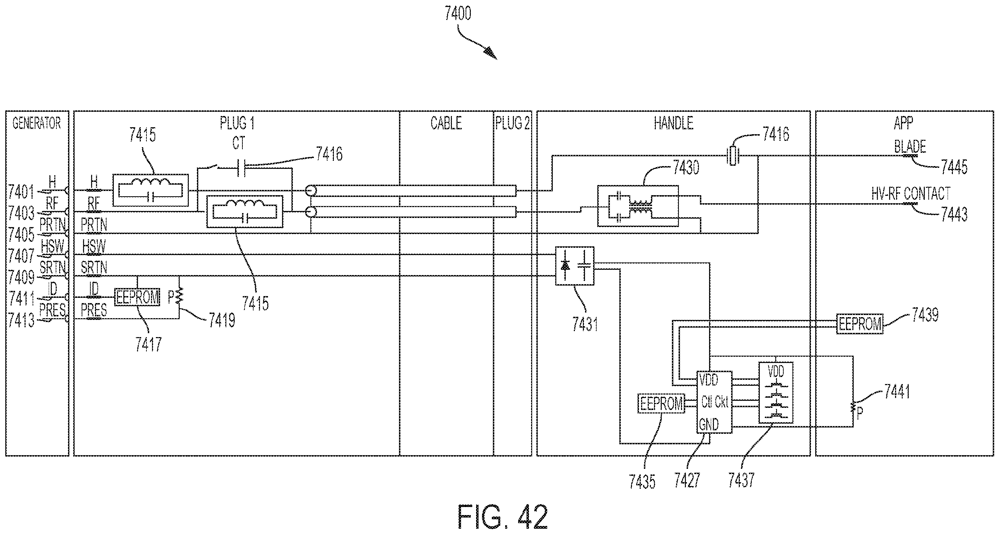

FIG. 42 illustrates a system 7400 that includes a fixed high voltage RF output in the application portion, bandstop filters in the proximal plug, and a control circuit and transformer in handle. The generator comprises interfaces for an ultrasonic signal 7401, an interface for an RF signal 7403, a primary return terminal interface 7405, an HSW interface 7407, a secondary return terminal interface 7409, an identification interface 7411, and a presence interface 7413. The proximal plug comprises matching interfaces to those of generator, an EEPROM 7417, presence resistor 7419, a pair of bandstop filters 7415, and switched capacitor 7416 (which include a switch circuit element and a capacitor circuit element). The proximal plug outputs are carried through the cable and distal plug without any component circuitry in either the cable or the distal plug. The handle comprises rectifier circuit 7431, EEPROM 7435, control circuit 7427, transformer 7430, switch array 7437, and resonator 7429. The control circuit 7427 is coupled to EEPROM 7435, switch array 7437, and rectifier circuit 7431. Rectifier circuit comprises at least one diode and at least one capacitor. The switch array 7437 may comprise electro-mechanical devices, transistor devices, and the like. The transistor devices may include bipolar junction transistors (BJT), FETs, MOSFETs, or a combination thereof.

The application portion comprises EEPROM 7439, presence resistor 7441, and high voltage RF and ultrasonic energy outputs 7443, 7445, respectively. Transformer 7430 is coupled to one of the bandstop filters 7115 and the secondary side of transformer 7130 is coupled to the high voltage RF and ultrasonic energy outputs 7443, 7445. EEPROM 7439 and presence resistor 7441 are coupled to control circuit 7427. The system 7400 allows switching between an RF mode and an ultrasonic mode and supports mixed output frequencies, which allows tissues impedance sensing while the ultrasonic output is active. It also supports high RF output voltage for surface coagulation and transfers weight, volume, and heat away from the handle and application portion.

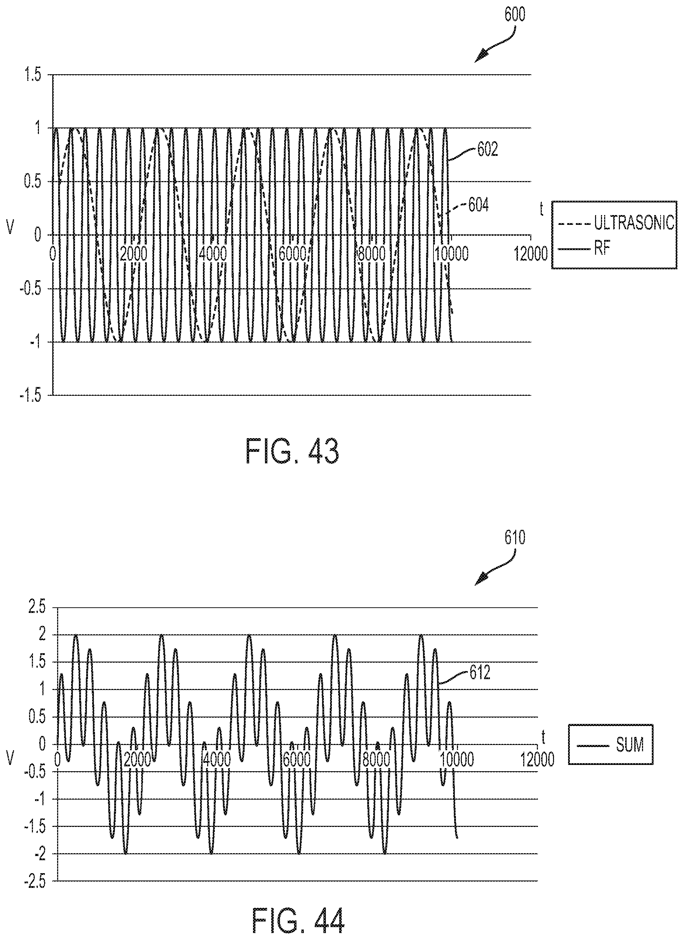

Examples of waveforms representing energy for delivery from a generator are illustrated in FIGS. 43-47. FIG. 43 illustrates an example graph 600 showing first and second individual waveforms representing an RF output signal 602 and an ultrasonic output signal 604 superimposed on the same time and voltage scale for comparison purposes. These output signals 602, 604 are provided at the ENERGY output of the generator 100. Time (t) is shown along the horizontal axis and voltage (V) is shown along the vertical axis. The RF output signal 602 has a frequency of about 330kHz RF and a peak-to-peak voltage of ±1V. The ultrasonic output signal 604 has a frequency of about 55kHz and a peak-to-peak voltage of ±1V. It will be appreciated that the time (t) scale along the horizontal axis and the voltage (V) scale along the vertical axis are normalized for comparison purposes and may be different actual implementations, or represent other electrical parameters such as current.

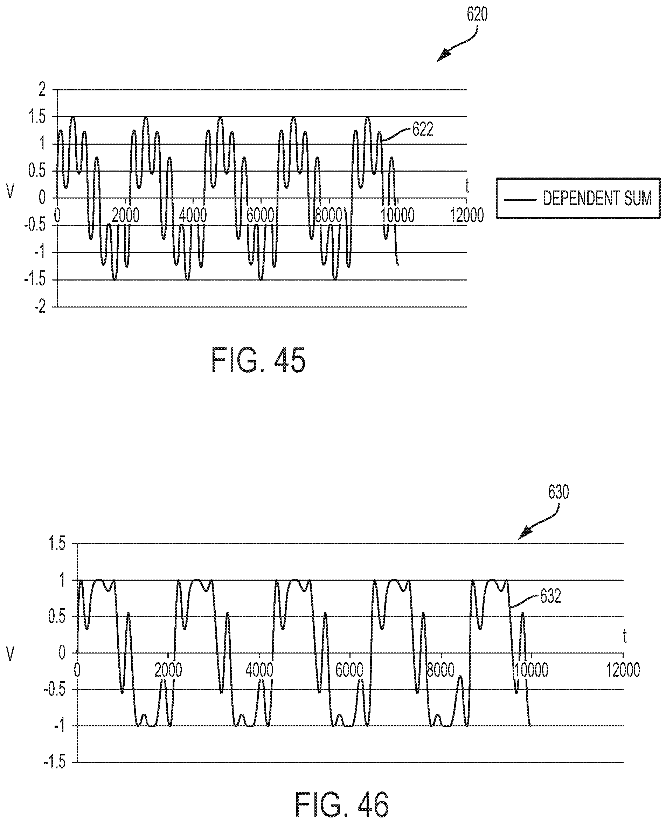

FIG. 44 illustrates an example graph 610 showing the sum of the two output signals 602, 604 shown in FIG. 43. Time (t) is shown along the horizontal axis and voltage (V) is shown along the vertical axis. The sum of the RF output signal 602 and the ultrasonic output signal 604 shown in FIG. 43 produces a combined output signal 612 having a 2V peak-to-peak voltage, which is twice the amplitude of the original RF and ultrasonic signals shown (1V peak-to-peak) shown in FIG. 43. An amplitude of twice the original amplitude can cause problems with the output section of the generator, such as distortion, saturation, clipping of the output, or stresses on the output components. Thus, the management of a single combined output signal 612 that has multiple treatment components is an important aspect of the generator 500 shown in FIG. 8. There are a variety of ways to achieve this management. In one form, one of the two RF or ultrasonic output signals 602, 604 can be dependent on the peaks of the other output signal. In one aspect, the RF output signal 602 may depend on the peaks of the ultrasonic signal 604, such that the output is reduced when a peak is anticipated. Such a function and resulting waveform is shown in FIG. 45.

For example, FIG. 45 illustrates an example graph 620 showing a combined output signal 622 representative of a dependent sum of the output signals 602, 604 shown in FIG. 43. Time (t) is shown along the horizontal axis and voltage (V) is shown along the vertical axis. As shown in FIG. 43, the RF output signal 602 component of FIG. 43 depends on the peaks of the ultrasonic output signal 604 component of FIG. 43 such that the amplitude of the RF output signal component of the dependent sum combined output signal 622 is reduced when an ultrasonic peak is anticipated. As shown in the example graph 620 in FIG. 43, the peaks have been reduced from 2 to 1.5. In another form, one of the output signals is a function of the other output signal.

For example, FIG. 46 illustrates an example graph of an analog waveform 630 showing an output signal 632 representative of a dependent sum of the output signals 602, 604 shown in FIG. 43. Time (t) is shown along the horizontal axis and voltage (V) is shown along the vertical axis. As shown in FIG. 46, the RF output signal 602 is a function of the ultrasonic output signal 604. This provides a hard limit on the amplitude of the output. As shown in FIG. 46, the ultrasonic output signal 604 is extractable as a sine wave while the RF output signal 602 has distortion but not in a way to affect the coagulation performance of the RF output signal 602.

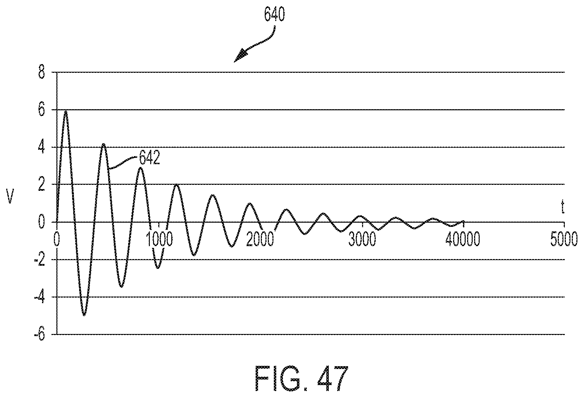

A variety of other techniques can be used for compressing and/or limiting the waveforms of the output signals. It should be noted that the integrity of the ultrasonic output signal 604 (FIG. 43) can be more important than the integrity of the RF output signal 602 (FIG. 43) as long as the RF output signal 602 has low frequency components for safe patient levels so as to avoid neuro-muscular stimulation. In another form, the frequency of an RF waveform can be changed on a continuous basis in order to manage the peaks of the waveform. Waveform control is important as more complex RF waveforms, such as a coagulation-type waveform 642, as illustrated in the graph 640 shown in FIG. 47, are implemented with the system. Again, time (t) is shown along the horizontal axis and voltage (V) is shown along the vertical axis. The coagulation-type waveform 642 illustrated in FIG. 47 has a crest factor of 5.8, for example.

While the examples herein are described mainly in the context of electrosurgical instruments, it should be understood that the teachings herein may be readily applied to a variety of other types of medical instruments. By way of example only, the teachings herein may be readily applied to tissue graspers, tissue retrieval pouch deploying instruments, surgical staplers, ultrasonic surgical instruments, etc. It should also be understood that the teachings herein may be readily applied to any of the instruments described in any of the references cited herein, such that the teachings herein may be readily combined with the teachings of any of the references cited herein in numerous ways. Other types of instruments into which the teachings herein may be incorporated will be apparent to those of ordinary skill in the art.

Aspects of the present disclosure have application in conventional endoscopic and open surgical instrumentation as well as application in robotic-assisted surgery. For instance, those of ordinary skill in the art will recognize that various teaching herein may be readily combined with various teachings of U.S. Patent No. 6,783,524, titled ROBOTIC SURGICAL TOOL WITH ULTRASOUND CAUTERIZING AND CUTTING INSTRUMENT, published Aug. 31, 2004.

Aspects of the devices disclosed herein can be designed to be disposed of after a single use, or they can be designed to be used multiple times. Various aspects may, in either or both cases, be reconditioned for reuse after at least one use. Reconditioning may include any combination of the steps of disassembly of the device, followed by cleaning or replacement of particular pieces, and subsequent reassembly. In particular, aspects of the device may be disassembled, and any number of the particular pieces or parts of the device may be selectively replaced or removed in any combination. Upon cleaning and/or replacement of particular parts, aspects of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, aspects described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various aspects of the present disclosure, further adaptations of the methods and systems described herein may be accomplished by appropriate modifications by one of ordinary skill in the art without departing from the scope of the claims. Several of such potential modifications have been mentioned, and others will be apparent to those skilled in the art. For instance, the examples, aspects, geometrics, materials, dimensions, ratios, steps, and the like discussed above are illustrative and are not required. Accordingly, the scope of the present disclosure should be considered in terms of the following claims and is understood not to be limited to the details of structure and operation shown and described in the specification and drawings.

While various details have been set forth in the foregoing description, it will be appreciated that the various aspects of the present disclosure may be practiced without these specific details. For example, for conciseness and clarity selected aspects have been shown in block diagram form rather than in detail. Some portions of the detailed descriptions provided herein may be presented in terms of instructions that operate on data that is stored in a computer memory. Such descriptions and representations are used by those skilled in the art to describe and convey the substance of their work to others skilled in the art. In general, an algorithm refers to a self-consistent sequence of steps leading to a desired result, where a "step" refers to a manipulation of physical quantities which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities.

Unless specifically stated otherwise as apparent from the foregoing discussion, it is appreciated that, throughout the foregoing description, discussions using terms such as "processing" or "computing" or "calculating" or "determining" or "displaying" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is worthy to note that any reference to "one aspect" or "an aspect" means that a particular feature, structure, or characteristic described in connection with the aspect is included in at least one aspect. Thus, appearances of the phrases "in one aspect" or "in an aspect" in various places throughout the specification are not necessarily all referring to the same aspect.

Although various aspects have been described herein, many modifications, variations, substitutions, changes, and equivalents to those aspects falling within the scope of the appended claims may be implemented and will occur to those skilled in the art.

Some or all of the aspects described herein may generally comprise technologies for managing RF and ultrasonic signals output by a generator, or otherwise according to technologies described herein. In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

The foregoing detailed description has set forth various aspects of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one aspect, several portions of the subject matter described herein may be implemented via Application Specific Integrated Circuits (ASICs), Field Programmable Gate Arrays (FPGAs), digital signal processors (DSPs), or other integrated formats. Those skilled in the art will recognize, however, that some aspects disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative aspect of the subject matter described herein applies regardless of the particular type of signal bearing medium used to actually carry out the distribution. Examples of a signal bearing medium include, but are not limited to, the following: a recordable type medium such as a floppy disk, a hard disk drive, a Compact Disc (CD), a Digital Video Disk (DVD), a digital tape, a computer memory, etc.; and a transmission type medium such as a digital and/or an analog communication medium (e.g., a fiber optic cable, a waveguide, a wired communications link, a wireless communication link (e.g., transmitter, receiver, transmission logic, reception logic, etc.), etc.).

One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

The herein described subject matter sometimes illustrates different components contained within, or connected with, different other components. It is to be understood that such depicted architectures are merely exemplary, and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected," or "operably coupled," to each other to achieve the desired functionality, and any two components capable of being so associated can also be viewed as being "operably couplable," to each other to achieve the desired functionality. Specific examples of operably couplable include but are not limited to physically mateable and/or physically interacting components, and/or wirelessly interactable, and/or wirelessly interacting components, and/or logically interacting, and/or logically interactable components.

Some aspects may be described using the expression "coupled" and "connected" along with their derivatives. It should be understood that these terms are not intended as synonyms for each other. For example, some aspects may be described using the term "connected" to indicate that two or more elements are in direct physical or electrical contact with each other. In another example, some aspects may be described using the term "coupled" to indicate that two or more elements are in direct physical or electrical contact. The term "coupled," however, also may mean that two or more elements are not in direct contact with each other, but yet still cooperate or interact with each other.

In some instances, one or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order that fall within the scope as defined in the appended claims. Also, although various operational flows are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

In certain cases, use of a system or method may occur in a territory even if components are located outside the territory. For example, in a distributed computing context, use of a distributed computing system may occur in a territory even though parts of the system may be located outside of the territory (e.g., relay, server, processor, signal-bearing medium, transmitting computer, receiving computer, etc. located outside the territory).

A sale of a system or method may likewise occur in a territory even if components of the system or method are located and/or used outside the territory. Further, implementation of at least part of a system for performing a method in one territory does not preclude use of the system in another territory.

In summary, numerous benefits have been described which result from employing the concepts described herein. The foregoing description of the one or more aspects has been presented for purposes of illustration and description. It is not intended to be exhaustive or limiting to the precise form disclosed. Modifications or variations are possible in light of the above teachings. The one or more aspects were chosen and described in order to illustrate principles and practical application to thereby enable one of ordinary skill in the art to utilize the various aspects and with various modifications as are suited to the particular use contemplated. It is intended that the claims submitted herewith define the overall scope.

**Claims**

1. A system (10) for managing radio frequency, RF, and ultrasonic signals output by a generator (100), comprising:

   a surgical instrument (108) comprising an RF energy output, an ultrasonic energy output, and a circuit (500; 1305);
   wherein the circuit is configured to:

   receive a combined RF and ultrasonic signal from the generator;
   generate an RF filtered signal by filtering RF frequency content from the combined signal;
   generate an ultrasonic filtered signal by filtering ultrasonic frequency content from the combined signal;
   provide the RF filtered signal to the RF energy output for applying RF energy to an end effector of the surgical instrument in a cutting mode, and/or a coagulation mode, and/or a desiccation mode; and
   provide the ultrasonic filtered signal to the ultrasonic energy output for driving an ultrasonic transducer of the surgical instrument in a cutting mode and/or in a coagulation mode.

2. The system of claim 1, wherein the circuit comprises:

   a first resonator tuned to a frequency of the RF output; and
   a second resonator tuned to a frequency of the ultrasonic output.

3. The system of claim 1, wherein the circuit comprises a high frequency band-stop filter.

4. The system of claim 3, wherein the high frequency band-stop filter comprises a first inductor-capacitor, LC, filter circuit configured to block the RF frequency content of the combined signal and a second LC filter circuit configured to block the ultrasonic frequency content of the combined signal.

5. The system of claim 1, wherein the circuit comprises a high frequency pass band filter.

6. The system of claim 5, wherein the high frequency pass band filter comprises a first resistor-inductor-capacitor, RLC, filter circuit configured to allow passage of the RF frequency content of the combined signal while blocking all other frequency content and a second RLC filter circuit configured to allow passage of the ultrasonic frequency content of the combined signal while blocking all other frequency content.

7. The system of claim 1, wherein the surgical instrument is configured to apply a therapy from the RF energy output and the ultrasonic energy output simultaneously.

8. A system (10) for managing radio frequency, RF, and ultrasonic signals output by a generator (100), comprising:

   a surgical instrument (108) comprising an RF energy output, an ultrasonic energy output, and a circuit (3405);
   wherein the circuit is configured to:
   receive a combined RF and ultrasonic signal from the generator, and
   wherein the circuit comprises a switch configured to switch between the RF energy output and the ultrasonic energy output according to the combined signal received from the generator, wherein the RF energy output is for applying RF energy to an end effector of the surgical instrument in a cutting mode, and/or a coagulation mode,
   and/or a desiccation mode, and wherein the ultrasonic energy output is for driving an ultrasonic transducer of the surgical instrument in a cutting mode and/or in a coagulation mode.

9. The system of claim 8, wherein the switch comprises two pairs of metal oxide semiconductor field effect transistor, MOSFET, switching elements.

10. The system of claim 9, wherein each of the two pairs of MOSFET switching elements is connected source to source.

11. The system of claim 9, wherein the circuit further comprises a first coupled inductor and a second coupled inductor.

12. The system of claim 11, wherein:

the two pairs of MOSFET switching elements comprises a first pair of MOSFET switching elements and a second pair of MOSFET switching elements; and
a gate of each MOSFET of the first pair of MOSFET switching elements is coupled together and is coupled to the first coupled inductor.

13. The system of claim 12, wherein the gate of each MOSFET of the second pair of MOSFET switching elements is coupled together and is coupled to the second coupled inductor.

14. The system of claim 12, wherein:

the circuit further comprises a first capacitor and a second capacitor;
the first capacitor is coupled to a primary side of the first coupled inductor; and
the second capacitor is coupled to a primary side of the second coupled inductor.

15. The system of claim 9, wherein the circuit further comprises:

an application-specific integrated circuit, ASIC;
a first pulse transformer coupled to the ASIC on a first side of the first pulse transformer and coupled to a first pair of the two pairs of MOSFET switching elements on a second side of the first pulse transformer; and
a second pulse transformer coupled to the ASIC on a first side of the second pulse transformer and coupled to a second pair of the two pairs of MOSFET switching elements on a second side of the second pulse transformer.

16. The system of claim 15, wherein one polarity of a differential pulse applied to the first pulse transformer is configured to enhance the first pair of MOSFET pairs, and an opposite polarity of the differential pulse applied to the first pulse transformer is configured to turn off the first pair of MOSFET pairs.

17. The system of claim 8, wherein the switch comprises:

an application-specific integrated circuit, ASIC;
a first electromechanical relay coupled to the ASIC and the RF energy output and is configured to switch to the RF energy output; and
a second electromechanical relay coupled to the ASIC and the ultrasonic energy output and is configured to switch to the ultrasonic energy output.

18. The system of claim 17, wherein the circuit further comprises a switch mechanism configured to actuate the first electromechanical relay and the second electromechanical relay.

19. The system of claim 18, wherein the switch mechanism comprises a mechanical rocker style switch mechanism.

20. The system of claim 8 wherein the switch comprises:

a filter circuit configured to filter frequency content of the combined signal; and
a switching element configured to switch between an on-state and an off-state to one of the RF energy output or the ultrasonic energy output according to the combined signal received from the generator.

**Patentansprüche**

1. System (10) zum Verwalten von Hochfrequenz-, HF- und Ultraschallsignalen, die durch einen Generator (100) ausgegeben werden, umfassend:

ein chirurgisches Instrument (108), umfassend einen HF-Energieausgang, einen Ultraschallenergieausgang und eine Schaltung (500; 1305); wobei die Schaltung konfiguriert ist zum:

Empfangen eines kombinierten HF- und Ultraschallsignals von dem Generator;
Erzeugen eines HF-gefilterten Signals durch Filtern von HF-Frequenzinhalt aus dem kombinierten Signal;
Erzeugen eines ultraschallgefilterten Signals durch Filtern von Ultraschallfrequenzinhalt aus dem kombinierten Signal;
Bereitstellen des HF-gefilterten Signals an den HF-Energieausgang zum Anwenden von HF-Energie auf einen Endeffektor des chirurgischen Instruments in einem Schneidmodus und/oder einem Koagulationsmodus und/oder einem Trocknungsmodus; und

Bereitstellen des ultraschallgefilterten Signals für den Ultraschallenergieausgang zum Antreiben eines Ultraschallwandlers des chirurgischen Instruments in einem Schneidmodus und/oder in einem Koagulationsmodus.

2. System nach Anspruch 1, wobei die Schaltung umfasst:

einen ersten Resonator, der auf eine Frequenz des HF-Ausgangs abgestimmt ist; und
einen zweiten Resonator, der auf die Frequenz des Ultraschallausgangs abgestimmt ist.

3. System nach Anspruch 1, wobei die Schaltung einen Hochfrequenz-Bandsperrfilter umfasst.

4. System nach Anspruch 3, wobei der Hochfrequenz-Bandsperrfilter eine erste Induktor-Kondensator-Filterschaltung, LC-Filterschaltung, die konfiguriert ist, um den HF-Frequenzinhalt des kombinierten Signals zu blockieren, und eine zweite LC-Filterschaltung, die konfiguriert ist, um den Ultraschallfrequenzinhalt des kombinierten Signals zu blockieren, umfasst.

5. System nach Anspruch 1, wobei die Schaltung einen Hochfrequenz-Durchlassbandfilter umfasst.

6. System nach Anspruch 5, wobei der Hochfrequenz-Durchlassbandfilter eine erste Widerstands-Induktor-Kondensator-Filterschaltung, RLC-Filterschaltung, die konfiguriert ist, um einen Durchgang des HF-Frequenzinhalts des kombinierten Signals zu erlauben, während jeder andere Frequenzinhalt blockiert wird, und eine zweite RLC-Filterschaltung, die konfiguriert ist, um den Durchgang des Ultraschallfrequenzinhalts des kombinierten Signals zu erlauben, während jeder andere Frequenzinhalt blockiert wird, umfasst.

7. System nach Anspruch 1, wobei das chirurgische Instrument konfiguriert ist, um eine Therapie aus dem HF-Energieausgang und dem Ultraschallenergieausgang gleichzeitig anzuwenden.

8. System (10) zum Verwalten von Hochfrequenz-, HF- und Ultraschallsignalen, die durch einen Generator (100) ausgegeben werden, umfassend:

ein chirurgisches Instrument (108), umfassend einen HF-Energieausgang, einen Ultraschallenergieausgang und eine Schaltung (3405); wobei die Schaltung konfiguriert ist zum:

Empfangen eines kombinierten HF- und Ultraschallsignals von dem Generator, und wobei die Schaltung einen Schalter, der konfiguriert ist, um gemäß dem kombinierten Signal, das von dem Generator empfangen wird, zwischen dem HF-Energieausgang und dem Ultraschallenergieausgang zu schalten, wobei der HF-Energieausgang zum Anwenden von HF-Energie auf einen Endeffektor des chirurgischen Instruments in einem Schneidmodus und/oder einem Koagulationsmodus und/oder einem Trocknungsmodus ist, und wobei der Ultraschallenergieausgang zum Antreiben eines Ultraschallwandlers des chirurgischen Instruments in einem Schneidmodus und/oder einem Koagulationsmodus ist.

9. System nach Anspruch 8, wobei der Schalter zwei Paare von Metalloxid-Halbleiter-Feldeffekttransistor-Schaltelementen, MOSFET-Schaltelementen, umfasst.

10. System nach Anspruch 9, wobei jedes der zwei Paare von MOSFET-Schaltelementen von Quelle zu Quelle verbunden ist.

11. System nach Anspruch 9, wobei die Schaltung ferner einen ersten gekoppelten Induktor und einen zweiten gekoppelten Induktor umfasst.

12. System nach Anspruch 11, wobei:

die zwei Paare von MOSFET-Schaltelementen ein erstes Paar von MOSFET-Schaltelementen und ein zweites Paar von MOSFET-Schaltelementen umfasst; und
ein Gate jedes MOSFET des ersten Paars von MOSFET-Schaltelementen miteinander gekoppelt ist und mit dem ersten gekoppelten Induktor gekoppelt ist.

13. System nach Anspruch 12, wobei das Gate jedes MOSFET des zweiten Paars von MOSFET-Schaltelementen miteinander gekoppelt ist und mit dem zweiten gekoppelten Induktor gekoppelt ist.

14. System nach Anspruch 12, wobei:

die Schaltung ferner einen ersten Kondensator und einen zweiten Kondensator umfasst;
der erste Kondensator mit einer Primärseite des ersten gekoppelten Induktors gekoppelt ist; und
der zweite Kondensator mit einer Primärseite des zweiten gekoppelten Induktors gekoppelt ist.

15. System nach Anspruch 9, wobei die Schaltung ferner umfasst:

eine anwendungsspezifische integrierte Schaltung, ASIC;

einen ersten Impulstransformator, der auf einer ersten Seite des ersten Impulstransformators mit der ASIC gekoppelt ist und auf einer zweiten Seite des ersten Impulstransformators mit einem ersten Paar der zwei Paare von MOSFET-Schaltelementen gekoppelt ist; und

einen zweiten Impulstransformator, der auf einer ersten Seite des zweiten Impulstransformators mit der ASIC gekoppelt ist und auf einer zweiten Seite des zweiten Impulstransformators mit einem zweiten Paar der zwei Paare von MOSFET-Schaltelementen gekoppelt ist.

16. System nach Anspruch 15, wobei eine Polarität eines Differenzpulses, die auf den ersten Impulstransformator angewendet wird, konfiguriert ist, um das erste Paar von MOSFET-Paaren zu verstärken, und eine entgegengesetzte Polarität des Differenzpulses, die auf den ersten Impulstransformator angewendet wird, konfiguriert ist, um das erste Paar von MOSFET-Paaren abzuschalten.

17. System nach Anspruch 8, wobei der Schalter umfasst:

eine anwendungsspezifische integrierte Schaltung, ASIC;

ein erstes elektromechanisches Relais, das mit der ASIC und dem HF-Energieausgang gekoppelt ist und konfiguriert ist, um auf den HF-Energieausgang zu schalten; und

ein zweites elektromechanisches Relais, das mit der ASIC und dem Ultraschallenergieausgang gekoppelt ist und konfiguriert ist, um auf den Ultraschallenergieausgang zu schalten.

18. System nach Anspruch 17, wobei die Schaltung ferner einen Schaltmechanismus, der konfiguriert ist, um das erste elektromechanische Relais und das zweite elektromechanische Relais zu betätigen, umfasst.

19. System nach Anspruch 18, wobei der Schaltmechanismus einen Mechanismus mit mechanischem Wippschalter umfasst.

20. System nach Anspruch 8, wobei der Schalter umfasst:

eine Filterschaltung, die konfiguriert ist, um Frequenzinhalt des kombinierten Signals zu filtern; und

ein Schaltelement, das konfiguriert ist, um gemäß dem kombinierten Signal, das von dem Generator empfangen wird, zwischen einem Ein-Zustand und einem Aus-Zustand auf einen des HF-Energieausgangs oder des Ultraschallenergieausgangs zu schalten.

## Revendications

1. Système (10) de gestion de signaux de radiofréquence (RF) et ultrasoniques délivrés en sortie par un générateur (100), comprenant :

un instrument chirurgical (108) comprenant une sortie d'énergie RF, une sortie d'énergie ultrasonique et un circuit (500 ; 1305) ;
dans lequel le circuit de commande est configuré pour :

recevoir un signal RF et ultrasonique combiné du générateur ;
générer un signal filtré RF en filtrant du contenu de fréquence RF à partir du signal combiné ;
générer un signal ultrasonique filtré en filtrant du contenu de fréquence ultrasonique à partir du signal combiné ;
fournir le signal filtré RF à la sortie d'énergie RF pour l'application d'énergie RF à un effecteur terminal de l'instrument chirurgical dans un mode de coupe, et/ou un mode de coagulation, et/ou un mode de dessiccation ; et
fournir le signal ultrasonique filtré à la sortie d'énergie ultrasonique pour la commande d'un transducteur ultrasonique de l'instrument chirurgical dans un mode de coupe et/ou dans un mode de coagulation.

2. Système selon la revendication 1, dans lequel le circuit comprend :

un premier résonateur accordé à une fréquence de la sortie RF ; et
un second résonateur accordé à une fréquence de la sortie ultrasonique.

3. Système selon la revendication 1, dans lequel le circuit comprend un filtre coupe-bande à haute fréquence.

4. Système selon la revendication 3, dans lequel le filtre coupe-bande haute fréquence comprend un premier circuit de filtrage inductance-capacité, LC, configuré pour bloquer le contenu de fréquence RF du signal combiné et un second circuit de filtrage LC configuré pour bloquer le contenu de fréquence ultrasonique du signal combiné.

5. Système selon la revendication 1, dans lequel le circuit comprend un filtre de bande passante à haute fréquence.

6. Système selon la revendication 5, dans lequel le filtre de bande passante à haute fréquence comprend un premier circuit de filtrage résistance-inducteur-condensateur (RLC) configuré pour permettre le passage du contenu de fréquence RF du signal combiné tout en bloquant tout autre contenu de fréquence et un second circuit de filtrage RLC configuré pour permettre le passage du contenu de fréquence ultrasonique du signal combiné tout en bloquant tout autre contenu de fréquence.

7. Système selon la revendication 1, dans lequel l'instrument chirurgical est configuré pour appliquer simultanément une thérapie à partir de la sortie d'énergie RF et de la sortie d'énergie ultrasonique.

8. Système (10) de gestion de signaux de radiofréquence (RF) et ultrasoniques délivrés en sortie par un générateur (100), comprenant :

   un instrument chirurgical (108) comprenant une sortie d'énergie RF, une sortie d'énergie ultrasonique et un circuit (3405) ;
   dans lequel le circuit de commande est configuré pour :
   recevoir un signal RF et ultrasonique combiné du générateur, et dans lequel le circuit comprend un commutateur configuré pour commuter entre la sortie d'énergie RF et la sortie d'énergie ultrasonique selon le signal combiné reçu du générateur, dans lequel la sortie d'énergie RF est destinée à appliquer l'énergie RF à un effecteur terminal de l'instrument chirurgical dans un mode coupe, et/ou un mode coagulation, et/ou un mode dessiccation, et dans lequel la sortie d'énergie ultrasonique est destinée à piloter un transducteur ultrasonique de l'instrument chirurgical dans un mode coupe et/ou un mode coagulation.

9. Système selon la revendication 8, dans lequel le commutateur comprend deux paires d'éléments de commutation de transistors à effet de champ à semiconducteur à oxyde métallique (MOSFET).

10. Système selon la revendication 9, dans lequel chacune des deux paires d'éléments de commutation MOSFET est connectée source à source.

11. Système selon la revendication 9, dans lequel le circuit comprend en outre un premier inducteur couplé et un second inducteur couplé.

12. Système selon la revendication 11, dans lequel :

   les deux paires d'éléments de commutation MOSFET comprennent une première paire d'éléments de commutation MOSFET et une seconde paire d'éléments de commutation MOSFET ; et
   une grille de chaque MOSFET de la première paire d'éléments de commutation MOSFET est couplée ensemble et est couplée au premier inducteur couplé.

13. Système selon la revendication 12, dans lequel une grille de chaque MOSFET de la seconde paire d'éléments de commutation MOSFET est couplée ensemble et est couplée au second inducteur couplé.

14. Système selon la revendication 12, dans lequel :

   le circuit comprend en outre un premier condensateur et un second condensateur ;
   le premier condensateur est couplé à un côté primaire du premier inducteur couplé ; et
   le second condensateur est couplé à un côté primaire du second inducteur couplé.

15. Système selon la revendication 9, dans lequel le circuit comprend en outre :

   un circuit intégré spécifique à une application, ASIC ;
   un premier transformateur d'impulsions couplé à l'ASIC sur un premier côté du premier transformateur d'impulsions et couplé à une première paire des deux paires d'éléments de commutation MOSFET sur un second côté du premier transformateur d'impulsions ; et
   un second transformateur d'impulsions couplé à l'ASIC sur un second côté du premier transformateur d'impulsions et couplé à une seconde paire des deux paires d'éléments de commutation MOSFET sur un second côté du second transformateur d'impulsions.

16. Système selon la revendication 15, dans lequel une polarité d'une impulsion différentielle appliquée au premier transformateur d'impulsion est configurée pour améliorer la première paire de paires de MOSFET, et une polarité opposée de l'impulsion différentielle appliquée au premier transformateur d'impulsion est configurée pour éteindre la première paire de paires de MOSFET.

17. Système selon la revendication 8, dans lequel l'interrupteur comprend :

   un circuit intégré spécifique à une application, ASIC ;
   un premier relais électromécanique couplé à l'ASIC et à la sortie d'énergie RF et configuré pour commuter sur la sortie d'énergie RF ; et

un second relais électromécanique couplé à l'ASIC et à la sortie d'énergie ultrasonique et configuré pour commuter sur la sortie d'énergie ultrasonique.

18. Système selon la revendication 17, dans lequel le circuit comprend en outre un mécanisme de commutation configuré pour actionner le premier relais électromécanique et le second relais électromécanique.

19. Système selon la revendication 18, dans lequel le mécanisme de commutation comprend un mécanisme de commutation mécanique à bascule.

20. Système selon la revendication 8 dans lequel le commutateur comprend :

un circuit de filtrage configuré pour filtrer du contenu de fréquence du signal combiné ; et un élément de commutation configuré pour passer d'un état actif à un état inactif sur l'une parmi la sortie d'énergie RF ou la sortie d'énergie ultrasonique selon le signal combiné reçu du générateur.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

EP 3 355 815 B1

FIG. 33

FIG. 34

FIG. 35

EP 3 355 815 B1

FIG. 36

EP 3 355 815 B1

FIG. 37

EP 3 355 815 B1

FIG. 38

FIG. 39

FIG. 40

EP 3 355 815 B1

FIG. 41

GENERATOR

7401 H
7403 RF
7405 PRTN
7407 HSW
7409 SRTN
7411 ID
7413 PRES

PLUG 1
CT

7415

7416

7419
7417
EEPROM
7415

PLUG 2

CABLE

HANDLE

7416

7430

7431

VDD
Ch Ck1
GND
7427

EEPROM
7435

VDD
7437

APP
BLADE
7445

HV-RF CONTACT
7443

EEPROM
7439

7441

7400

FIG. 42

600

602
604
t
ULTRASONIC
RF

## FIG. 43

610

612
t
SUM

## FIG. 44

FIG. 45

FIG. 46

FIG. 47

# EP 3 355 815 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62235260
- US 62235368
- US 62235466
- US 2011273465 A1
- US 2003181898 A1
- GB 2521228 A
- US 5938633 A
- US 5935144 A
- US 5944737 A
- US 5322055 A
- US 5630420 A
- US 5449370 A
- US 17743016
- US 17743916
- US 17744916
- US 17745616
- US 17746616
- US 9060775 B
- US 6783524 B